(19) Europäisches Patentamt
European Patent Office
Office européen des brevets

(11) EP 3 867 247 B1

(12) EUROPEAN PATENT SPECIFICATION

(45) Date of publication and mention of the grant of the patent:
**24.07.2024 Bulletin 2024/30**

(21) Application number: **19790048.3**

(22) Date of filing: **16.10.2019**

(51) International Patent Classification (IPC):
***C07D 405/14*** (2006.01) ***C07D 403/04*** (2006.01)
***C07D 405/12*** (2006.01) ***A61P 25/00*** (2006.01)
***A61P 27/00*** (2006.01) ***A61K 31/497*** (2006.01)

(52) Cooperative Patent Classification (CPC):
**C07D 405/14; A61P 25/00; A61P 27/00; C07D 403/04; C07D 405/12**

(86) International application number:
**PCT/GB2019/052937**

(87) International publication number:
**WO 2020/079422 (23.04.2020 Gazette 2020/17)**

# (54) NOVEL COMPOUNDS

NEUARTIGE VERBINDUNGEN

NOUVEAUX COMPOSÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2018 EP 18200626**

(43) Date of publication of application:
**25.08.2021 Bulletin 2021/34**

(73) Proprietor: **Autifony Therapeutics Limited**
**Hertfordshire SG1 2FX (GB)**

(72) Inventors:
- **ALVARO, Giuseppe**
  **Stevenage Hertfordshire SG1 2FX (GB)**
- **MARASCO, Agostino**
  **Stevenage, Hertfordshire SG1 2FX (GB)**

(74) Representative: **Sagittarius IP**
**Marlow International**
**Parkway**
**Marlow SL7 1YL (GB)**

(56) References cited:
**WO-A1-2012/076877** **WO-A1-2013/083994**
**WO-A1-2013/182851** **WO-A1-2017/103604**
**WO-A1-2018/109484**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 867 247 B1

## Description

Technical field

This invention relates to novel compounds, pharmaceutical compositions containing them and their use in therapy, in particular in the prophylaxis or treatment of hearing disorders, including hearing loss and tinnitus, as well as schizophrenia, substance abuse disorders, pain and Fragile X syndrome.

Background to the invention

The Kv3 voltage-gated potassium channel family includes four members, Kv3.1, Kv3.2, Kv3.3, and Kv3.4. Kv3 channels are activated by depolarisation of the plasma membrane to voltages more positive than -20mV; furthermore, the channels deactivate rapidly upon repolarisation of the membrane. These biophysical properties ensure that the channels open towards the peak of the depolarising phase of the neuronal action potential to initiate repolarisation. Rapid termination of the action potential mediated by Kv3 channels allows the neuron to recover more quickly to reach sub-threshold membrane potentials from which further action potentials can be triggered. As a result, the presence of Kv3 channels in certain neurons contributes to their ability to fire at high frequencies (Rudy *et al.,* 2001). Kv3.1-3 subtypes are predominant in the CNS, whereas Kv3.4 channels are also found in skeletal muscle and sympathetic neurons (Weiser *et al.,* 1994). Kv3.1-3 channel subtypes are differentially expressed by sub-classes of interneurons in cortical and hippocampal brain areas (e.g. Chow *et al.,* 1999; Martina *et al.,* 1998; McDonald *et al.,* 2006; Chang *et al.,* 2007), in the thalamus (e.g. Kasten *et al.,* 2007), cerebellum (e.g. Sacco *et al.,* 2006; Puente *et al.,* 2010), and auditory brain stem nuclei (Li *et al.,* 2001).

Tetraethylammonium (TEA) has been shown to inhibit the channels at low millimolar concentrations (Rudy *et al.,* 2001), and blood-depressing substance (BDS) toxins from the sea anemone, *Anemonia sulcata* (Diochot *et al.,* 1998), have been shown to selectively inhibit Kv3 channels with high affinity (Yeung *et al.,* 2005).

Kv3 channels are important determinants of the function of the cerebellum, a region of the brain important for motor control (Joho *et al.,* 2009). Characterisation of mice in which one or more of the Kv3 subtypes has been deleted shows that the absence of Kv3.1 gives rise to increased locomotor activity, altered electroencephalographic activity, and a fragmented sleep pattern (Joho *et al.,* 1999). The deletion of Kv3.2 leads to a reduction in seizure threshold and altered cortical electroencephalographic activity (Lau *et al.,* 2000). Deletion of Kv3.3 is associated with mild ataxia and motor deficits (McMahon *et al.,* 2004). Double deletion of Kv3.1 and Kv3.3 gives rise to a severe phenotype characterised by spontaneous seizures, ataxia, and an increased sensitivity to the effects of ethanol (Espinosa *et al.,* 2001; Espinosa *et al.,* 2008). A spontaneous mutation in the Kv3.1 gene (KCNC1) causes progressive myoclonic epilepsy (Muona et al., 2014). Mutations of the Kv3.3 gene (KCNC3) in humans have been associated with forms of spinocerebellar ataxia (SCA13) (Figueroa *et al.,* 2010).

Bipolar disorder, schizophrenia, anxiety, and epilepsy are serious disorders of the central nervous system that have been associated with reduced function of inhibitory interneurons and gammaamino butyric acid (GABA) transmission (Reynolds *et al.,* 2004; Benes *et al.,* 2008; Brambilla *et al*., 2003; Aroniadou-Anderjaska *et al.,* 2007; Ben-Ari, 2006). Parvalbumin positive basket cells that express Kv3 channels in the cortex and hippocampus play a key role in generating feedback inhibition within local circuits (Markram *et al.,* 2004). Given the relative dominance of excitatory synaptic input over inhibitory input to glutamatergic pyramidal neurons in these circuits, fast-firing of interneurons supplying inhibitory input is essential to ensure balanced inhibition. Furthermore, accurate timing of inhibitory input is necessary to sustain network synchronisation, for example, in the generation of gamma frequency field potential oscillations that have been associated with cognitive function (Fisahn *et al.,* 2005; Engel *et al.,* 2001). Notably, a reduction in gamma oscillations has been observed in patients with schizophrenia (Spencer *et al.,* 2004), and evidence suggests reduced expression of Kv3.1, but not Kv3.2 in the dorsolateral prefrontal cortex of patients with schizophrenia who had not been taking antipsychotic drugs for at least 2 months before death (Yanagi *et al.,* 2014). Consequently, positive modulators of Kv3 channels might be expected to enhance the firing capabilities of specific groups of fast-firing neurons in the brain. These effects may be beneficial in disorders associated with abnormal activity of these neuronal groups. In addition, Kv3.2 channels have been shown to be expressed by neurons of the superchiasmatic nucleus (SCN) the main circadian pacemaker in the CNS (Schulz *et al.,* 2009).

Voltage-gated ion channels of the Kv3 family are expressed at high levels in auditory brainstem nuclei (Li *et al.,* 2001) where they permit the fast firing of neurons that transmit auditory information from the cochlear to higher brain regions. Phosphorylation of Kv3.1 and Kv3.3 channels in auditory brainstem neurons is suggested to contribute to the rapid physiological adaptation to sound levels that may play a protective role during exposure to noise (Desai *et al.,* 2008; Song *et al.,* 2005). Loss of Kv3.1 channel expression in central auditory neurons is observed in hearing impaired mice (von Hehn *et al.,* 2004); furthermore, a decline in Kv3.1 expression may be associated with loss of hearing in aged mice (Jung *et al.* 2005), and loss of Kv3 channel function may also follow noise-trauma induced hearing loss (Pilati *et*

*al.,* 2012). Furthermore, pathological plasticity of auditory brainstem networks is likely to contribute to symptoms that are experienced by many people suffering from hearing loss of different types. Recent studies have shown that regulation of Kv3.1 channel function and expression has a major role in controlling auditory neuron excitability (Kaczmarek *et al.,* 2005; Anderson *et al.,* 2018; Glait *et al.,* 2018; Olsen *et al.,* 2018, Chambers *et al.,* 2017), suggesting that this mechanism could account for some of the plastic changes that give rise to tinnitus. Tinnitus may follow noise-induced hearing loss as a result of adaptive changes in central auditory pathways from brainstem to auditory cortex (Roberts *et al.,* 2010). Kv3.1 and/or Kv3.2 channels are expressed in many of these circuits and contribute to the function of GABAergic inhibitory interneurons that may control the function of these circuits.

It is known that Kv3.1 and/or Kv3.2 modulators have utility in the treatment of pain (WO2017/098254). In the broadest sense, pain can be grouped in to acute pain and chronic pain. Acute pain is defined as pain that is self-limited and generally requires treatment for no more than up to a few weeks, for example postoperative or acute musculoskeletal pain, such as fractures (US Food and Drug Administration, 2014). Chronic pain can be defined either as pain persisting for longer than 1 month beyond resolution of the initial trauma, or pain persisting beyond three months. There is often no clear cause of chronic pain, and a multitude of other health problems such as fatigue, depression, insomnia, mood changes and reduction in movement, often accompany chronic pain.

Chronic pain can be sub-divided in to the following groups: neuropathic pain, chronic musculoskeletal pain and miscellaneous chronic pain. Neuropathic pain usually accompanies tissue injury and is initiated or caused by damage to the nervous system (peripheral nervous system and/or central nervous system), such as amputation, stroke, diabetes, or multiple sclerosis. Chronic musculoskeletal pain can be a symptom of diseases such as osteoarthritis and chronic lower back pain and can occur following damage to muscle tissue as well as trauma to an area for example, fractures, sprains and dislocation. Miscellaneous chronic pain encompasses all other types of long term pain and includes non-neuropathic pain conditions such as cancer pain and fibromyalgia as well as headaches and tendinitis.

Chronic pain is a highly heterogeneous condition that remains amongst the most troublesome and difficult to manage of clinical indications (McCarberg *et al.,* 2008; Woolf, 2010; Finnerup *et al*., 2015). Despite years of research and drug development, there has been little progress in identifying treatments that can match the opioids for efficacy without significant side effects and risk of dependence. Voltage-gated ion channels have been important targets for the management of specific pain indications, in particular neuropathic pain states. Furthermore, genetic mutations in specific ion channels have been linked to some chronic pain disorders (Bennett *et al.,* 2014). Examples of voltage-gated ion channels that are being explored as pharmaceutical targets include: *Sodium channels (in particular NaV1.7)* - Sun *et al.,* 2014; Dib-Hajj *et al.,* 2013; *N-type calcium channels* - Zamponi *et al.,* 2015; *Kv7 potassium channels* - Devulder, 2010; Wickenden *et al.,* 2009; and *SLACK*- Lu *et al.,* 2015.

The hypothesis underlying these approaches is that chronic pain states are associated with increased excitability and/or aberrant firing of peripheral sensory neurons, in particular neurons involved in the transmission of painful sensory stimuli, such as the C-fibres of the dorsal root ganglia and specific circuits within the spinal cord (Baranauskas *et al.,* 1998; Cervero, 2009; Woolf *et al.,* 2011; Baron *et al.,* 2013). Animal models of neuropathic and inflammatory chronic pain provide the main support for this hypothesis, although demonstration of causality is still lacking (Cervero, 2009).

Drugs targeting hyperexcitability, such as sodium channel blockers (e.g. CNV1014802, lamotrigine, carbamazepine, and local anaesthetics), Kv7 positive modulators (e.g. flupertine and retigabine), and N-type calcium channel modulators (e.g. gabapentin, which interacts with the $\alpha 2\delta$ subunit of the N-type calcium channel, and ziconitide, derived from a cone snail toxin) show efficacy in models of inflammatory and/or neuropathic pain. However, amongst these drugs, there is mixed evidence for clinical efficacy, for example, balancing efficacy and increased burden of side effects on the central nervous system. The disparity between efficacy in animal models and efficacy in humans is likely to be due to a range of factors, but in particular, drug concentration achievable in humans (due to poor tolerability) and heterogeneity of human pain conditions are likely to be the main culprits. For pain indications, there is also a need to identify targets through which pain relief can be achieved with reduced tolerance or tachyphylaxis and reduced abuse liability and/or risk of dependence.

Thus, improving the pharmacological management of pain is focused on mechanisms that can deliver good efficacy with a reduced side-effect burden, reduced tolerance or tachyphylaxis, and reduced abuse liability and/or risk of dependence.

Recently, Kv3.4 channels have become a target of interest for the treatment of chronic pain. Kv3.4 channels are expressed on neurons of the dorsal root ganglia (Ritter *et al.,* 2012; Chien *et al.,* 2007), where they are predominantly expressed on sensory C-fibres (Chien *et al.,* 2007). Kv3 channels are also expressed by specific subsets of neurons in the spinal cord. Specifically, Kv3.1b (Deuchars *et al.,* 2001; Brooke *et al.,* 2002), Kv3.3 (Brooke *et al.,* 2006), and Kv3.4 subunits (Brooke *et al.,* 2004) have been identified in rodent spinal cord, although not always in association with circuits involved with sensory processing. It is likely that Kv3 channels shape the firing properties of spinal cord neurons, including motoneurons.

In addition recent studies showed the Kv3.4 channels expressed in DRG nociceptors have a significant impact on glutamatergic synaptic transmission (Muqeem *et al.,* 2018). animal model data suggest a down-regulation of Kv3.4

channel surface expression in DRG neurons following spinal cord injury associated with hypersensitivity to painful stimuli (Ritter *et al.,* 2015; Zemel *et* al., 2017; Zemel *et al.,* 2018). Similarly, it has been observed that there is a down-regulation of Kv3.4 expression in DRGs of rodents following spinal cord ligation (Chien *et al.,* 2007). This latter study also showed that intrathecal administration to rats of an antisense oligonucleotide to supress the expression of Kv3.4 led to hypersensitivity to mechanical stimuli. It has been shown that Kv3.4 channel inactivation could be influenced by protein kinase C-dependent phosphorylation of the channels, and that this physiological mechanism might allow DRG neurons to alter their firing characteristics in response to painful stimuli (Ritter *et al.,* 2012). These studies suggest a causal relationship between the emergence of mechanical allodynia and reduced Kv3.4 channel expression or function. No evaluation of Kv3.1, Kv3.2, or Kv3.3 expression in SC or DRG neurons was conducted in any of these studies, and expression of these two subtypes has not been explicitly demonstrated on DRG neurons (although as mentioned above, they are abundant within specific regions of the spinal cord). The *in vivo* studies reported above provide a rationale for modulation of Kv3.4 as a novel approach to the treatment of certain neuropathic pain states.

Dementia with Lewy Bodies (DLB) and Parkinson's disease (PD) are serious neurodegenerative disorders that are associated with the accumulation of the protein, alpha-synuclein in Lewy bodies, which leads to loss of connectivity and neuronal cell death. Symptoms of DLB include progressive cognitive deficits, in particular difficulties with planning and attention. Visual hallucinations are also common, occurring in approximately 60% of patients. PD is associated initially with motor deficits, primarily due to loss of dopamine neurons. While there are currently no studies directly linking Kv3 channels to DLB or PD, the location and role of Kv3 channels, in particular Kv3.1, in cortical and basal ganglia circuits suggests that modulators of these channels could improve symptoms of DLB or PD, either alone, or in combination with current treatments, such as acetyl-cholinesterase inhibitors for DLB or L-DOPA for PD.

Patent applications WO2011/069951, WO2012/076877, WO2012/168710, WO2013/175215, WO2013/083994, WO2013/182850, WO2017/103604, WO2018/020263 and WO2018/109484 disclose compounds which are modulators of Kv3.1 and Kv3.2. Further, the utility of such compounds is demonstrated in animal models of seizure, hyperactivity, sleep disorders, psychosis, hearing disorders and bipolar disorders.

Patent application WO2013/182851 discloses modulation of Kv3.3 channels by certain compounds.

Patent application WO2013/175211 discloses that modulation of Kv3.1, Kv3.2 and/or Kv3.3 channels has been found to be beneficial in preventing or limiting the establishment of a permanent hearing loss resulting from acute noise exposure. The benefits of such prevention may be observed even after administration of the Kv3.1, Kv3.2 and/or Kv3.3 modulator has ceased.

Patent application WO2017/098254 discloses that modulation of Kv3.1, Kv3.2 and/or Kv3.3 channels has been found to be beneficial in the prophylaxis or treatment of pain, in particular neuropathic or inflammatory pain.

There remains a need for the identification of alternative modulators of Kv3.1, Kv3.2 and/or Kv3.3, in particular modulators of Kv3.1 and/or Kv3.2. Such modulators may demonstrate high *in vivo* potency, channel selectivity, an improved safety profile, or desirable pharmacokinetic parameters, for example high brain availability, that reduces the dose required for therapeutic effect *in vivo.* Alternative modulators may provide a benefit through having distinct metabolites from known modulators. Compounds which have balanced Kv3.1, Kv3.2 and/or Kv3.3 modulatory properties may be desirable e.g. compounds with modulate Kv3.1 and Kv3.2 to the same, or a similar extent. For certain therapeutic indications, there is also a need to identify compounds with a different modulatory effect on Kv3.1, Kv3.2 and/or Kv3.3 channels, for example, compounds that alter the kinetics of channel gating or channel inactivation, and which may behave *in vivo* as negative modulators of the channels.

Summary of the invention

The present invention provides a compound of formula (I):

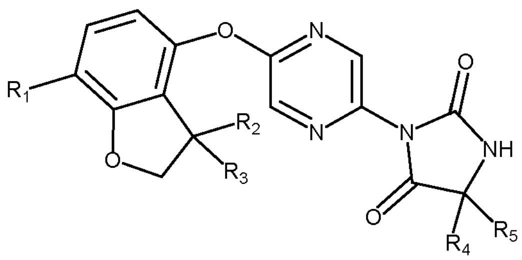

(I)

wherein:

$R_1$ is H or methyl;

$R_2$ and $R_3$ are both methyl, or $R_2$ and $R_3$, together with the carbon atom to which they are attached, are a spirocyclopropyl ring;
$R_4$ is methyl or ethyl;
$R_5$ is H or methyl;
or $R_4$ and $R_5$, together with the carbon atom to which they are attached, form a $C_3$-$C_4$ spiro carbocyclyl;
or a salt and/or solvate and/or derivative thereof of formula (Z),
wherein said derivative of formula (Z), is functionalised at the secondary nitrogen of the hydantoin with group L as illustrated below:

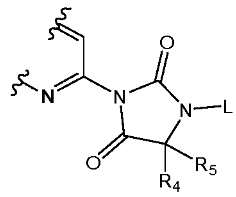
(Z),

and wherein L is selected from:

a) $-PO(OH)O^- \bullet M^+$, wherein $M^+$ is a pharmaceutically acceptable monovalent counterion,
b) $-PO(O^-)_2 \bullet 2M^+$,
c) $-PO(O^-)_2 \bullet D^{2+}$, wherein $D^{2+}$ is a pharmaceutically acceptable divalent counterion,
d) $-CH(R^X)-PO(OH)O^- \bullet M^+$, wherein $R^X$ is hydrogen or $C_{1-3}$ alkyl,
e) $-CH(R^X)-PO(O^-)_2 \bullet 2M^+$,
f) $-CH(R^X)-PO(O^-)_2 \bullet D^{2+}$,
g) $-SO_3^- \bullet M^+$,
h) $-CH(R^X)-SO_3^- \bullet M^+$, and
i) $-CO-CH_2CH_2-CO_2 \bullet M^+$.

A compound of formula (I) may be provided in the form of a salt and/or solvate thereof. Suitably, the compound of formula (I) may be provided in the form of a pharmaceutically acceptable salt and/or solvate thereof and/or derivative thereof of formula (Z). In one embodiment of the invention a compound of formula (I) is provided in the form of a pharmaceutically acceptable salt.

The compounds of formula (I) may be used as medicaments, in particular for use in the prophylaxis or treatment of hearing disorders, including hearing loss and tinnitus, as well as schizophrenia, substance abuse disorders, pain or Fragile X syndrome.

Also provided are pharmaceutical compositions containing a compound of formula (I) and a pharmaceutically acceptable carrier or excipient.

## Detailed description of the invention

The present invention provides compounds of formula (I):

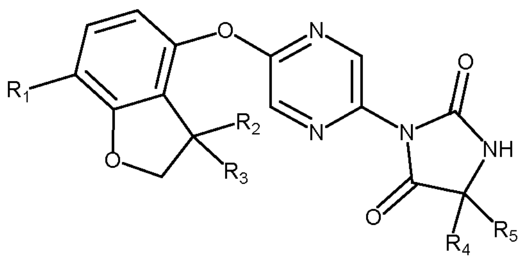
(I)

wherein:

$R_1$ is H or methyl;
$R_2$ and $R_3$ are both methyl, or $R_2$ and $R_3$, together with the carbon atom to which they are attached, are a spirocyclopropyl ring;

$R_4$ is methyl or ethyl;
$R_5$ is H or methyl;
or $R_4$ and $R_5$, together with the carbon atom to which they are attached, form a $C_3$-$C_4$ spiro carbocyclyl;
or a pharmaceutically acceptable salt and/or solvate and/or derivative thereof of formula (Z),
wherein said derivative of formula (Z) is functionalised at the secondary nitrogen of the hydantoin with group L as illustrated below:

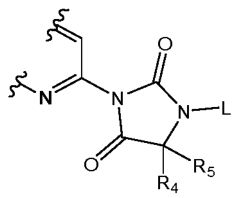

,

and wherein L is selected from:

a) $-PO(OH)O^- \cdot M^+$, wherein $M^+$ is a pharmaceutically acceptable monovalent counterion,
b) $-PO(O^-)_2 \cdot 2M^+$,
c) $-PO(O^-)_2 \cdot D^{2+}$, wherein $D^{2+}$ is a pharmaceutically acceptable divalent counterion,
d) $-CH(R^X)-PO(OH)O^- \cdot M^+$, wherein $R^X$ is hydrogen or $C_{1-3}$ alkyl,
e) $-CH(R^X)-PO(O^-)_2 \cdot 2M^+$,
f) $-CH(R^X)-PO(O^-)_2 \cdot D^{2+}$,
g) $-SO_3^- \cdot M^+$,
h) $-CH(R^X)-SO_3^- \cdot M^+$, and
i) $-CO-CH_2CH_2-CO_2 \cdot M^+$.

Embodiments set out below relating to relative stereochemistry and the nature of groups, including $R_1$, $R_2$, $R_3$, $R_4$, $R_5$, are envisaged as being independently, fully combinable with one another where appropriate to the circumstances (i.e. where chemically sensible) to form further embodiments of the invention. Such embodiments apply equally to intermediates which may be of use in the synthesis of a compound of formula (I) e.g. compounds of formulae (II), (IV), (VI), (VII) and (XVI).

Compounds of formula (I) may optionally be provided in the form of a pharmaceutically acceptable salt and/or solvate. In one embodiment of the invention a compound of formula (I) is provided in the form of a pharmaceutically acceptable salt. In a second embodiment of the invention a compound of formula (I) is provided in the form of a pharmaceutically acceptable solvate. In a third embodiment of the invention a compound of formula (I) is not in the form of a salt or solvate.

In one embodiment, $R_1$ is H. In a second embodiment $R_1$ is methyl.

In one embodiment, $R_2$ is methyl and $R_3$ is methyl. In another embodiment, $R_2$ and $R_3$ are a spiro cyclopropyl such that that the following moiety is formed:

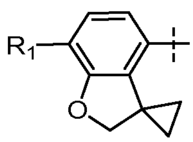

.

In one embodiment, $R_4$ is methyl. In a second embodiment, $R_4$ is ethyl.

In one embodiment, $R_5$ is hydrogen. In a second embodiment, $R_5$ is methyl.

In one embodiment $R_4$ and $R_5$ are the same (i.e. methyl).

In embodiments wherein $R_4$ and $R_5$ are different, they may have the following stereochemical arrangement:

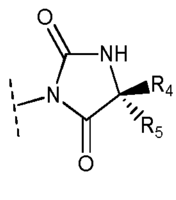

In this embodiment, for example, $R_4$ is methyl and $R_5$ is H, $R_4$ is ethyl and $R_5$ is H or $R_4$ is ethyl and $R_5$ is methyl.

In embodiments wherein $R_4$ and $R_5$ are different, they may alternatively have the following stereochemical arrangement:

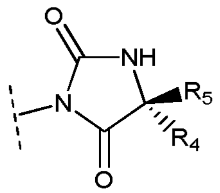

In this embodiment, for example, $R_4$ is methyl and $R_5$ is H, $R_4$ is ethyl and $R_5$ is H or $R_4$ is ethyl and $R_5$ is methyl.

In one embodiment $R_4$ and $R_5$, together with the carbon atom to which they are attached, form a spirocyclopropyl.

In another embodiment $R_4$ and $R_5$, together with the carbon atom to which they are attached, form a spirocyclobutyl.

In one embodiment, the compound of formula (I) is selected from the group consisting of:

5,5-dimethyl-3-[5-(7-methylspiro[2H-benzofuran-3,1'-cyclopropane]-4-yl)oxypyrazin-2-yl]imidazolidine-2,4-dione;
3-[5-[(3,3-dimethyl-2H-benzofuran-4-yl)oxy]pyrazin-2-yl]-5,5-dimethyl-imidazolidine-2,4-dione;
(5R)-5-ethyl-5-methyl-3-(5-spiro[2H-benzofuran-3,1'-cyclopropane]-4-yloxypyrazin-2-yl)imidazolidine-2,4-dione;
5,5-dimethyl-3-(5-spiro[2H-benzofuran-3,1'-cyclopropane]-4-yloxypyrazin-2-yl)imidazolidine-2,4-dione;
(5R)-5-ethyl-5-methyl-3-[5-(7-methylspiro[2H-benzofuran-3,1'-cyclopropane]-4-yl)oxypyrazin-2-yl]imidazolidine-2,4-dione;
(5R)-3-[5-[(3,3-dimethyl-2H-benzofuran-4-yl)oxy]pyrazin-2-yl]-5-ethyl-5-methylimidazolidine-2,4-dione;
5,5-dimethyl-3-[5-[(3,3,7-trimethyl-2H-benzofuran-4-yl)oxy]pyrazin-2-yl]imidazolidine-2,4-dione;
(5R)-5-ethyl-5-methyl-3-[5-[(3,3,7-trimethyl-2H-benzofuran-4-yl)oxy]pyrazin-2-yl]imidazolidine-2,4-dione;
(5R)-5-ethyl-3-[5-(7-methylspiro[2H-benzofuran-3,1'-cyclopropane]-4-yl)oxypyrazin-2-yl]imidazolidine-2,4-dione;
(5R)-5-ethyl-3-(5-spiro[2H-benzofuran-3,1'-cyclopropane]-4-yloxypyrazin-2-yl)imidazolidine-2,4-dione;
(5R)-3-[5-[(3,3-dimethyl-2H-benzofuran-4-yl)oxy]pyrazin-2-yl]-5-ethyl-imidazolidine-2,4-dione;
(5R)-5-ethyl-3-[5-[(3,3,7-trimethyl-2H-benzofuran-4-yl)oxy]pyrazin-2-yl]imidazolidine-2,4-dione;
7-[5-(7-methylspiro[2H-benzofuran-3,1'-cyclopropane]-4-yl)oxypyrazin-2-yl]-5,7-diazaspiro[3.4]octane-6,8-dione;

or a pharmaceutically acceptable salt and/or solvate thereof and/or derivative thereof of formula (Z).

In one embodiment, the compound of formula (I) is:
6-[5-(7-methylspiro[2H-benzofuran-3,1'-cyclopropane]-4-yl)oxypyrazin-2-yl]-4,6-diazaspiro[2.4]heptane-5, 7-dione;
or a pharmaceutically acceptable salt and/or solvate thereof and/or derivative thereof of formula (Z).

In one embodiment, the compound of formula (I) is:
(5S)-5-ethyl-3-[5-(7-methylspiro[2H-benzofuran-3,1'-cyclopropane]-4-yl)oxypyrazin-2-yl]imidazolidine-2,4-dione;
or a pharmaceutically acceptable salt and/or solvate thereof and/or derivative thereof of formula (Z).

When the compound contains a $C_{1-3}$alkyl group, whether alone or forming part of a larger group, the alkyl group may be straight chain, branched or cyclic. Examples of $C_{1-3}$alkyl are methyl, ethyl, n-propyl, isopropyl and cyclopropyl. Reference to "propyl" includes n-propyl, isopropyl and cyclopropyl.

The term 'halo' or 'halogen' as used herein, refers to a fluorine, chlorine, bromine or iodine atom. Particular examples of halo are fluorine, chlorine and bromine, such as chlorine or bromine.

The term '$C_{3-4}$ spiro carbocyclyl' as used herein means a cyclic ring system containing 3 or 4 carbon atoms, namely a cyclopropyl or cyclobutyl group, wherein the cyclic ring system is attached to a secondary carbon via a spirocentre such that the secondary carbon is one of the 3 to 4 carbon atoms in the cyclic ring as follows:

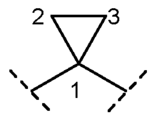

$C_3$ spiro carbocyclyl

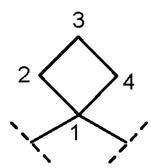

$C_4$ spiro carbocyclyl

It will be appreciated that for use in medicine the salts of the compounds of formula (I) should be pharmaceutically

acceptable. Suitable pharmaceutically acceptable salts will be apparent to those skilled in the art. Pharmaceutically acceptable salts include those described by Berge, Bighley and Monkhouse J.Pharm.Sci. (1977) 66, pp 1-19. Such pharmaceutically acceptable salts include acid addition salts formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Nonpharmaceutically acceptable salts may be used, for example, in the isolation of compounds of formula (I) and are included within the scope of this invention.

Certain of the compounds of formula (I) may form acid addition salts with one or more equivalents of the acid. The present invention includes within its scope all possible stoichiometric and nonstoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

A pharmaceutically acceptable prodrug, of formula (Z), is formed by functionalising the secondary nitrogen of the hydantoin, for example with a group "L" as illustrated below (wherein $R_4$ and $R_5$ are as described above):

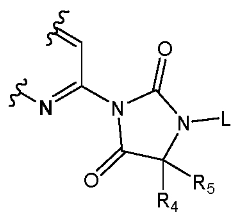

(Z)

wherein L is selected from:

a) $-PO(OH)O^{-}{\bullet}M^{+}$, wherein $M^{+}$ is a pharmaceutically acceptable monovalent counterion,
b) $-PO(O^{-})_2 {\bullet}2M^{+}$,
c) $-PO(O^{-})_2 {\bullet}D^{2+}$, wherein $D^{2+}$ is a pharmaceutically acceptable divalent counterion,
d) $-CH(R^X)-PO(OH)O^{-}{\bullet}M^{+}$, wherein $R^X$ is hydrogen or $C_{1-3}$ alkyl,
e) $-CH(R^X)-PO(O^{-})_2{\bullet}2M^{+}$,
f) $-CH(R^X)-PO(O^{-})_2{\bullet}D^{2+}$,
g) $-SO_3^{-}{\bullet}M^{+}$,
h) $-CH(R^X)-SO_3^{-}{\bullet}M^{+}$, and
i) $-CO-CH_2CH_2-CO_2{\bullet}M^{+}$.

It is to be understood that the present invention encompasses all geometric, tautomeric and optical forms, and mixtures thereof (e.g. racemic mixtures) isomers of formula (I) and their pharmaceutically acceptable derivatives thereof of formula (Z). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or > 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^{2}H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{31}Cl$), fluorine-18 ($^{18}F$) iodine-123 ($^{123}I$), iodine-125 ($^{125}I$) in one or more atoms or may contain an increased proportion

of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^3H$, and carbon-14, i.e. $^{14}C$, are particularly useful for this purpose in view of their ease of incorporation and ready means of detection. Unnatural variant isotopic forms which incorporate deuterium i.e. $^2H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^2H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the compounds of formula (I) may be made according to the organic synthesis techniques known to those skilled in this field, as well as by the representative methods set forth below, those in the Examples and modifications thereof.

Patent applications WO2011/069951, WO2012/076877, WO2012/168710, WO2013/175215, WO2013/083994, WO2013/182850, WO2017/103604, WO2018/020263 and WO2018/109484 provide methods for the synthesis of intermediates which may be of use in the production of compounds of the present invention.

General Synthesis Schemes

The following schemes detail synthetic routes to compounds of the invention and intermediates in the synthesis of such compounds. In the following schemes reactive groups can be protected with protecting groups and deprotected according to established techniques well known to the skilled person.

Compounds of formula (I), and salts and solvates thereof, may be prepared by the general methods outlined hereinafter. In the following description, the groups $R_1$, $R_2$, $R_3$, $R_4$ and $R_5$ have the meanings as previously defined for compounds of formula (I) unless otherwise stated.

Scheme 1a

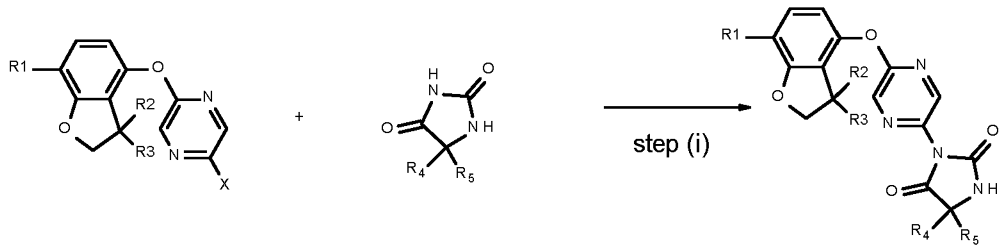


(II) (III) (I)

step (i): Compounds of formula (I) can be prepared by metal catalysed cross coupling reactions. In this reaction a halo-pyrazine derivative of formula (II) wherein typically X=Br and a hydantoin of formula (III) are reacted in the presence of a metal catalyst such as copper(I) oxide in a suitable solvent, e.g. in N,N-dimethylacetamide, with conventional heating or microwave heating.

Scheme 1b

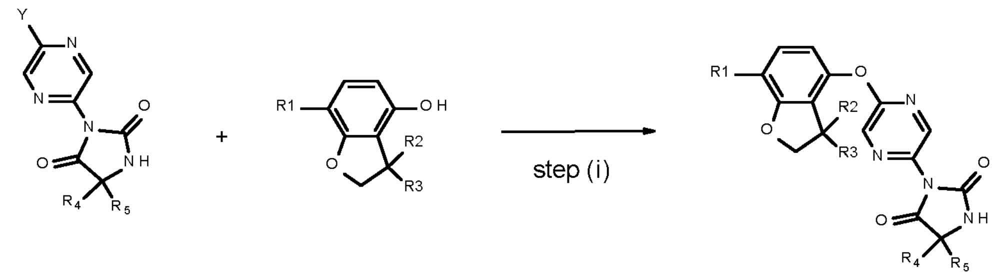


(IV) (V) (I)

Compounds of formula (I), wherein $R_4$ and $R_5$ are not H, can be prepared by nucleophilic aromatic substitution. In this reaction a halo-pyrazine derivative of formula (IV) wherein typically Y=Cl and a phenol of formula (V) are reacted in the presence of a suitable base such as potassium carbonate in a suitable solvent, e.g. in N,N-dimethylformamide or in acetonitrile, with conventional heating or microwave heating.

Scheme 1c

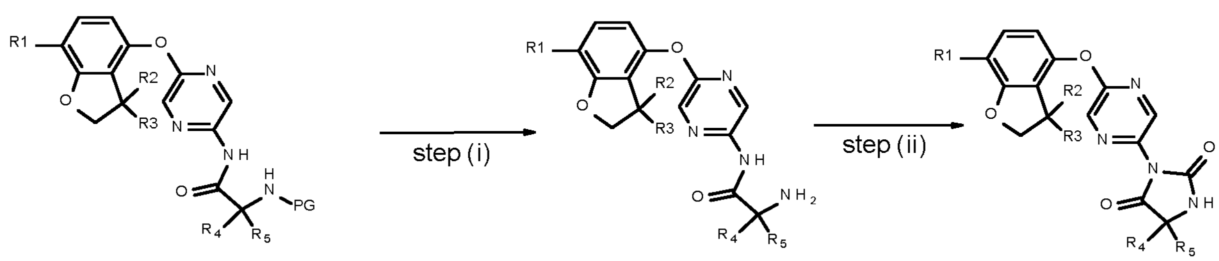


(VII) (VI) (I)

**step** (ii): Compounds of formula (I) can be prepared by cyclization of compounds of formula (VI) in a suitable solvent *e.g.* dichloromethane with a carbonylating agent *e.g.* triphosgene preferentially prediluted in the same solvent and added in a second time at 0°C in presence of a suitable base e.g. triethylamine. Alternatively compounds of formula (I) can be prepared by cyclization of compounds of formula (VI) using a carbonylating agent such as carbonyldiimidazole in a suitable solvent such as ethyl acetate in presence of a base such as triethylamine or DIPEA.

step (i): Compounds of formula (VI) can be prepared by deprotection of compounds of formula (VII) wherein PG is a protecting group, suitably the protecting group is BOC, BOC may be removed in acidic conditions *e.g.* TFA in a suitable solvent *e.g.* dichloromethane at approximately 0°C to room temperature.

Scheme 1d

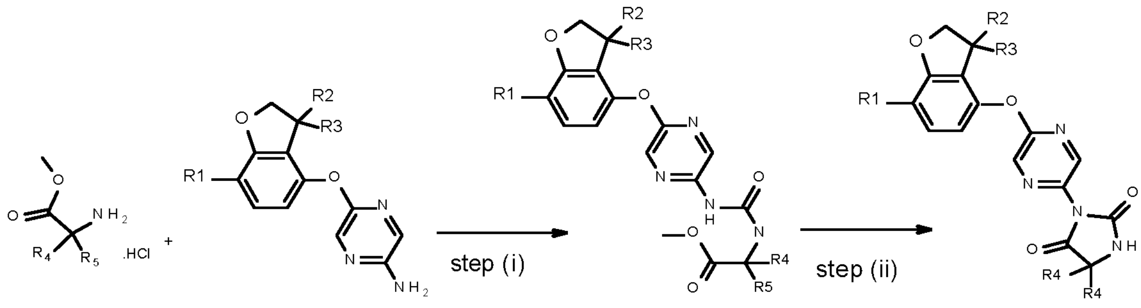


(IX) (XVI) (XVII) (I)

**step (ii):** Compounds of formula (I) can be prepared by reaction of ureas of formula (XVII) and a suitable base such as sodium methoxide in a suitable solvent such as methanol at temperature ranging from 0°C to room temperature.

**step (i):** Ureas of formula (XVII) can be prepared by reaction of anilines of formula (XVI) and amino esters (such as the hydrochloride salt) of formula (IX) in a suitable solvent e.g. dichloromethane or ethyl acetate with a carbonylating agent e.g. triphosgene preferentially prediluted in the same solvent in presence of a suitable base e.g. triethylamine or diisopropylethylamine at temperature ranging from 0°C to room temperature.

Scheme 2a

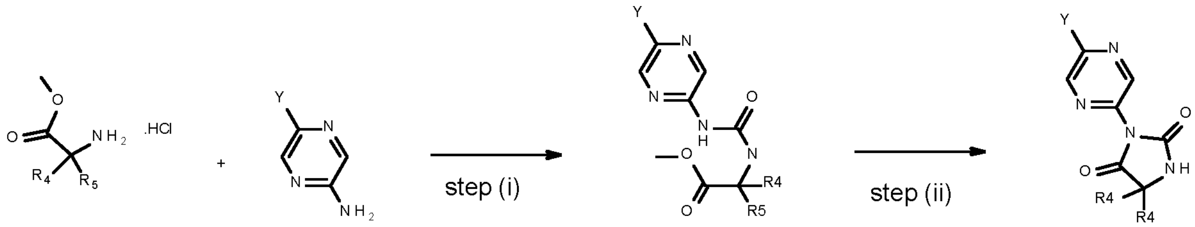


(IX) (X) (VIII) (IV)

**step (ii):** Compounds of formula (IV) can be prepared by reaction of ureas of formula (VIII) and a suitable base such as sodium methoxide in a suitable solvent such as methanol at temperature ranging from 0°C to room temperature.

**step (i):** Ureas of formula (VIII) can be prepared by reaction of commercially available halo-pyrazine derivative of formula (X), wherein typically Y=Cl, and amino esters (such as the hydrochloride salt) of formula (IX) in a suitable solvent *e.g.* dichloromethane or ethyl acetate with a carbonylating agent *e.g.* triphosgene preferentially prediluted in the same solvent in presence of a suitable base *e.g.* triethylamine or diisopropylethylamine at temperature ranging from 0°C to room temperature.

Scheme 2b

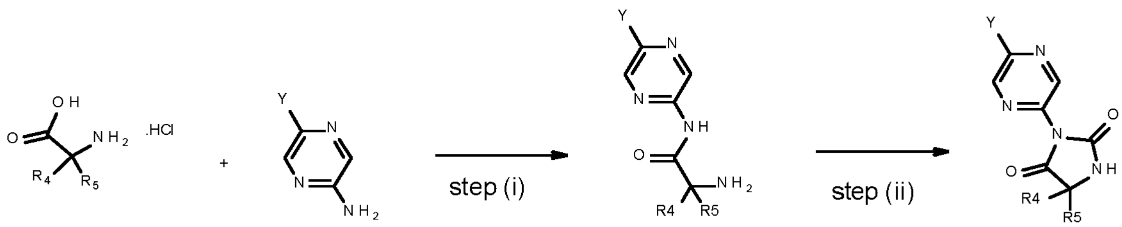


(XII) (X) (XI) (IV)

**step (ii):** Compounds of formula (IV) can be prepared by cyclization of compounds of formula (XI) in a suitable solvent *e.g.* dichloromethane with a carbonylating agent *e.g.* triphosgene preferentially prediluted in the same solvent and added in a second time at 0°C in presence of a suitable base e.g. triethylamine.

**step (i):** Compounds of formula (XI) can be prepared from anilines of formula (X), wherein typically Y=Cl, and amino acids (as free base or hydrochloride salt) of formula (XII) by amidic coupling in the presence of a coupling agent *e.g.* T3P in a suitable solvent such as ethyl acetate, acetonitrile or a mixture of them.

Scheme 3

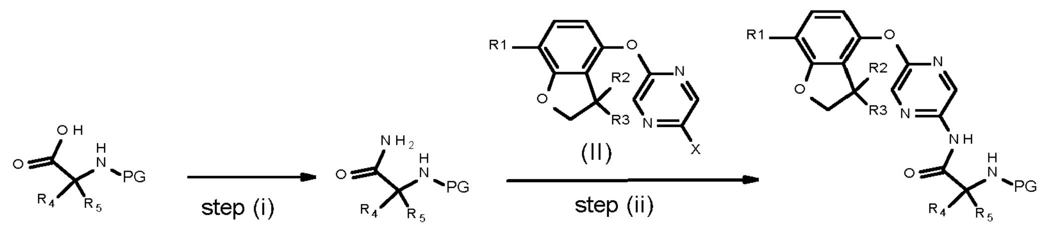


(XIV) (XIII) (II) (VII)

**step (ii):** Compounds of formula (IV) can be prepared by metal catalysed cross coupling reactions. In this reaction a halo-pyrazine derivative of formula (II) wherein typically X=Br and an amide of formula (XIII) are reacted in the presence of a metal catalyst such as Tris(dibenzylideneacetone)dipalladium(0), a suitable ligand such as dicyclohexyl-[2-(2,4,6-triisopropylphenyl)phenyl]phosphane (XPhos) and a suitable base such as cesium carbonate in a suitable solvent, *e.g.* in 1,4-dioxane, with conventional heating or microwave heating. Alternatively in this reaction a halo-pyrazine derivative of formula (II) wherein typically X=Br and an amide of formula (XIII) are reacted in the presence of a metal catalyst such as copper(I) iodide, a suitable ligand such as N,N'-dimethylethane-1,2-diamine and a suitable base such dipotassium carbonate in a suitable solvent, *e.g.* in 1-butanol, with conventional heating or microwave heating. A further alternative for the preparation of compounds of formula (IV) is to react a halo-pyrazine derivative of formula (II) wherein typically X=Br and an amide of formula (XIII) in the presence of a metal catalyst such as palladium (II) acetate, a suitable ligand such as Xantphos and a suitable base such as cesium carbonate in a suitable solvent, *e.g.* in 1,4-dioxane, with conventional heating or microwave heating.

**step (i):** Compounds of formula (XIII) can be prepared from N-protected (e.g. BOC) amino acids of formula (XIV) and an amine such as hexamethyldisilazane by amidic coupling in the presence of a base *e.g.* DIPEA and of a coupling agent *e.g.* HATU or TBTU in a solvent such as N,N-dimethylformamide.

Scheme 4

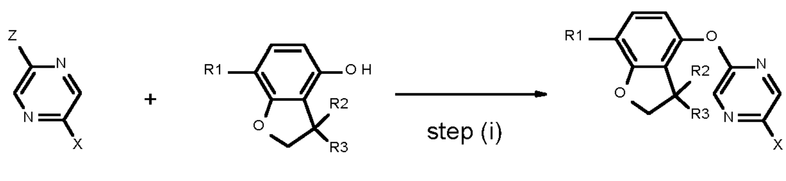


(XV) (V) (II)

**step (i):** Compounds of formula (II) wherein typically X=Br can be prepared by nucleophilic aromatic substitution. In this reaction a halo-pyrazine derivative of formula (XV) wherein typically X=Z=Br and a phenol of formula (V) are reacted in the presence of a base such as potassium carbonate in a suitable solvent, *e.g.* in N,N-dimethylformamide, with conventional heating or microwave heating.

Scheme 5

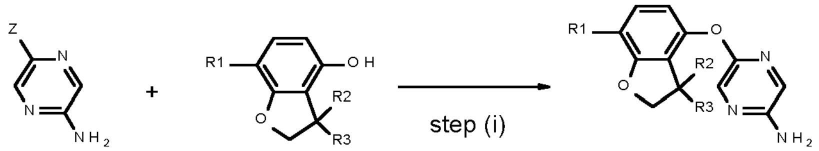


(XVIII) (V) (XVI)

**step (i):** Anilines of formula (XVI) can be prepared by metal catalysed cross coupling reactions. In this reaction a halo-pyrazine derivative of formula (XVIII) wherein typically Z=Br and a phenol of formula (V) are reacted in the presence of a metal catalyst such as Copper(I)Iodide, a suitable ligand like picolinic acid, in a suitable solvent, *e.g.* in N,N-dimethylformamide or N,N-dimethylacetamide, with conventional heating or microwave heating optionally a suitable base such as potassium carbonate or caesium carbonate can be used.

Scheme 6

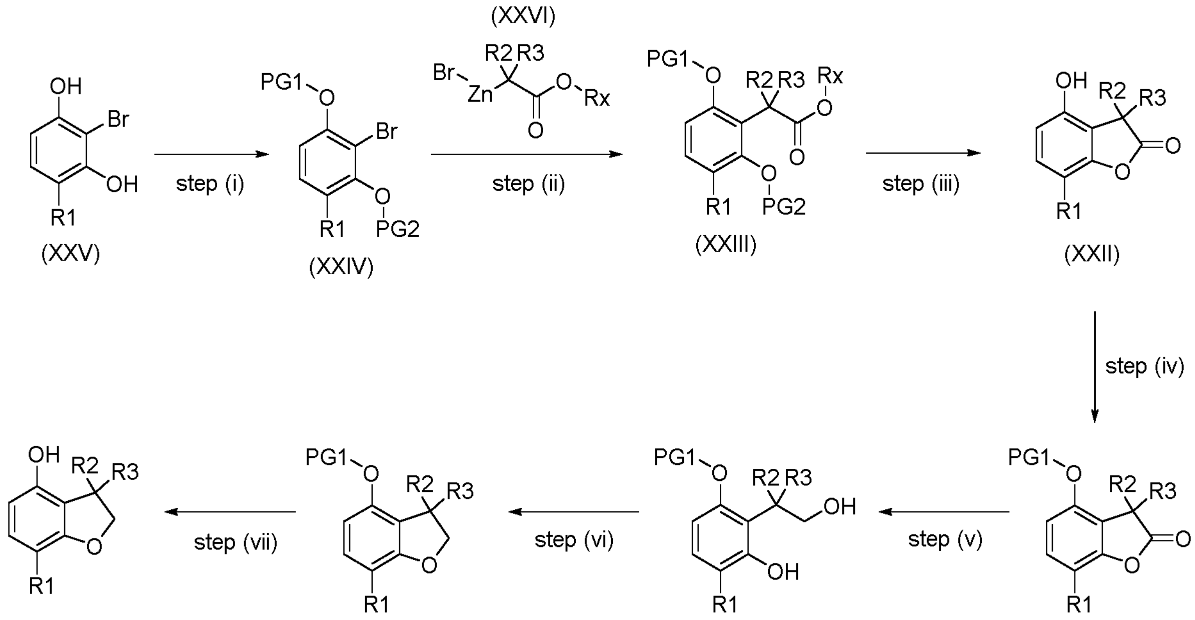


(XXV) (XXIV) (XXVI) (XXIII) (XXII) (XXI) (XX) (XIX) (V)

In Scheme 6 shown above, $PG_1$ and $PG_2$ represent suitable protecting groups. $PG_1$ in steps (i)=-(iii) may be different from $PG_1$ in Steps (iv)-(vii). Suitable protecting groups include benzyl. tetrahydropyranyl or methyloxymethyl. Suitably $PG_2$ is the same as $PG_1$, e.g. both are benzyl.

**Description of the scheme wherein $PG_1$ and $PG_2$ are both benzyl**

**step (vii):** Phenols of formula (V) can be prepared from the benzylated compounds of formula (XIX), by deprotection such as using a metal catalyst such as palladium on carbon and a hydrogen source such as hydrogen atmosphere or ammonium formate in a suitable solvent such as ethanol or methanol at a temperature ranging from room temperature to reflux.

**step (vi):** Benzylated compounds of formula (XIX) can be prepared from diols of formula (XX) using a base such as potassium tert-butoxide and a suitable solvent such as dimethyl carbonate at a temperature ranging from room temperature to reflux.

**step (v):** Diols of formula (XX) can be prepared from lactones of formula (XXI) using a reducing agent such as lithium aluminium hydride in a suitable solvent such as THF at a temperature ranging from 0°C to room temperature.

**step (iv):** Lactones of formula (XXI) can be prepared from phenols of formula (XXII) using a benzylating agent

such as benzyl bromide in presence of a base such as potassium carbonate in a suitable solvent such as acetonitrile or THF or a mixture thereof at a temperature ranging from room temperature to reflux.

step (iii): Phenols of formula (XXII) can be prepared from di-benzylated esters of formula (XXIII) wherein Rx is a suitable alkylic group such as methyl or ethyl, using a metal catalyst such as palladium on carbon and a hydrogen source such as hydrogen atmosphere or ammonium formate in a suitable solvent such as ethanol or methanol at a temperature ranging from room temperature to reflux.

step (ii): Di-benzylated esters of formula (XXIII) wherein Rx is a suitable alkylic group such as methyl or ethyl can be prepared from di-benzylated bromo derivatives of formula (XXIV) by using pre-formed organozinc derivatives of formula (XXVI) wherein Rx is a suitable alkylic group such as methyl or ethyl in presence of a metal catalyst complex such as Bis(tri-tert-butylphosphine)palladium(0) in a suitable solvent such as THF or DMF or a mixture thereof at a temperature ranging from room temperature to reflux.

step (i): Di-benzylated bromo derivatives of formula (XXIV) can be prepared from commercially available derivatives of formula (XXV) using a benzylating agent such as benzyl bromide in presence of a base such as potassium carbonate in a suitable solvent such as acetonitrile or THF or acetone or a mixture thereof at a temperature ranging from room temperature to reflux.

When $PG_1$ and/or $PG_2$ are protecting groups such as tetrahydropyranyl or methyloxymethyl, usual protection/deprotection conditions apply:

- Protection conditions of phenols with tetrahydropyranyl include the reaction of a phenol with dihydro-2H-pyran in presence of a catalyst such C:Py •p-$MePhSO_3H$ in a suitable solvent such us dichloromethane at a temperature ranging from 0°C to reflux.
- Cleavage conditions for a tetrahydropyranyl protecting group from phenols include the reaction of a THP protected phenol in presence of an acid such as sulphuric acid or p-$MePhSO_3H$ or HCl in a suitable solvent such us methanol or ethanol at a temperature ranging from 0oC to reflux.
- Protection conditions of phenols with methyloxymethyl include the reaction of a phenol with chloromethyl methyl ether in presence of a base such us potassium carbonate in a suitable solvent such us tetrahydrofuran or acetonitrile at a temperature ranging from 0°C to reflux.
- Cleavage conditions for a methyloxymethyl protecting group from phenols include the reaction of a MOM protected phenol in presence of an acid such as sulphuric acid or p-$MePhSO_3H$ or HCl in a suitable solvent such us methanol or ethanol at a temperature ranging from 0°C to reflux.

Scheme 7

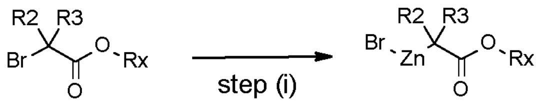


(XXVII) (XXVI)

Step (i): Organozinc derivatives of formula (XXVI) wherein Rx is a suitable alkylic group such as methyl or ethyl can be prepared by adding commercially available bromo esters of formula (XXVII) to a refluxing suspension of zinc (0) in presence of 1,2-dibromoethane and chlorotrimethylsilane in a suitable solvent such as THF.

Intermediates

Intermediates of the invention include:

- compounds of formula (II):

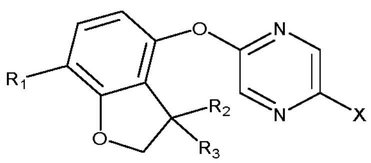

wherein $R_1$, $R_2$ and $R_3$ are as defined previously, X is halo, such as Br;

- compounds of formula (IV):

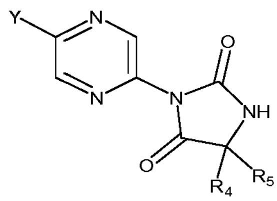

wherein $R_1$, $R_2$ and $R_3$ are as defined previously, Y is halo, such as Cl;

- compounds of formula (XVI):

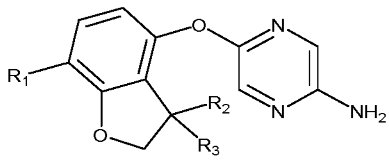

wherein $R_1$, $R_2$ and $R_3$ are as defined previously.

Salts, such as pharmaceutically acceptable salts, of such intermediates are also provided by the present invention.

Kv3.1, Kv3.2 and/or Kv3.3 modulation

Compounds of formula (I) of the present invention are modulators of Kv3.1. Compounds of formula (I) may also be modulators of Kv3.2 and/or Kv3.3. Compounds of the invention may be tested in the assay of Biological Example 1 to determine their modulatory properties for Kv3.1 and/or Kv3.2 and/or Kv3.3 channels.

A 'modulator' as used herein refers to a compound which is capable of producing at least 10% potentiation, and suitably at least 20% potentiation of whole-cell currents mediated by human Kv3.1 and/or human Kv3.2 and/or human Kv3.3 channels recombinantly expressed in mammalian cells.

The term 'Kv3.1, Kv3.2 and/or Kv3.3' shall be taken to mean the same as 'Kv3.1 and/or Kv3.2 and/or Kv3.3' and may also be referred to as 'Kv3.1/Kv3.2/Kv3.3'.

In one embodiment the modulator is capable of producing at least 10% potentiation and suitably at least 20% potentiation of whole-cell currents mediated by human Kv3.1 channels recombinantly expressed in mammalian cells. Suitably the $pEC_{50}$ of the modulator is in the range of 4-7 (such as 5-6.5).

In one embodiment the modulator is capable of producing at least 10% potentiation and suitably at least 20% potentiation of whole-cell currents mediated by human Kv3.2 channels recombinantly expressed in mammalian cells. Suitably the $pEC_{50}$ of the modulator is in the range of 4-7 (such as 5-6.5).

In one embodiment the modulator is capable of producing at least 10% potentiation and suitably at least 20% potentiation of whole-cell currents mediated by human Kv3.3 channels recombinantly expressed in mammalian cells. Suitably the $pEC_{50}$ of the modulator is in the range of 4-7 (such as 5-6.5).

In another embodiment the modulator is capable of producing at least 10% potentiation and suitably at least 20% potentiation of whole-cell currents mediated by human Kv3.1 and Kv3.2 channels recombinantly expressed in mammalian cells.

In another embodiment the modulator is capable of producing at least 10% potentiation and suitably at least 20% potentiation of whole-cell currents mediated by human Kv3.1 and Kv3.3 channels recombinantly expressed in mammalian cells.

In another embodiment the modulator is capable of producing at least 10% potentiation and suitably at least 20% potentiation of whole-cell currents mediated by human Kv3.2 and Kv3.3 channels recombinantly expressed in mammalian cells.

In a further embodiment the modulator is capable of producing at least 10% potentiation and suitably at least 20% potentiation of whole-cell currents mediated by human Kv3.1, Kv3.2 and Kv3.3 channels recombinantly expressed in mammalian cells.

The compounds of formula (I) or their pharmaceutically acceptable salts and/or solvates thereof and/or derivatives thereof of formula (Z) may be of use for the treatment or prophylaxis of a disease or disorder where a modulator of the Kv3.1 or Kv3.2 or Kv3.1 and Kv3.2 channels is required. As used herein, a modulator of Kv3.1 or Kv3.2 or Kv3.1 and Kv3.2 is a compound which alters the properties of these channels, either positively or negatively. In a particular

aspect of the invention, the compound of formula (I) is a positive modulator. Compounds of the invention may be tested in the assay of Biological Example 1 to determine their modulatory properties.

In one embodiment of the invention the compounds of formula (I) or their pharmaceutically acceptable salts and/or solvates thereof and/or derivatives thereof of formula (Z) are selective for modulation of Kv3.1 channels over modulation of Kv3.2 channels. By selective, is meant that compounds demonstrate, for example, at least a 2 fold, 5 fold or 10 fold activity for Kv3.1 channels than for Kv3.2 channels. The activity of a compound is suitably quantified by its potency as indicated by an Ec50 value.

In another embodiment of the invention, the compounds of formula (I) or their pharmaceutically acceptable salts and/or solvates thereof and/or derivatives thereof of formula (Z) are selective for modulation of Kv3.2 channels over modulation of Kv3.1 channels. Once again, by selective is meant that compounds demonstrate, for example at least a 2 fold, 5 fold or 10 fold activity for Kv3.2 channels than for Kv3.1 channels.

In a particular embodiment of the invention the compounds of formula (I) or their pharmaceutically acceptable salts and/or solvates thereof and/or derivatives thereof of formula (Z) demonstrate comparable activity between modulation of Kv3.1 and Kv3.2 channels, for example the activity for one channel is less than 2 fold that for the other channel, such as less than 1.5 fold or less than 1.2 fold.

In certain disorders it may be of benefit to utilise a modulator of Kv3.3 or Kv3.1, or Kv3.3 and Kv3.1 which demonstrates a particular selectivity profile between the two channels. For example a compound may be selective for modulation of Kv3.3 channels over modulation of Kv3.1 channels demonstrating, for example, at least a 2 fold, 5 fold or 10 fold activity for Kv3.3 channels than for Kv3.1 channels.

In another embodiment of the invention, the compounds of formula (I) or their pharmaceutically acceptable salts and/or solvates thereof and/or derivatives thereof of formula (Z) are selective for modulation of Kv3.1 channels over modulation of Kv3.3 channels. Once again, by selective is meant that compounds demonstrate, for example at least a 2 fold, 5 fold or 10 fold activity for Kv3.1 channels than for Kv3.3 channels.

In a particular embodiment of the invention, a compound may demonstrate comparable activity between modulation of Kv3.3 and Kv3.1 channels, for example the activity for each channel is less than 2 fold that for the other channel, such as less than 1.5 fold or less than 1.2 fold.

In certain disorders it may be of benefit to utilise a modulator of Kv3.3 or Kv3.2, or Kv3.3 and Kv3.2 which demonstrates a particular selectivity profile between the two channels. A compound may be selective for modulation of Kv3.3 channels over modulation of Kv3.2 channels demonstrating, for example, at least a 2 fold, 5 fold or 10 fold activity for Kv3.3 channels than for Kv3.2 channels.

In another embodiment of the invention, the compounds of formula (I) or their pharmaceutically acceptable salts and/or solvates thereof and/or derivatives thereof of formula (Z) are selective for modulation of Kv3.2 channels over modulation of Kv3.3 channels. Once again, by selective is meant that compounds demonstrate, for example at least a 2 fold, 5 fold or 10 fold activity for Kv3.2 channels than for Kv3.3 channels.

In another particular embodiment a compound may demonstrate comparable activity between modulation of Kv3.3 and Kv3.2 channels, for example the activity for each channel is less than 2 fold that for the other channel, such as less than 1.5 fold or less than 1.2 fold.

In a yet further particular embodiment of the invention a compound may demonstrate comparable activity between modulation of Kv3.3, Kv3.2 and Kv3.1 channels, for example the activity for each channel is less than 2 fold that for any other channel, such as less than 1.5 fold or less than 1.2 fold. The activity of a compound is suitably quantified by its potency as indicated by an EC50 value.

## Therapeutic methods

The invention also provides a compound of formula (I), or a pharmaceutically acceptable salt and/or solvate (e.g. salt) thereof and/or derivative thereof of formula (Z), for use in the treatment or prophylaxis of a disease or disorder where a modulator of Kv3.1, Kv3.2 and/or Kv3.3 is required, for example those diseases and disorders mentioned herein below.

In one embodiment is provided a compound of formula (I) or a pharmaceutically acceptable salt and/or solvate thereof and/or derivative thereof of formula (Z) for use as a medicament.

The term "treatment" or "treating" as used herein includes the control, mitigation, reduction, or modulation of the disease state or its symptoms.

The term "prophylaxis" is used herein to mean preventing symptoms of a disease or disorder in a subject or preventing recurrence of symptoms of a disease or disorder in an afflicted subject and is not limited to complete prevention of an affliction.

Suitably the subject is a human.

Diseases or disorders that may be mediated by modulation of Kv3.1 and/or Kv3.2 channels may be selected from the list below. The numbers in brackets after the listed diseases below refer to the classification code in Diagnostic

and Statistical Manual of Mental Disorders, 4th Edition, published by the American Psychiatric Association (DSM-IV) and/or the International Classification of Diseases, 10th Edition (ICD-10).

In one embodiment of the invention, the compounds of formula (I) or their pharmaceutically acceptable salts and/or solvates thereof and/or derivatives thereof of formula (Z) may be of use for the treatment or prophylaxis of a disease or disorder selected from the group consisting of hearing disorders, schizophrenia, depression and mood disorders, bipolar disorder, substance abuse disorders, anxiety disorders, sleep disorders, hyperacusis and disturbances of loudness perception, Ménière's disease, disorders of balance, and disorders of the inner ear, impulse control disorder, personality disorders, attention-deficit/hyperactivity disorder, autism spectrum disorders, eating disorders, cognition impairment, ataxia, pain such as neuropathic pain, inflammatory pain and miscellaneous pain, Lewy body dementia and Parkinson's disease.

In one embodiment of the invention, the compounds of formula (I) or their pharmaceutically acceptable salts and/or solvates thereof and/or derivatives thereof of formula (Z) may be of use for the treatment or prophylaxis of a disease or disorder selected from the group consisting of hearing disorders including hearing loss and tinnitus, schizophrenia, substance abuse disorders, pain such as neuropathic pain, inflammatory pain and miscellaneous pain, Lewy body dementia and Parkinson's disease.

In one embodiment of the invention, the compounds of formula (I) or their pharmaceutically acceptable salts and/or solvates thereof and/or derivatives thereof of formula (Z) may be of use for the treatment or prophylaxis of a disease or disorder selected from the group consisting of Fragile-X, Rett's Disorder and Alzheimer's disease.

In a particular embodiment of the invention, there is provided a compound of formula (I) or their pharmaceutically acceptable salts and/or solvates (e.g. salts) thereof and/or derivatives thereof of formula (Z) for use in the treatment of prophylaxis of hearing disorders. Hearing disorders include auditory neuropathy, auditory processing disorder, hearing loss, which includes sudden hearing loss, noise induced hearing loss, substance-induced hearing loss, and hearing loss in adults over 60, over 65, over 70 or over 75 years of age (presbycusis), and tinnitus.

The compounds of formula (I) or their pharmaceutically acceptable salts and/or solvates (e.g. salts) thereof and/or derivatives thereof of formula (Z) may be of use for the treatment or prophylaxis of Ménière's disease, disorders of balance, and disorders of the inner ear.

In a particular embodiment of the invention, there is provided a compound of formula (I) or their pharmaceutically acceptable salts and/or solvates (e.g. salts) thereof and/or derivatives thereof of formula (Z) for use in the treatment or prophylaxis of schizophrenia. Schizophrenia includes the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

The compounds of formula (I) or their pharmaceutically acceptable salts and/or solvates (e.g. salts) thereof and/or derivatives thereof of formula (Z) may be of use for the treatment or prophylaxis of depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90); Seasonal affective disorder.

The compounds of formula (I) or their pharmaceutically acceptable salts and/or solvates (e.g. salts) thereof and/or derivatives thereof of formula (Z) may be of use for the treatment or prophylaxis of Epilepsy, (including, but not limited to, localization-related epilepsies, generalized epilepsies, epilepsies with both generalized and local seizures, and the like), seizures associated with Lennox-Gastaut syndrome, seizures as a complication of a disease or condition (such as seizures associated with encephalopathy, phenylketonuria, juvenile Gaucher's disease, Lundborg's progressive myoclonic epilepsy, stroke, head trauma, stress, hormonal changes, drug use or withdrawal, alcohol use or withdrawal, sleep deprivation, fever, infection, and the like), essential tremor, restless limb syndrome, partial and generalised seizures (including tonic, clonic, tonic-clonic, atonic, myoclonic, absence seizures), secondarily generalized seizures, temporal lobe epilepsy, absence epilepsies (including childhood, juvenile, myoclonic, photo- and patterninduced), severe epileptic encephalopathies (including hypoxia-related and Rasmussen's syndrome), febrile convulsions, epilepsy partialis continua, progressive myoclonus epilepsies (including Unverricht-Lundborg disease and Lafora's disease), post-traumatic seizures/epilepsy including those related to head injury, simple reflex epilepsies (including photosensive, somatosensory

and proprioceptive, audiogenic and vestibular), metabolic disorders commonly associated with epilepsy such as pyridoxine-dependent epilepsy, Menkes' kinky hair disease, Krabbe's disease, epilepsy due to alcohol and drug abuse (e.g. cocaine), cortical malformations associated with epilepsy (e.g. double cortex syndrome or subcortical band heterotopia), chromosomal anomalies associated with seizures or epilepsy such as Partial monosomy (15Q) / Angelman syndrome).

The compounds of formula (I) or their pharmaceutically acceptable salts and/or solvates (e.g. salts) thereof and/or derivatives thereof of formula (Z) may be of use for the treatment or prophylaxis of substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide.

The compounds of formula (I) or their pharmaceutically acceptable salts and/or solvates (e.g. salts) thereof and/or derivatives thereof of formula (Z) may be of use for the treatment or prophylaxis of anxiety disorders including Panic Attack; Panic Disorder including Panic Disorder without Agoraphobia (300.01) and Panic Disorder with Agoraphobia (300.21); Agoraphobia; Agoraphobia Without History of Panic Disorder (300.22), Specific Phobia (300.29, formerly Simple Phobia) including the subtypes Animal Type, Natural Environment Type, Blood-Injection-Injury Type, Situational Type and Other Type), Social Phobia (Social Anxiety Disorder, 300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder, Separation Anxiety Disorder (309.21), Adjustment Disorders with Anxiety (309.24) and Anxiety Disorder Not Otherwise Specified (300.00).

The compounds of formula (I) or their pharmaceutically acceptable salts and/or solvates (e.g. salts) thereof and/or derivatives thereof of formula (Z) may be of use for the treatment or prophylaxis of sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition, in particular sleep disturbances associated with such diseases as neurological disorders, neuropathic pain, restless leg syndrome, heart and lung diseases; and Substance-Induced Sleep Disorder including the subtypes Insomnia Type, Hypersomnia Type, Parasomnia Type and Mixed Type; sleep apnea and jet-lag syndrome.

The compounds of formula (I) or their pharmaceutically acceptable salts and/or solvates (e.g. salts) thereof and/or derivatives thereof of formula (Z) may be of use for the treatment or prophylaxis of hyperacusis and disturbances of loudness perception, including Fragile-X syndrome and autism.

The compounds of formula (I) or their pharmaceutically acceptable salts and/or solvates (e.g. salts) thereof and/or derivatives thereof of formula (Z) may be of use for the treatment or prophylaxis of Impulse control disorder including: Intermittent Explosive Disorder (312.34), Kleptomania (312.32), Pathological Gambling (312.31), Pyromania (312.33), Trichotillomania (312.39), Impulse-Control Disorders Not Otherwise Specified (312.3), Binge Eating, Compulsive Buying, Compulsive Sexual Behaviour and Compulsive Hoarding.

The compounds of formula (I) or their pharmaceutically acceptable salts and/or solvates (e.g. salts) thereof and/or derivatives thereof of formula (Z) may be of use for the treatment or prophylaxis of Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

The compounds of formula (I) or their pharmaceutically acceptable salts and/or solvates (e.g. salts) thereof and/or derivatives thereof of formula (Z) may be of use for the treatment or prophylaxis of Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9).

The compounds of formula (I) or their pharmaceutically acceptable salts and/or solvates (e.g. salts) thereof and/or derivatives thereof of formula (Z) may be of use for the treatment or prophylaxis of Attention-Deficit/Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23).

The compounds of formula (I) or their pharmaceutically acceptable salts and/or solvates (e.g. salts) thereof and/or derivatives thereof of formula (Z) may be of use for the treatment or prophylaxis of Autism Spectrum Disorders including Autistic Disorder (299.00), Asperger's Disorder (299.80), Rett's Disorder (299.80), Childhood Disintegrative Disorder (299.10) and Pervasive Disorder Not Otherwise Specified (299.80, including Atypical Autism).

The compounds of formula (I) or their pharmaceutically acceptable salts and/or solvates (e.g. salts) thereof and/or derivatives thereof of formula (Z) may be of use for the treatment or prophylaxis of eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder; Binge Eating Disorder; and Eating Disorder Not Otherwise Specified (307.50).

The compounds of formula (I) or their pharmaceutically acceptable salts and/or solvates (e.g. salts) thereof

and/or derivatives thereof of formula (Z) may be of use for the enhancement of cognition including the treatment of cognition impairment in other diseases such as schizophrenia, bipolar disorder, depression, other psychiatric disorders and psychotic conditions associated with cognitive impairment, e.g. Alzheimer's disease. Alternatively, the compounds of formula (I) or their pharmaceutically acceptable salts and/or solvates thereof may be of use for the prophylaxis of cognition impairment, such as may be associated with in diseases such as schizophrenia, bipolar disorder, depression, other psychiatric disorders and psychotic conditions associated with cognitive impairment, e.g. Alzheimer's disease.

The compounds of formula (I) or their pharmaceutically acceptable salts and/or solvates (e.g. salts) thereof and/or derivatives thereof of formula (Z) may be of use for the treatment or prophylaxis of ataxia including ataxia, in particular spinocerebellar ataxia, especially ataxia associated with R420H, R423H or F448L mutations.

The compounds of formula (I) or their pharmaceutically acceptable salts and/or solvates (e.g. salts) thereof and/or derivatives thereof of formula (Z) may be of use for the treatment or prophylaxis of pain including nociceptive, neuropathic, inflammatory or miscellaneous pain.

Nociceptive pain represents the normal response to noxious insult or injury of tissues such as skin, muscles, visceral organs, joints, tendons, or bones. Examples of nociceptive pain which form part of the invention include somatic pain: musculoskeletal (joint pain, myofascial pain) or cutaneous, which is often well localized; or visceral pain: hollow organs or smooth muscle.

Neuropathic pain is pain initiated or caused by a primary lesion or disease in the somatosensory nervous system. Sensory abnormalities range from deficits perceived as paraesthesia (numbness) to hypersensitivity (hyperalgesia or allodynia), and dysaesthesia (tingling and other sensations). Examples of neuropathic pain which form part of the invention include, but are not limited to, diabetic neuropathy, post-herpetic neuralgia, spinal cord injury pain, phantom limb (post-amputation) pain, and post-stroke central pain. Other causes of neuropathic pain include trauma, chemotherapy and heavy metal exposure.

Inflammatory pain occurs as a result of activation and sensitization of the nociceptive pain pathway by a variety of mediators released at a site of tissue inflammation. Mediators that have been implicated as key players in inflammatory pain are pro-inflammatory cytokines such IL-1-alpha, IL-1-beta, IL-6 and TNF-alpha, chemokines, reactive oxygen species, vasoactive amines, lipids, ATP, acid, and other factors released by infiltrating leukocytes, vascular endothelial cells, or tissue resident mast cells. Examples causes of inflammatory pain which form part of the invention include appendicitis, rheumatoid arthritis, inflammatory bowel disease, and herpes zoster.

Miscellaneous pain refers to pain conditions or disorders which are not easily classifiable. The current understanding of their underlying mechanisms is still rudimentary though specific therapies for those disorders are well known; they include cancer pain, migraine and other primary headaches and wide-spread pain of the fibromyalgia type.

Suitably, specific pain indications that may be mediated by a modulator of Kv3.1 and/or Kv3.2 and/or Kv3.3 channels are neuropathic pain and/or inflammatory pain.

Pain is a subjective condition and in a clinical setting tends to be measured by a patient's selfassessment. Therefore it can be difficult to measure and quantify pain threshold. For chronic pain, typically a subjective 11-point rating scale is used where 0 is no pain and 10 is the worst pain imaginable. Subjects generally record their worst pain over a given period, usually a day. A minimum mean baseline score is also recorded and response to the medication is measured relative to the baseline, for example, a reduction of at least 10%, 20%, 30%, 40% or 50% in pain from the baseline score may be observed.

Since individual responses to medicaments may vary, not all individuals may experience a reduction in pain from the baseline score. Consequently, suitably a reduction is observed in at least at least 10%, 20%, 30%, 40%, 50%, 60%, 70%, 80%, 90% or all individuals tested.

Therefore, in one embodiment of the invention, a reduction of at least 10%, 20%, 30%, 40% or 50% in pain from the baseline score is observed upon administration of a Kv3.1/Kv3.2/Kv3.3 modulator, such as a compound of formula (I) or a pharmaceutically acceptable salt and/or solvate thereof and/or derivative thereof of formula (Z) to a subject in need thereof.

Administration of a Kv3.1/Kv3.2/Kv3.3 modulator can occur before an anticipated onset of pain or after the onset of pain. In cases where it is anticipated that development of a disease or disorder may lead to an increase in pain experienced by the subject, a Kv3.1/Kv3.2/Kv3.3 modulator, such as a compound of formula (I) or a pharmaceutically acceptable salt and/or solvate thereof and/or derivative thereof of formula (Z) can be administered. In cases where a subject is already experiencing pain, a Kv3.1/Kv3.2/Kv3.3 modulator, such as a compound of formula (I) or a pharmaceutically acceptable salt and/or solvate thereof and/or derivative thereof of formula (Z) may be administered to a subject in need thereof.

Treatment of the subject in need thereof may continue for as long as treatment is required, for example, 1 day, 1 week, 2 weeks, 3 weeks, 1 month, 6 months, 1 year, more than 1 year more than 2 years, more than 5 years or more than 10 years. Therefore in one embodiment of the invention, a therapeutically effective amount of a Kv3.1/Kv3.2/Kv3.3 modulator, such as a compound of formula (I) or a pharmaceutically acceptable salt and/or solvate thereof and/or derivative thereof of formula (Z), is administered to a subject in need thereof for 1 day to 1 month, 1 week to 3 months,

1 month to 6 months, 3 months to 1 year or more than 1 year.

Reduction in pain in a subject can be measured by assessing the response to an external stimuli such as mechanical or thermal (e.g. cold) stimuli (such as described in the Experimental section). The reduction can either be considered as a percentage reversal (calculated by measuring the pre- and post-dose thresholds of the affected pain site with a non-affected pain site, such as described in more detail under Data Analysis in the Experimental Section) or by measuring withdrawal thresholds of the affected pain site. Preferably, the percentage reversal calculation is used.

Therefore, in one embodiment of the invention, the sensitivity to pain (such as neuropathic pain or inflammatory pain) is reversed by more than 20%, more than 30%, more than 40%, more than 50%, more than 60%, more than 70%, more than 80% or more than 90%, upon administration of a therapeutically effective amount of a Kv3.1/Kv3.2/Kv3.3 modulator, such as a compound of formula (I) or a pharmaceutically acceptable salt and/or solvate thereof and/or derivative thereof of formula (Z). Suitably, the sensitivity to pain is reversed by more than 80% or more than 90%.

Subjects receiving the Kv3.1/Kv3.2/Kv3.3 modulator may experience secondary benefits, such as one or more of improved function, mood, sleep, quality of life, reduced time off work.

In a particular embodiment, the compounds of formula (I) or their pharmaceutically acceptable salts and/or solvates (e.g. salts) thereof and/or derivatives thereof of formula (Z) may be of use for the treatment or prophylaxis of neuropathic pain.

In a particular embodiment, the compounds of formula (I) or their pharmaceutically acceptable salts and/or solvates (e.g. salts) thereof and/or derivatives thereof of formula (Z) may be of use for the treatment or prophylaxis of inflammatory pain.

In a particular embodiment, the compounds of formula (I) or their pharmaceutically acceptable salts and/or solvates (e.g. salts) thereof and/or derivatives thereof of formula (Z) may be of use for the treatment or prophylaxis of miscellaneous pain.

In one embodiment is provided a compound of formula (I) for use in the prophylaxis of acute noise-induced hearing loss.

Acute noise-induced hearing loss may be caused by events such as exposure to loud noise or a blast. In these cases, where it is anticipated that a future event may result in acute noise-induced hearing loss, the compound of formula (I) or a pharmaceutically acceptable salt and/or solvate thereof and/or derivative thereof of formula (Z) may be administered before the event in order to prevent or reduce acute noise-induced hearing loss. The administration of compound (I) or a pharmaceutically acceptable salt and/or solvate thereof and/or derivative thereof of formula (Z) may prevent any acute noise-induced hearing loss, or may reduce the severity of the acute noise-induced hearing loss or may mitigate other symptoms arising from acute noise-induced hearing loss, such as tinnitus.

"Acute hearing loss" is defined as hearing loss which occurs rapidly over a period of hours or days. For example, hearing loss may occur over a period of minutes, hours or days (for example over a period of up to 1 day, such as up to 2 days, 3 days, 4 days, 5 days, 6 days or 7 days). Acute hearing loss will typically be caused by exposure to loud sound or blast. Hearing loss caused by exposure to loud sound or blast is referred to herein as "noise-induced induced hearing loss". "Acute noise induced hearing loss" is therefore hearing loss which occurs rapidly over a period of hours or days caused by exposure to loud sound or blast.

Important symptoms of acute hearing loss include:

1. a shift in the auditory threshold, i.e. an increase in the minimum sound level of a pure tone that can be heard with no other sound present;
2. tinnitus; and
3. degradation in central auditory processing, for example impaired auditory temporal processing and/or speech understanding.

A "loud" noise or blast may be at least 90dB, for example, at least 100dB, at least 110dB, at least 120 dB or at least 130 dB.

In one embodiment, administration of the compound of formula (I) or a pharmaceutically acceptable salt and/or solvate thereof and/or derivative thereof of formula (Z) is initiated before an event which is anticipated to cause noise-induced acute hearing loss. For example, administration of the compound of formula (I) or a pharmaceutically acceptable salt and/or solvate thereof and/or derivative thereof of formula (Z) may be initiated up to 2 weeks in advance, such as up to 1 week, 6 days, 5 days, 4 days, 3 days, 2 days, 24 h, 12 h, 6 h, 5 h, 4 h, 3 h, 2 h, 1 h, 30 minutes or up to 15 minutes in advance of an event which is anticipated to cause noise-induced acute hearing loss. The compound of formula (I) or a pharmaceutically acceptable salt and/or solvate thereof and/or derivative thereof of formula (Z) may be administered on multiple occasions before event which is anticipated to cause noise-induced acute hearing loss.

In one embodiment, a compound of formula (I) or a pharmaceutically acceptable salt and/or solvate thereof and/or derivative thereof of formula (Z) is administered in advance of potential exposure to a noise or blast which is anticipated to cause acute noise-induced hearing loss, for preventing or reducing the development of permanent tinnitus;

for preventing or reducing the development of a permanent shift in auditory thresholds; or for preventing or reducing the development of permanently degraded central auditory processing, including for example auditory temporal processing and/or speech understanding.

It will be appreciated that administration in advance may be in circumstances where the subject is considered to be at risk of exposure to a noise or blast which is anticipated to cause acute noise-induced hearing loss and is not limited to those circumstances where such exposure ultimately occurs.

In one embodiment, administration of the compound of formula (I) or a pharmaceutically acceptable salt and/or solvate thereof and/or derivative thereof of formula (Z) is initiated during an event which is anticipated to cause noise-induced acute hearing loss. The compound of formula (I) or a pharmaceutically acceptable salt and/or solvate thereof and/or derivative thereof of formula (Z) may be administered on multiple occasions during an event which is anticipated to cause noise-induced acute hearing loss.

In one embodiment, a compound of formula (I) or a pharmaceutically acceptable salt and/or solvate thereof and/or derivative thereof of formula (Z) is initially administered during a noise or blast which is anticipated to cause acute noise-induced hearing loss, for preventing or reducing the development of permanent tinnitus; for preventing or reducing the development of a permanent shift in the auditory threshold; or for preventing or reducing the development of permanently degraded central auditory processing, including for example auditory temporal processing and/or speech understanding.

In one embodiment, administration of the compound of formula (I) or a pharmaceutically acceptable salt and/or solvate thereof and/or derivative thereof of formula (Z) is initiated after an event which is anticipated to cause acute noise-induced hearing loss.

Thus, in one embodiment, a compound of formula (I) or a pharmaceutically acceptable salt and/or solvate thereof and/or derivative thereof of formula (Z) is initially administered after a noise or blast which is anticipated to cause acute noise-induced hearing loss, for preventing or reducing the development of permanent tinnitus; for preventing or reducing the development of a permanent shift in the auditory threshold; or for preventing or reducing the development of permanently degraded central auditory processing, including for example auditory temporal processing and/or speech understanding.

When the compound of formula (I) is administered after an event which is anticipated to cause acute noise-induced hearing loss, such administration is normally undertaken during the "acute phase" i.e. before the hearing loss has become established.

In one embodiment, administration of the compound of formula (I) or a pharmaceutically acceptable salt and/or solvate thereof and/or derivative thereof of formula (Z) may be initiated up to 2 months after an event which is anticipated to cause noise-induced acute hearing loss, such as up to 1 month, 2 weeks, 1 week, 6 days, 5 days, 4 days, 3 days, 2 days, 24 h, 12 h, 6 h, 5 h, 4 h, 3 h, 2 h, 1 h, 30 minutes or up to 15 minutes after an event which is anticipated to cause acute noise-induced hearing loss. The compound of formula (I) or a pharmaceutically acceptable salt and/or solvate thereof and/or derivative thereof of formula (Z) may be administered on multiple occasions after an event which is anticipated to cause noise-induced acute hearing loss.

The compound of formula (I) or a pharmaceutically acceptable salt and/or solvate thereof and/or derivative thereof of formula (Z) may be administered over a period of up to 7 days (for example, up to 1 day, up to 2 days, up to 3 days, up to 4 days, up to 5 days, up to 6 days or up to 7 days), for 1-2 weeks (for example, 7-8 days, 7-9 days, 7-10 days, 7-11 days, 7-12 days, 7-13 days or 7-14 days), for 2-4 weeks (for example, 2-3 weeks or 2-4 weeks) or for 1-2 months (for example, 4-6 weeks or 4-8 weeks).

The compound of formula (I) or a pharmaceutically acceptable salt and/or solvate thereof and/or derivative thereof of formula (Z) may initially be administered up to 1 day in advance, such as up to 2 days in advance, up to 3 days in advance, up to 5 days in advance, up to 1 week in advance, up to 2 weeks in advance or up to 1 month in advance of a noise or blast which is anticipated to cause acute noise-induced hearing loss, administration which is initiated at any point in advance exposure to a noise or blast which is anticipated to cause acute noise-induced hearing loss will typically continue for up to 2 months after exposure to the noise or blast which is anticipated to cause acute noise-induced hearing loss, such as for up to 1 month after, up to 3 weeks after, up to two weeks after, up to 1 week after, up to 5 days after, up to 3 days after, up to 2 days after, or up to 1 day after.

In one embodiment is provided a compound of formula (I) or a pharmaceutically acceptable salt and/or solvate thereof and/or derivative thereof of formula (Z) for use in preventing or reducing the development of a permanent shift in the auditory threshold, wherein the permanent shift in auditory threshold is reduced by at least 10dB, such as at least 15dB, at least 20dB, at least 30dB, at least 40dB, or completely.

Pharmaceutical compositions

For use in therapy the compounds of the invention are usually administered as a pharmaceutical composition. The invention also provides a pharmaceutical composition comprising a compound of formula (I), or a pharmaceutically

acceptable salt and/or solvate (e.g. salt) thereof and/or derivative thereof of formula (Z), and a pharmaceutically acceptable carrier or excipient.

In one embodiment, there is provided a pharmaceutical composition comprising a compound of formula (I), or a pharmaceutically acceptable salt and/or solvate (e.g. salt) thereof and/or derivative thereof of formula (Z), for use in the treatment or prevention of a disease or disorder selected from the group consisting of hearing disorders, schizophrenia, depression and mood disorders, bipolar disorder, substance abuse disorders, anxiety disorders, sleep disorders, hyperacusis and disturbances of loudness perception, Ménière's disease, disorders of balance, and disorders of the inner ear, impulse control disorder, personality disorders, attention-deficit/hyperactivity disorder, autism spectrum disorders, eating disorders, cognition impairment, ataxia, pain such as neuropathic pain, inflammatory pain and miscellaneous pain, Lewy body dementia and Parkinson's disease.

The compounds of formula (I) or their pharmaceutically acceptable salts and/or solvates thereof and/or derivatives thereof of formula (Z) may be administered by any convenient method, e.g. by oral, parenteral, buccal, sublingual, nasal, rectal or transdermal administration, and the pharmaceutical compositions adapted accordingly. Other possible routes of administration include intratympanic and intracochlear.

The compounds of formula (I) or their pharmaceutically acceptable salts and/or solvates thereof and/or derivatives thereof of formula (Z) which are active when given orally can be formulated as liquids or solids, e.g. as syrups, suspensions, emulsions, tablets, capsules or lozenges.

A liquid formulation will generally consist of a suspension or solution of the active ingredient (such as a compound of formula (I) or a pharmaceutically acceptable salt and/or solvate (e.g. salt) thereof and/or derivative thereof of formula (Z)) in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient (such as a compound of formula (I) or a pharmaceutically acceptable salt and/or solvate (e.g. salt) thereof and/or derivative thereof of formula (Z)) can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient (such as a compound of formula (I) or a pharmaceutically acceptable salt and/or solvate (e.g. salt) thereof and/or derivative thereof of formula (Z)) in a sterile aqueous carrier or parenterally acceptable oil, e.g. polyethylene glycol, polyvinyl pyrrolidone, lecithin, arachis oil or sesame oil.

Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively, the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluorochlorohydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches. In one embodiment the composition is in unit dose form such as a tablet, capsule or ampoule.

The composition may contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05 mg to 1000 mg, for example from 1.0 mg to 500 mg, of the active material, depending on the method of administration. The composition may contain from 50 mg to 1000 mg, for example from 100 mg to 400 mg of the carrier, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 mg to 1000 mg, more suitably 1.0 mg to 500 mg,

and such unit doses may be administered more than once a day, for example two or three a day. Such therapy may extend for a number of weeks or months.

The dose provided to a subject will typically be a safe and effective dose, i.e. an acceptable balance of desired benefits and undesired side effects.

The invention provides, in a further aspect, a combination comprising a compound of formula (I) or a pharmaceutically acceptable, salt, solvate and/or derivative thereof of formula (Z) (e.g. a combination comprising a compound of formula (I) or a pharmaceutically acceptable derivative thereof of formula (Z)) together with a further pharmaceutically acceptable active ingredient or ingredients.

The invention provides a compound of formula (I), for use in combination with a further pharmaceutically acceptable active ingredient or ingredients.

When the compounds are used in combination with other therapeutic agents, the compounds may be administered either sequentially or simultaneously by any convenient route. Alternatively, the compounds may be administered separately.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) or a pharmaceutically acceptable derivative thereof of formula (Z) is used in combination with a second therapeutic agent active against the same disease state the dose of each compound may differ from that when the compound is used alone. Appropriate doses will be readily appreciated by those skilled in the art.

## Experimental

The invention is illustrated by the compounds described below. The following examples describe the laboratory synthesis of specific compounds of the invention and are not meant to limit the scope of the invention in any way with respect to compounds or processes. It is understood that, although specific reagents, solvents, temperatures and time periods are used, there are many possible equivalent alternatives that can be used to produce similar results. This invention is meant to include such equivalents.

## Analytical Equipment

Starting materials, reagents and solvents were obtained from commercial suppliers and used without further purification unless otherwise stated. Unless otherwise stated, all compounds with chiral centres are racemic. Where reactions are described as having been carried out in a similar manner to earlier, more completely described reactions, the general reaction conditions used were essentially the same. Work up conditions used were of the types standard in the art, but may have been adapted from one reaction to another. The starting material may not necessarily have been prepared from the batch referred to. Compounds synthesised may have various purities, ranging from for example 85% to 99%. Calculations of number of moles and yield are in some cases adjusted for this.

HPLC-Mass spectra (HPLC-MS) were taken on an Agilent 1100 Series LC/MSD Mass Spectrometer coupled with HPLC instrument Agilent 1100 Series, operating in positive electrospray ionization mode and in acidic gradient conditions.

Quality Control (3 minutes method): LC/MS-ES+ under acidic conditions was performed on a Zorbax SB C18 column (1.8 μm 3 x 50 mm). Mobile phase: A: (H2O + 0.05% TFA by vol.) / B: (CH3CN + 0.05% TFA by vol). Gradient: t = 0 min 0% (B), from 0 to 95% (B) in 2.5 min, 95% (B)for 0.2 min, from 95 to 100% (B) in 0.2 min, 100% (B) for 0.4 min, from 100% to 0% (B) in 0.1 min. Stop time 4 min. Column T = 60°C. Flow rate: 1.5 ml/min. Mass range ES+: (100-1000 amu, F=60). UV detection wavelengths: DAD 1A = 220.8, DAD 1B = 254.8. The use of this methodology is indicated by "QC_3_MIN" in the analytic characterization of the described compounds.

Chiral control: LC/MS-ES+ under acidic conditions was performed on a CHIRALCEL® OD-H (250 x 4,6 mm - 5 um). Mobile phase: A: (H2O + 0.05% TFA by vol.) / B: (CH3CN + 0.05% TFA by vol). Gradient: t = 0 - 6 min 35% (B), t = 6 - 40 min from 35% to 50% (B), t = 40 - 45 min from 50% to 70% (B), t = 45 - 50 min from 70% to 35% (B), t = 50 - 55 min 35% (B). Stop time 60 min. Column T = 40°C. Flow rate: 1.0 ml/min. UV detection wavelengths: DAD 1A = 220.8, DAD 1B = 254.8.

Proton Magnetic Resonance (NMR) spectra were recorded either on Varian instruments at 300, 400, 500 or 600 MHz, or on Bruker instruments at 400 MHz. Chemical shifts are reported in ppm (δ) using the residual solvent line as internal standard. Splitting patterns are designed as s (singlet), br.s (broad singlet), d (doublet), t (triplet), q (quartet), dd (doublet of doublets), dt (doublet of triplets) and m (multiplet). The NMR spectra were recorded at temperatures

ranging from 25 to 60°C.

2D NMR NOESY experiments were acquired with a mixing time of 500 ms using a spectral width of 3355 Hz in both f1 and f2. A total of 256 increments were collected, processed to 1 K with linear prediction, 8 scans each. Data were processed with sine bell shift in both dimensions and with lb=0.3 Hz in f1. In a number of preparations, purification was performed using Biotage automatic flash chromatography (SP1 and SP4) or Flash Master Personal systems.

Flash chromatographies were carried out on silica gel 230-400 mesh (supplied by Merck AG Darmstadt, Germany) or on silica gel 300-400 mesh (supplied by Sinopharm Chemical Reagent Co., Ltd.), Varian Mega Be-Si pre-packed cartridges, pre-packed Biotage silica cartridges (e.g. Biotage SNAP cartridge).

Abbreviations

| | |
|---|---|
| AIBN | azobisisobutyronitrile |
| BuLi | butyllithium |
| $CDCl_3$ | deuterated chloroform |
| $CCl_4$ | carbon tetrachloride |
| $D_2O$ | deuterated water |
| DCM | dichloromethane |
| DIPEA | N,N-diisopropylethylamine |
| DMAP | 4-dimethylaminopyridine |
| DMF | N,N-dimethylformamide |
| DMSO | dimethylsulfoxide |
| DMSO-$d_6$ | deutrated dimethylsulfoxide |
| $Et_2O$ | diethyl ether |
| EtOAc | ethyl acetate |
| h | hours |
| HATU | (O-7-azabenzotriazol-1-yl)-N,N,N',N'-tetramethyluroniumhexafluoro phosphate) |
| HCl | hydrogen chloride |
| $K_2CO_3$ | potassium carbonate |
| MeCN /$CH_3CN$ | acetonitrile |
| MeOH | methanol |
| MOM | methyloxymethyl |
| NaH | sodium hydride |
| $Na_2SO_4$ | sodium sulphate |
| $Na_2CO_3$ | sodium carbonate |
| NaOH | sodium hydroxide |
| NaOMe | sodium methoxide |
| NMR | Nuclear Magnetic Resonance |
| r.t. | room temperature |
| T3P | propylphosphonic anhydride |
| MTBE | Methyl *tert*-butyl ether |
| TBTU | Benzotriazol-1-yl-N,N,N',N'-tetramethyluronium tetrafluoroborate |
| TEA | triethylamine |
| TFA | trifluoroacetic acid |
| THF | tetrahydrofuran |
| THP | tetrahydropyran |
| wt. | weight |

Compound Examples

Intermediate 1

**2-bromo-5-(7-methylspiro[2H-benzofuran-3,1'-cyclopropane]-4-yl)oxy-pyrazine**

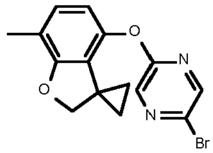

A mixture of 7-methylspiro[2H-benzofuran-3,1'-cyclopropane]-4-ol (Intermediate 156 WO2012076877, 1.11g, 6,30mmol), 2,5-dibromopyrazine (1.5g, 6,30mmol) and dipotassium carbonate (1.31g, 9.46mmol) in N,N-dimethylformamide (14mL) was stirred at 120°C for 3 hours. After cooling, the reaction mixture was diluted with MTBE (100 ml) and washed with brine (50 ml). Phases were separated and the aqueous layer was washed with MTBE (100ml) and EtOAc (100ml). All organic phases are collected, dried over $Na_2SO_4$, filtered and evaporated. The residue was purified by flash chromatography (Biotage System) on silica gel using a SNAP 100g as column and Cyclohexane: Ethyl acetate from 100:0 to 90:10 as eluent affording 2-bromo-5-(7-methylspiro[2H-benzofuran-3,1'-cyclopropane]-4-yl)oxy-pyrazine (1.8g) as white solid.

LC/MS: QC_3_MIN: Rt = 2.705 min; m/z 333 & 335 [M+H]+.

The following compounds were prepared using the foregoing methodology, replacing 7-methylspiro[2H-benzofuran-3,1'-cyclopropane]-4-ol with the appropriate phenol. Final products were purified by flash-chromatography (Silica cartridge; Cyclohexane/EtOAc or other appropriate solvent system).

| Int. | Structure | Name | Phenol | LCMS |
|---|---|---|---|---|
| 2 | 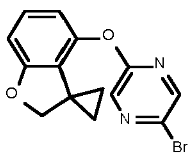 | 2-bromo-5-spiro[2H-benzofuran-3,1'-cyclopropane]-4-yloxy-pyrazine | spiro[2H-benzofuran-3,1'-cyclopropane]-4-ol (Intermediate 85 WO2012076877) | LC/MS: QC_3_MIN: Rt = 2.575 min; m/z 319 & 321 [M+H]+. |
| 3 | 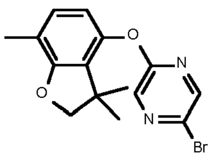 | 2-bromo-5-[(3,3,7-trimethyl-2H-benzofuran-4-yl)oxy]pyrazine | 3,3,7-trimethyl-2H-benzofuran-4-ol (Intermediate 184 WO2012076877) | LC/MS: QC_3_MIN: Rt = 2.365 min; m/z 335 & 337 [M+H]+. |
| 4 | 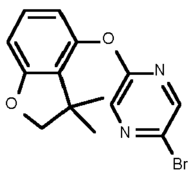 | 2-bromo-5-[(3,3-dimethyl-2H-benzofuran-4-yl)oxy] pyrazine | 3,3-dimethyl-2H-benzofuran-4-ol (Intermediate 50 WO2012076877) | LC/MS: QC_3_MIN: Rt = 2.632 min; m/z 321 & 323 [M+H]+. |

Intermediate 5 route 1

**3-(5-chloropyrazin-2-yl)-5,5-dimethyl-imidazolidine-2,4-dione**

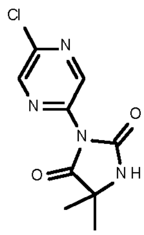

To a solution of bis(trichloromethyl) carbonate (950mg, 3.20mmol) in ethyl acetate (30mL) at 0°C a solution of 5-chloropyrazin-2-amine (0.75g, 5.79mmol)/N,N-diisopropylethylamine (6.05ml, 34.74mmol) in ethyl acetate (12mL) was added dropwise and the reaction mixture was stirred for 15 minutes at the same temperature. Maintaining the

reaction mixture at 0°C, vacuum was applied (5 minutes) in order to remove the excess of phosgene. A solution of 4-(dimethylamino)pyridine (710mg, 5.81mmol) in ethyl acetate (8mL)/ dichloromethane (2mL) was added and the reaction mixture was stirred for 5 minutes at the same temperature. Then, methyl 2-amino-2-methyl-propanoate hydrochloride (1.4g, 9.1mmol) was added at 0°C and the reaction mixture was stirred for 30 minutes at the same temperature. The reaction was quenched with a solution 0.2 N of HCl (100 ml) and the two phases were separated. The organic layer was washed with brine (100 ml), dried over $Na_2SO_4$, filtered and evaporated affording the urea intermediate.

The urea was dissolved in dichloromethane (20mL) and at 0°C sodium methoxide (315mg, 5.83mmol) was added. The reaction mixture was stirred 15 minutes at the same temperature; the reaction was quenched with a saturated solution of $NH_4Cl$ to allow the pH to reach 3-4. The mixture was extracted with ethyl acetate (50 ml); phases were separated, and the organic layer was washed with brine (50 ml), dried over $Na_2SO_4$, filtered and evaporated. The residue were purified by reverse phase flash chromatography (Biotage System) on C-18 phase using a SNAP 30g as column and Water:Acetonitrile from 95:5 to 40:60 as eluent. The appropriate fractions were combined and evaporated to dryness affording 3-(5-chloropyrazin-2-yl)-5,5-dimethyl-imidazolidine-2,4-dione (220mg) as a pale brown solid.

LC/MS: QC_3_MIN: Rt = 1.649 min; m/z 241 & 243 [M+H]+.

The following compounds were prepared using the foregoing methodology, replacing 2,2-dimethylglycine methyl ester hydrochloride with the appropriate amino ester hydrochloride. Final products were purified by flash-chromatography (Silica cartridge; Cyclohexane/EtOAc or other appropriate solvent system) or triturated in an appropriate solvent or crystallised from an appropriate solvent.

| Int. | Structure | Name | Amino ester hydrochloride | LCMS |
|---|---|---|---|---|
| 6 | 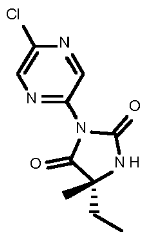 | 5R)-3-(5-chloropyrazin-2-yl)-5-ethyl-5-methylimidazolidine-2,4-dione | methyl (2R)-2-amino-2-methylbutanoate hydrochloride | LC/MS: QC_3_MIN: Rt = 1.546 min; m/z 255 & 257 [M+H]+. |

<u>Intermediate 5 route 2</u>

<u>**3-(5-chloropyrazin-2-yl)-5,5-dimethyl-imidazolidine-2,4-dione**</u>

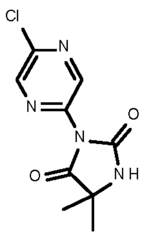

To a solution of 5-chloropyrazin-2-amine (500mg, 3.86mmol) and 2-amino-2-methyl-propanoic acid hydrochloride (646mg, 4.63mmol) in acetonitrile (10mL), Propylphosphonic anhydride solution ≥50 wt. % in ethyl acetate (3.68g, 5.78mmol) was slowly added at RT. The reaction mixture was stirred at 80°C for 6h. The reaction mixture was diluted with Ethyl Acetate (10ml) and an aqueous solution of NaOH 1 N was added, while the ph was allowed to reach ~8. The two phases were separated and the organic one was washed with brine (10ml), dried with $Na_2SO_4$, concentrated under vacuum and the crude was purified by Flash Chromatography on silica gel (BIOTAGE SYSTEM), using a SNAP 25g as column and DCM:MEOH from 99/1 to 90/10 as eluent, affording 2-amino-N-(5-chloropyrazin-2-yl)-2-methyl-propanamide (190mg) as yellow solid.

LC/MS: QC_3_MIN: Rt = 1.181 min; m/z 215 & 217 [M+H]+.

To a solution of 2-amino-N-(5-chloropyrazin-2-yl)-2-methyl-propanamide (190mg, 0.88mmol) and triethylamine (268mg, 2,6555mmol) in dichloromethane (5mL), at 0°C a solution of bis(trichloromethyl) carbonate (105,07mg,0,3541mmol) in dichloromethane (4mL) was slowly added. and the reaction mixture was stirred for 30 minutes at the same temperature. The reaction mixture was diluted in DCM (10mL), washed with an aqueous

solution 0.2N of HCl (10mL) and Brine (10mL). The organic phases were concentrated under *vacuum* and the crude was purified by flash chromatography on silica gel (Biotage system) using a SNAP 25g as column and Chexane/EtOAc from 80/20 to 0/100 as eluent affording 3-(5-chloropyrazin-2-yl)-5,5-dimethyl-imidazolidine-2,4-dione (130mg) as white solid.

LC/MS: QC_3_MIN: Rt = 1.598 min; m/z 241 & 243 [M+H]+.

Intermediate 7

**tert-butyl N-[(1R)-1-carbamoylpropyl]carbamate**

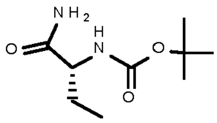

A mixture of [dimethylamino-(3-oxidotriazolo[4,5-b]pyridin-3-ium-1-yl)methylene]-dimethylammonium tetrafluoroborate (1,1084g,3,4415mmol), N,N-diisopropylethylamine (0,7939g,6,1431mmol) and (2R)-2-(tert-butoxycarbonylamino)butanoic acid (0,5000g,2,4601mmol) in dry N,N-dimethylformamide (8mL) was stirred at room temperature for 10 minutes. Hexamethyldisilazane (0,5960g,3,6928mmol) was added and the mixture stirred for 18h.

Reaction mixture was separated in MTBE (30 mL) and Brine (20 mL). The organic layer was dried with sodium sulphate, filtered and the solvent removed. The resulting oil triturated in MTBE (3 mL) and the resulting precipitate was washed with MTBE and dried via vacuum to give tert-butyl N-[(1R)-1-carbamoylpropyl]carbamate (0,3000g,1,4833mmol, 60,294 %) as a white solid.

LC/MS: QC_3_MIN: m/z 147 [M-tBu+H]+.

The following compounds were prepared using the foregoing methodology, replacing (2R)-2-(tert-butoxycarbonilamino) butanoic acid with the appropriate protected amino-acid.

| Int. | Structure | Name | Amino-acid | LCMS |
|---|---|---|---|---|
| 8 | 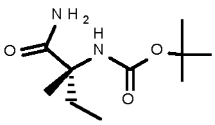 | tert-butyl N-[(1R)-1-carbamoyl-1-methylpropyl]carbamate | (2R)-2-(tertbutoxycarbonylamino)-2-methylbutanoic acid | LC/MS: QC_3_MIN: m/z 455 [2M+Na]+. |

Intermediate 9 (route 1)

**tert-butyl N-[(1R)-1-[[5-(7-methylspiro[2H-benzofuran-3,1'-cyclopropane]-4-yl)oxypyrazin-2-yl]carbamoyl]propyl]carbamate**

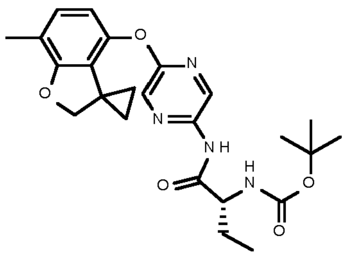

A mixture of 2-bromo-5-(7-methylspiro[2H-benzofuran-3,1'-cyclopropane]-4-yl)oxy-pyrazine (Intermediate 1, 50mg, 0.15mmol), tert-butyl N-[(1R)-1-carbamoylpropyl]carbamate (Intermediate 7 , 46mg, 0.23mmol), Tris(dibenzylideneacetone)dipalladium(0) (10.3mg, 0.011mmol), dicyclohexyl-[2-(2,4,6-triisopropylphenyl)phenyl]phosphane (XPhos) (5.4mg, 0.011mmol) and cesium carbonate (73mg, 0.22mmol) in 1,4-dioxane (2mL) was stirred under an

atmosphere of nitrogen at 80°C for 3h.

The reaction was partitioned between ethyl acetate and brine. The organic layer was separated, dried with sodium sulphate, filtered and evaporated to dryness. The residue was purified by flash chromatography on silica gel (Biotage system) using a SNAP 10g column and cyclohexane and EtOAc from 100/0 to 0/100 as eluent. The appropriate fractions were combined and evaporated to dryness, affording tert-butyl N-[(1R)-1-[[5-(7-methylspiro[2H-benzofuran-3,1'-cyclopropane]-4-yl)oxypyrazin-2-yl]carbamoyl]propyl]carbamate (10mg). LC/MS: QC_3_MIN: Rt = 2.696 min; m/z 455 [M+H]+.

Intermediate 9 (route 2)

**tert-butyl N-[(1R)-1-[[5-(7-methylspiro[2H-benzofuran-3,1'-cyclopropane]-4-yl)oxypyrazin-2-yl]carbamoyl]propyl]carbamate**

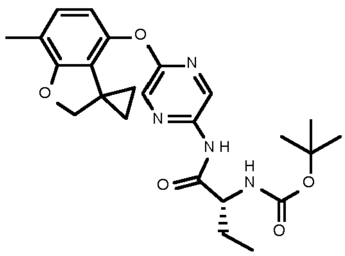

To a mixture of 2-bromo-5-(7-methylspiro[2H-benzofuran-3,1'-cyclopropane]-4-yl)oxy-pyrazine (Intermediate 1, 16g, 48.0mmol), tert-butyl N-[(1R)-1-carbamoylpropyl]carbamate (Intermediate 7, 10g, 49.4mmol), cesium carbonate (24.16g, 74.17mmol) in 1,4-dioxane (150mL), after flushing with argon, diacetoxypalladium (0.555g, 2.47mmol) and (5-diphenylphosphanyl-9,9-dimethyl-xanthen-4-yl)-diphenyl-phosphane (2.15g, 3.71mmol) were added. For three times cycle vacuum-argon was applied and the reaction mixture was stirred at 95°C for 1.5h. The reaction mixture was cooled using an external ice bath and then filtered under vacuum to remove cesium carbonate. The filtrate was collected, diluted with EtOAc (150ml) and washed with an aqueous saturated solution of $NH_4Cl$ (100ml) and then with a n aqueous saturated solution of NaCl (100ml), dried with sodium sulphate, filtered and evaporated to dryness.

The residue was purified by flash chromatography on silica gel (Biotage system) using 2x SNAP 100g column (200g silica) and cyclohexane/EtOAc from 0 to 40% as eluent affording tert-butyl N-[(1R)-1-[[5-(7-methylspiro[2H-benzofuran-3,1'-cyclopropane]-4-yl)oxypyrazin-2-yl]carbamoyl]propyl]carbamate (16.8g) as yellow solid.

The following compounds were prepared using the foregoing methodology (either route 1 or route 2), replacing 2-bromo-5-(7-methylspiro[2H-benzofuran-3,1'-cyclopropane]-4-yl)oxy-pyrazine (Intermediate 1) with the appropriate bromopyrazine. Final products were purified by flash-chromatography (Silica cartridge; Cyclohexane/EtOAc or other appropriate solvent system).

| Int. | Structure | Name | bromopyrazine | LCMS |
|---|---|---|---|---|
| 10 | 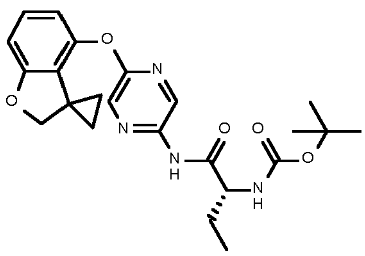 | tert-butyl N-[(1R)-1-[(5-spiro[2H-benzofuran-3,1'-cyclopropane]-4-yloxypyrazin-2-yl) carbamoyl]propyl]carbamate | 2-bromo-5-spiro[2H-benzofuran-3,1'-cyclopropane]-4-yloxy-pyrazine (Intermediate 2) | LC/MS: QC_3_MIN: Rt = 2.246 min; m/z 441 [M+H]+. |

(continued)

| Int. | Structure | Name | bromopyrazine | LCMS |
|---|---|---|---|---|
| 11 | 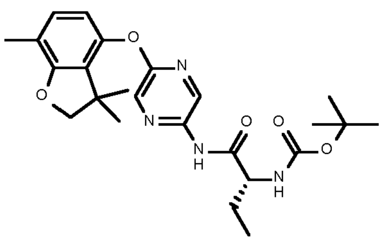 | tert-butyl N-[(1R)-1-[[5-[(3,3,7-trimethyl-2H-benzofuran-4-yl)oxy] pyrazin-2-yl]carbamoyl] propyl]c arbamate | 2-bromo-5-[(3,3,7-trimethyl-2H-benzofuran-4-yl)oxy] pyrazine (Intermediate 3) | LC/MS: QC_ 3_MIN: Rt = 2.309 min; m/z 457 [M+H] +. |
| 12 | 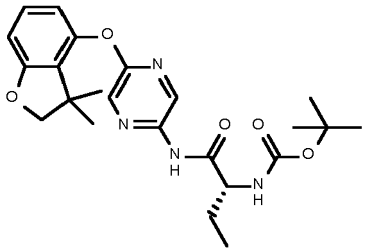 | tert-butyl N-[(1R)-1-[[5-[(3,3-dimethyl-2H-benzofuran-4-yl)oxy] pyrazin-2-yl]carbamoyl] propyl]c arbamate | 2-bromo-5-[(3,3-dimethyl-2H-benzofuran-4-yl)oxy] pyrazine (Intermediate 4) | LC/MS: QC_ 3_MIN: Rt = 2.366 min; m/z 443 [M+H] +. |

Intermediate 13

**(2R)-2-amino-N-[5-(7-methylspiro[2H-benzofuran-3,1'-cyclopropane]-4-yl)oxypyrazin-2-yl]butanamide**

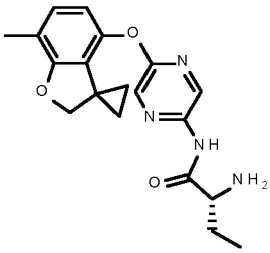

A mixture of tert-butyl N-[(1R)-1-[[5-(7-methylspiro[2H-benzofuran-3,1'-cyclopropane]-4-yl)oxypyrazin-2-yl]carbamoyl]propyl]carbamate (Intermediate 9, 16mg, 0.035mmol) and 2,2,2-trifluoroacetic acid (0.50mL, 6.53mmol) in dichloromethane (2mL) was stirred at room temperature for 2h.

The reaction mixture was diluted with dichloromethane (20 ml) and a saturated solution of NaHCOs (aq) was added while the pH was allowed to reach 8. The phases were separated and the organic layer was washed with brine (20 ml), dried over $Na_2SO_4$, filtered and evaporated affording (2R)-2-amino-N-[5-(7-methylspiro[2H-benzofuran-3,1'-cyclopropane]-4-yl)oxypyrazin-2-yl]butanamide (13mg) that was used in the next step without further purification.

LC/MS: QC_3_MIN: Rt = 2.009 min; m/z 355 [M+H]+.

The following compounds were prepared using the foregoing methodology, replacing tert-butyl N-[(1R)-1-[[5-(7-methylspiro[2H-benzofuran-3,1'-cyclopropane]-4-yl)oxypyrazin-2-yl]carbamoyl]propyl]carbamate (Intermediate 9) with the appropriate Boc amine.

| Int. | Structure | Name | Boc amine | LCMS |
|---|---|---|---|---|
| 14 | 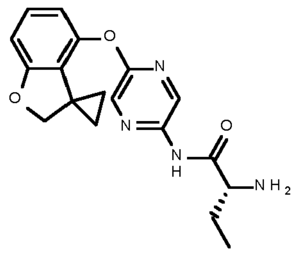 | (2R)-2-amino-N-(5-spiro [2H-benzofuran-3,1'-cyclopropane]-4-yloxypyrazin-2-yl) butanamide | tert-butyl N-[(1R)-1-[(5-spiro [2H-benzofuran-3,1'-cyclopropane]-4-yloxypyrazin-2-yl)carbamoyl]propyl]carba mate (Intermediate 10) | LC/MS: QC_ 3_MIN: Rt = 1.675 min; m/z 342 [M+H] +. |
| 15 | 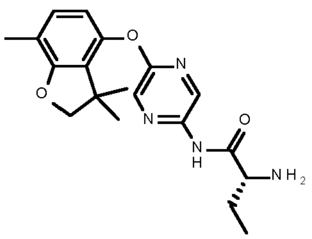 | (2R)-2-amino-N-[5-[(3,3,7-trimethyl-2H-benzofuran-4-yl)oxy] pyrazin-2-yl]butanamide | tert-butyl N-[(1R)-1-[[5-[(3,3,7-trimethyl-2H-benzofuran-4-yl) oxy]pyrazin-2-yl]carbamoyl] propyl]carba mate(Intermediate 11) | LC/MS: QC_ 3_MIN: Rt = 1.756 min; m/z 357 [M+H] +. |
| 16 | 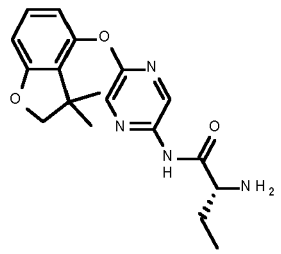 | (2R)-2-amino-N-[5-[(3,3-dimethyl-2H-benzofuran-4-yl)oxy] pyrazin-2-yl]butanamide | tert-butyl N-[(1R)-1-[[5-[(3,3-dimethyl-2H-benzofuran-4-yl) oxy]pyrazin-2-yl]carbamoyl] propyl]carba mate(Intermediate 12) | LC/MS: QC_ 3_MIN: Rt = 1.673 min; m/z 343 [M+H] +. |

Intermediate 17

**(5R)-5-ethyl-5-methyl-imidazolidine-2,4-dione**

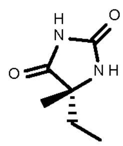

A mixture of tert-butyl N-[(1R)-1-carbamoyl-1-methyl-propyl]carbamate (Intermediate 8, 100mg,0,4624mmol) and potassium carbonate (191,71mg,1,3871mmol) in 1-butanol (5mL) was stirred under an atmosphere of nitrogen at 95°C overnight. After cooling, potassium carbonate was filtered off and the reaction mixture was diluted with ethyl acetate (30 ml) and washed with an aqueous 0.1N HCl solution (30 ml) and then with brine (30 ml). Phases were separated and the organic layer was collected, dried over $Na_2SO_4$, filtered and evaporated affording (5R)-5-ethyl-5-methyl-imidazolidine-2,4-dione (60mg,0,4221mmol, 91,283 %).

LC/MS: QC_3_MIN: m/z 285 [2M+H]+.

Intermediate 18

**tert-butyl N-(1-carbamoylcyclobutyl)carbamate**

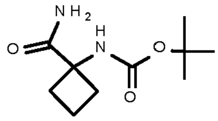

Intermediate 18 was prepared using the methodology described for Intermediate 7, replacing (2R)-2-(tert-butoxycarbonylamino)butanoic acid with 1-(tert-butoxycarbonylamino)cyclobutanecarboxylic acid.

LC/MS: QC_3_MIN: m/z 159 [M-tBu+H]+.

Intermediate 19

**tert-butyl N-[1-[[5-(7-methylspiro[2H-benzofuran-3,1'-cyclopropane]-4-yl)oxypyrazin-2-yl]carbamoyl]cyclobutyl]carbamate**

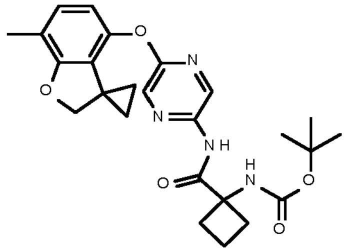

A mixture of 2-bromo-5-(7-methylspiro[2H-benzofuran-3,1'-cyclopropane]-4-yl)oxy-pyrazine (Intermediate 1, 50mg, 0.1501mmol), tert-butyl N-(1-carbamoylcyclobutyl)carbamate (Intermediate 18, 64mg, 0.2987mmol), dipotassium carbonate (62mg,0,4486mmol), copper(I) iodide (2.9mg,0.0152mmol) and N,N'-dimethylethane-1,2-diamine (0.0065mL,0.0601mmol) in 1-butanol (1mL) was stirred under an atmosphere of nitrogen at 95°C for 4h. After cooling, the reaction mixture was diluted with ethyl acetate (30 ml) and washed with an aqueous 0.1M HCl solution (30 ml) and then with brine (30 ml). Phases were separated, and the organic layer was collected, dried over $Na_2SO_4$, filtered and evaporated. The residue was purified by flash chromatography (Biotage System) on silica gel using a SNAP 10g as column and Cyclohexane: Ethyl acetate from 100:0 to 30:70 as eluent affording tert-butyl N-[1-[[5-(7-methylspiro[2H-benzofuran-3,1'-cyclopropane]-4-yl)oxypyrazin-2-yl]carbamoyl]cyclobutyl]carbamate (18mg). LC/MS: QC_3_MIN: Rt = 2.675 min; m/z 467 [M+H]+.

Intermediate 20

**1-amino-N-[5-(7-methylspiro[2H-benzofuran-3,1'-cyclopropane]-4-yl)oxypyrazin-2-yl]cyclobutanecarboxamide**

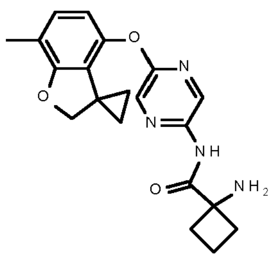

Intermediate 20 was prepared using the methodology described for Intermediate 13, replacing tert-butyl N-[(1R)-1-[[5-(7-methylspiro[2H-benzofuran-3,1'-cyclopropane]-4-yl)oxypyrazin-2-yl]carbamoyl]propyl]carbamate (Intermediate 9) with tert-butyl N-[1-[[5-(7-methylspiro[2H-benzofuran-3,1'-cyclopropane]-4-yl)oxypyrazin-2-yl]carbamoyl]cyclobutyl]carbamate (Intermediate 19).

LC/MS: QC_3_MIN: Rt = 1.979 min; m/z 367 [M+H]+.

Intermediate 21

**tert-butyl N-(1-carbamoylcyclopropyl)carbamate**

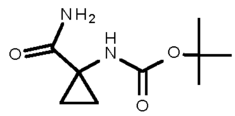

Intermediate 21 was prepared using the methodology described for Intermediate 7, replacing (2R)-2-(tert-butoxycarbonylamino)butanoic acid with 1-(tert-butoxycarbonylamino)cyclopropanecarboxylic acid.

Intermediate 22

**tert-butyl N-[1-[[5-(7-methylspiro[2H-benzofuran-3,1'-cyclopropane]-4-yl)oxypyrazin-2-yl]carbamoyl]cyclopropyl]carbamate**

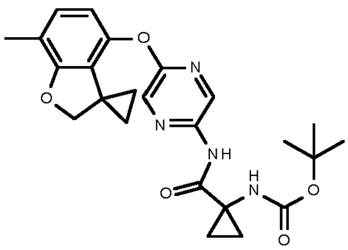

A mixture of dicyclohexyl-[2-(2,4,6-triisopropylphenyl)phenyl]phosphane (12mg, 0.0252mmol), tert-butyl N-(1-carbamoylcyclopropyl)carbamate (Intermediate 21, 67mg, 0.3346mmol), Tris(dibenzylideneacetone)dipalladium(0) (22mg, 0.0240mmol), 2-bromo-5-(7-methylspiro[2H-benzofuran-3,1'-cyclopropane]-4-yl)oxy-pyrazine (Intermediate 1, 79.518mg, 0.2387mmol) and caesium carbonate (116mg, 0.3560mmol) in 1,4-dioxane (1mL) were stirred under an atmosphere of nitrogen at 95°C for 2h. Additional tert-butyl N-(1-carbamoylcyclopropyl)carbamate (Intermediate 21, 67mg, 0.3346mmol) and Tris(dibenzylideneacetone)dipalladium(0) (22mg, 0.0240mmol) was added and the reaction mixture was stirred at 95°C under nitrogen for a further 2h, followed by the addition of a further dicyclohexyl-[2-(2,4,6-triisopropylphenyl)phenyl]phosphane (12mg, 0.0252mmol), Tris(dibenzylideneacetone)dipalladium(0) (22mg, 0.0240mmol) and caesium carbonate (58mg) and the mixture was stirred under nitrogen for a further 2h. The reaction mixture was then quenched with water (10mL), $NH_4Cl$ (10mL) and extracted with ethyl acetate (20mL). The organic layer was then washed with brine (15mL), dried over $Na_2SO_4$, filtered, then concentrated in vacuo. The crude was purified by flash chromatography (Biotage System) on silica gel using a SNAP 10g as column and Cyclohexane:Ethyl acetate 90:10 to 70:30 as eluent to afford tert-butyl N-[1-[[5-(7-methylspiro[2H-benzofuran-3,1'-cyclopropane]-4-yl)oxypyrazin-2-yl]carbamoyl]cyclopropyl]carbamate (55mg) as a yellow solid.

LC/MS: QC_3_MIN: Rt = 2.634 min; m/z 453 [M+H]+.

Intermediate 23

**1-amino-N-[5-(7-methylspiro[2H-benzofuran-3,1'-cyclopropane]-4-yl)oxypyrazin-2-yl]cyclopropanecarboxamide**

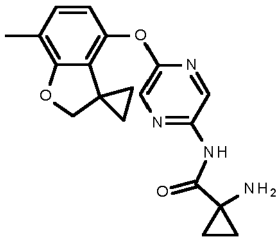

tert-butyl N-[1-[[5-(7-methylspiro[2H-benzofuran-3,1'-cyclopropane]-4-yl)oxypyrazin-2-yl]carbamoyl]cyclopropyl]carbamate (Intermediate 22, 55mg, 0.1215mmol) was dissolved in dichloromethane (4mL) and cooled to 0 °C. 2,2,2-trifluoroacetic acid (1154.7mg, 10.026mmol) (0.8mL) was added dropwise and the reaction was stirred at room temperature for 1 hour. The reaction mixture was then cooled to 0 °C and $NaHCO_3$ was added until the pH reached 8. The mixture was then allowed to warm to room temperature and extracted with DCM (10mL). The organic layer was dried over $Na_2SO_4$, filtered and concentrated in vacuum to afford1-amino-N-[5-(7-methylspiro[2H-benzofuran-3,1'-cyclopropane]-4-yl)oxypyrazin-2-yl]cyclopropanecarboxamide (40mg) as a yellow oil.

LC/MS: QC_3_MIN: Rt = 1.935 min; m/z 353 [M+H]+.

Intermediate 24

**1,3-dibenzyloxy-2-bromo-benzene**

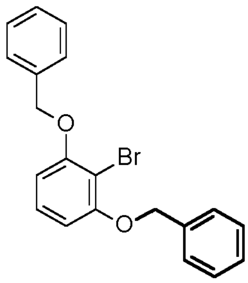

To a solution of 2-bromobenzene-1,3-diol (20g,105.8mmol) in acetone (200mL), potassium carbonate (43.87g, 317.4mmol) was added followed by the addition of benzyl bromide (40.72g, 238.1mmol) (28ml) and the reaction mixture was refluxed for 1.5 hours. After cooling, the reaction mixture was filtered under vacuum and the filtrate was concentrated to dryness. The residue was diluted with ethyl acetate (100 ml) and washed with water (100 ml) and then with brine (100 ml). Phases were separated and the organic layer was dried over $Na_2SO_4$, filtered and concentrated. The residue was suspended in isopropanol (8 volumes) and the mixture heated at 80°C and stirred for 1 hour at this temperature (to obtain a clear solution). Then, the mixture was allowed to reach room temperature (in 1h) and the obtained suspension was filtered. The solid was washed with ice cold isopropanol and then dried affording the title compound 1,3-dibenzyloxy-2-bromo-benzene (34g) as pale pink solid.

LC/MS: QC_3_MIN: Rt = 2.688 min.

Intermediate 25

**bromo-(1-methoxycarbonylcyclopropyl)zinc**

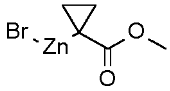

In a two-neck round-bottom flask activated zinc powder (6.84g,104.6mmol) was added and the powder was heated under vacuum. The system was put under argon and dry tetrahydrofuran (58mL) was added. Then, 1,2-dibromoethane (2.18g, 11.62mmol) was added and the mixture was heated to reflux. Chlorotrimethylsilane (505mg, 4.65mmol) was added in a single portion and the mixture kept stirring at reflux temperature. A solution of methyl 1-bromocyclopropylcarboxylate (10.4g, 58.1mmol) in dry tetrahydrofuran (12mL) was slowly added at the same temperature and the reaction mixture was refluxed for 1.5h. The reaction mixture was cooled down to room temperature and the zinc was allowed to settle affording 70ml of a 0.83M (theoretical) solution of bromo-(1-methoxycarbonylcyclopropyl)zinc in THF which was used in the next step without further work up.

Intermediate 26

**methyl 1-(2,6-dibenzyloxyphenyl)cyclopropanecarboxylate**

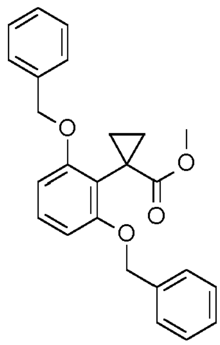

To a solution of 1,3-dibenzyloxy-2-bromo-benzene (Intermediate 24, 16g, 43.33mmol) and Bis(tri-tert-butylphosphine)palladium(0) (221mg, 0.43mmol) in N,N-dimethylformamide (150mL) pre-heated at 70 °C, a 0.83M (theoretical) solution of bromo-(1-methoxycarbonylcyclopropyl)zinc in THF (Intermediate 25, 60ml) was added (via cannulation) and the reaction mixture was stirred at the same temperature for 40 minutes. After cooling, the reaction mixture was concentrated under vacuum up to ~30 ml and the residue was diluted with ethyl acetate (450 ml) and washed twice with a 1N aqueous solution of HCl (2×100 ml) and then three times with ice cold brine (3x100ml). Phases were separated and the organic layer was filtered under vacuum on a Gooch filter assembled with filter paper and cellulose and washing with ethyl acetate. The filtrate was dried over $Na_2SO_4$, filtered and evaporated affording the title compound methyl 1-(2,6-dibenzyloxyphenyl)cyclopropanecarboxylate (15.5g) that was in the next step without further purification.

LC/MS: QC_3_MIN: Rt = 2.606 min; m/z 389 [M+H]+.

Intermediate 27

**4-hydroxyspiro[benzofuran-3,1'-cyclopropane]-2-one**

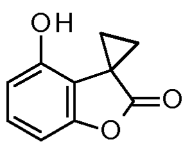

The reaction was performed in three different runs using about 20g of starting material each. General procedure: to a mixture of methyl 1-(2,6-dibenzyloxyohenyl)cyclopropanecarboxylate (Intermediate 26, 20.4g, 52.52mmol) and palladium 5% wt. on carbon (1.02g) in ethanol (200ml), ammonium formate (16.56g, 262.6mmol) was added and the reaction mixture was stirred at 80°C for 1 hour. After cooling, the catalyst was filtered off on a cellulose pad and the filtrate was concentrated under vacuum up to ~20ml.

The residues coming from the 3 runs were put together and diluted with ethyl acetate (400ml) and washed twice with water (2x300 ml). The two phases were separated and the organic one was washed with brine (300 ml), dried with $Na_2SO_4$ and concentrated under vacuum affording 4-hydroxyspiro[benzofuran-3,1'-cyclopropane]-2-one (27.55g) (containing -10-15% of the uncyclized methyl 1-(2,6-dihydroxyphenyl)cyclopropanecarboxylate intermediate) that was used in the next step without further purification.

LC/MS: QC_3_MIN: Rt = 1.707 min.

Intermediate 28

**4-benzyloxyspiro[benzofuran-3,1'-cyclopropane]-2-one**

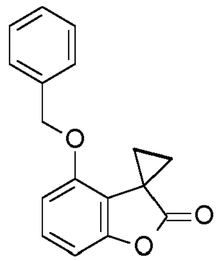

To a solution of 4-hydroxyspiro[benzofuran-3,1'-cyclopropane]-2-one (Intermediate 27, 28.5g,161.8mmol) (containing -10-15% of the uncyclized methyl 1-(2,6-dihydroxyphenyl)cyclopropanecarboxylate intermediate) in acetonitrile (200mL)/tetrahydrofuran (50mL), potassium carbonate (33.54g, 242,7mmol) was added and the reaction mixture was stirred at 70°C for 1.5 hours. The reaction mixture was then cooled to room temperature and benzyl bromide (27.67g, 161.8mmol) was slowly added. The reaction mixture was stirred at 60°C for 5 hours. After cooling, the reaction mixture was filtered under vacuum and the solid discarded, the filtrate was concentrated up to 50 ml, diluted with ethyl acetate (250 ml) and washed twice with brine (2x100 ml). Phases were separated and the organic layer was dried over $Na_2SO_4$, filtered and evaporated affording the title compound 4-benzyloxyspiro[benzofuran-3,1'-cyclopropane]-2-one (42,4g) that was used in the next step without further purification. LC/MS: QC_3_MIN: Rt = 2.389 min; m/z 267 [M+H]+.

Intermediate 29

**3-benzyloxy-2-[1-(hydroxymethyl)cyclopropyl]phenol**

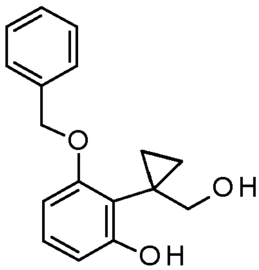

To a solution of 4-benzyloxyspiro[benzofuran-3,1'-cyclopropane]-2-one (Intermediate 28, 42.4g,159.2mmol) in dry tetrahydrofuran (300mL), a 1M solution of lithium aluminium hydride in THF (79.6ml, 79,6mmol) was slowly added at 0 °C and the reaction mixture was stirred at the same temperature for 30 minutes. The reaction was quenched with ice, water (400 ml) and an aqueous 1M solution of HCl (160 ml) and then diluted with ethyl acetate (700 ml). Phases were separated and the aqueous layer was back extracted with ethyl acetate (500 ml). The combined organic phases were washed with brine (600 ml), dried over $Na_2SO_4$, filtered and evaporated affording the title compound 3-benzyloxy-2-[1-(hydroxymethyl)cyclopropyl]phenol (43g) which was used in the next step without further purification.

LC/MS: QC_3_MIN: Rt = 2.148 min; m/z 271 [M+H]+, m/z 293 [M+Na]+, m/z 253 [M-OH]+.

Intermediate 30

**4-benzyloxyspiro[2H-benzofuran-3,1'-cyclopropane]**

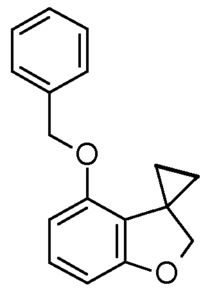

To a solution of 3-benzyloxy-2-[1-(hydroxymethyl)cyclopropyl]phenol (Intermediate 29, 43g, 159.1mmol) in dimethyl carbonate (430 mL), potassium tert-butoxide (35.7g, 318.1mmol) was slowly added and the reaction mixture was stirred at 85°C for 3.5 hours. The reaction mixture was cooled to room temperature, concentrated under vacuum up to 150mL, diluted with MTBE (400 ml) and washed with water (400 ml). Phases were separated and the aqueous layer was back extracted with MTBE (250 ml).The combined organic layers were washed with brine (350 ml), dried over $Na_2SO_4$, filtered and concentrated affording the title compound 4-benzyloxyspiro[2H-benzofuran-3,1'-cyclopropane] (40g) that was used in the next step without further purification.
LC/MS: QC_3_MIN: Rt = 2.457 min; m/z 253 [M+H]+.

Intermediate 31 (Intermediate 85 WO2012/076877)

**1 spiro[2H-benzofuran-3,1'-cyclopropane]-4-ol**

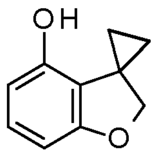

The reaction was done in two runs using 20g of starting material each.
To a mixture of 4-benzyloxyspiro[2H-benzofuran-3,1'-cyclopropane] (Intermediate 30, 20g, 79.27mmol) and ammonium formate (24.99g, 396.34mmol) in ethanol (160ml), palladium 5% wt. on carbon (2.0g) was added and the reaction mixture was stirred at 80°C for 10 minutes. After cooling, the catalyst was filtered off through a cellulose pad and the filtrate was concentrated under vacuum up to ~20 ml. The residues coming from the two reactions were combined and the mixture was diluted with ethyl acetate (300 ml) and washed three times with water (3x200 ml) and then with brine (200 ml). The two phases were separated and the organic one was dried over $Na_2SO_4$ and concentrated under vacuum. The residue was purified by flash chromatography (Biotage System) on silica gel using Cyclohexane: Ethyl acetate from 99:1 to 85:15 as eluent affording spiro[2H-benzofuran-3,1'-cyclopropane]-4-ol (17,75g) as white solid. LC/MS: QC_3_MIN: Rt = 1.723 min; m/z 163 [M+H]+.

Intermediate 32

**tert-butyl N-[(1S)-1-carbamoylpropyl]carbamate**

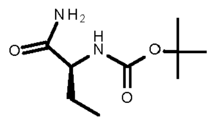

The title compound was synthesized following the same methodology used for the synthesis of Intermediate 7 replacing (2R)-2-(tert-butoxycarbonylamino)butanoic acid with (2S)-2-(tert-butoxycarbonylamino)butanoic acid
LC/MS: QC_3_MIN: m/z 147 [M-tBu+H]+, m/z 427 [2M+Na]+

Intermediate 33

**tert-butyl N-[(1S)-1-[[5-(7-methylspiro[2H-benzofuran-3,1'-cyclopropane]-4-yl)oxypyrazin-2-yl]carbamoyl]propyl]carbamate**

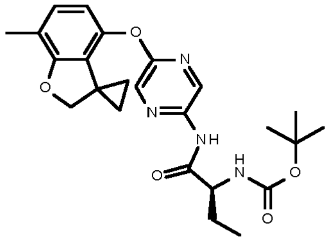

The title compound was synthesized following the "route 1" methodology used for the synthesis of Intermediate 9 replacing tert-butyl N-[(1R)-1-carbamoylpropyl]carbamate (Intermediate 7) with tert-butyl N-[(1S)-1-carbamoylpropyl]carbamate (Intermediate 32).

LC/MS: QC_3_MIN: Rt = 2.65 min; m/z 455 [M+H]+.

Intermediate 34

**(2S)-2-amino-N-[5-(7-methylspiro[2H-benzofuran-3,1'-cyclopropane]-4-yl)oxypyrazin-2-yl]butanamide**

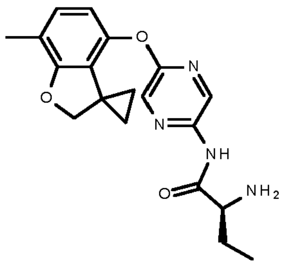

The title compound was synthesized following the same methodology used for the synthesis of Intermediate 13 replacing tert-butyl N-[(1R)-1-[[5-(7-methylspiro[2H-benzofuran-3,1'-cyclopropane]-4-yl)oxypyrazin-2-yl]carbamoyl]propyl]carbamate (Intermediate 9) with tert-butyl N-[(1S)-1-[[5-(7-methylspiro[2H-benzofuran-3,1'-cyclopropane]-4-yl)oxypyrazin-2-yl]carbamoyl]propyl]carbamate (Intermediate 33)

LC/MS: QC_3_MIN: Rt = 1.98 min; m/z 355 [M+H]+.

Example 1 route 1

**5,5-dimethyl-3-[5-(7-methylspiro[2H-benzofuran-3,1'-cyclopropane]-4-yl)oxypyrazin-2-yl]imidazolidine-2,4-dione**

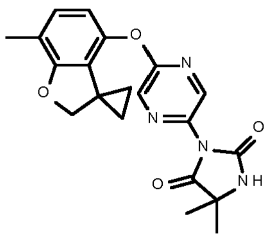

To a solution of 2-bromo-5-(7-methylspiro[2H-benzofuran-3,1'-cyclopropane]-4-yl)oxy-pyrazine (Intermediate 1, 30mg, 0.069mmol) in N,N-dimethylacetamide (1mL) 5,5-dimethylimidazolidine-2,4-dione (44.4mg, 0.345mmol) and copper (I) oxide (5mg, 0.035mmol) were added. The flask was flushed with nitrogen gas and left stirring overnight at 135 °C. The reaction was diluted with EtOAc (10 mL) and first washed with an aqueous saturated solution of ammonium chloride (20 mL) and then brine (20 mL). The organic layer was collected, dried with sodium sulphate and evaporated to dryness. The residue was then purified using flash column chromatography using cyclohexane:ethyl acetate from 80:20 to 40:60 as eluent to afford 5,5-dimethyl-3-[5-(7-methylspiro[2H-benzofuran-3,1'-cyclopropane]-4-yl)oxypyrazin-2-yl]imidazolidine-2,4-dione (17mg) as a white solid.

$^1$H-NMR (400 MHz; DMSO-d6): δ ppm 8.72 (bs, 1H), 8.51 (d, 1H), 8.30 (d, 1H), 6.95 (dd, 1H), 6.53 (d, 1H), 4.46 (s, 2H), 2.14 (s, 3H), 1.42 (s, 6H), 1.07-1.14 (m, 2H),0.89-0.95 (m, 2H).

The following compounds were prepared using the foregoing methodology, replacing 2-bromo-5-(7-methylspiro[2H-benzofuran-3,1'-cyclopropane]-4-yl)oxy-pyrazine (Intermediate 1) with the appropriate bromopyrazine and 5,5-dimethylimidazolidine-2,4-dione with the appropriate hydantoin. Final products were purified by flash-chromatography (Silica cartridge; Cyclohexane/EtOAc or other appropriate solvent system) and/or reverse chromatography (C-18 cartridge; water/acetonitrile or other appropriate solvent system).

| Ex. | Structure | Name | Bromopyrazine | Hydantoin | LCMS/NMR |
|---|---|---|---|---|---|
| 2 | 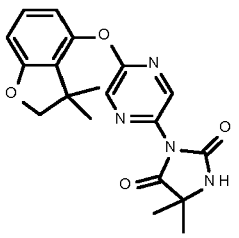 | 3-[5-[(3,3-dimethyl-2H-benzofuran-4-yl)oxy]pyrazin-2-yl]-5,5-dimethyl-imidazolidine-2,4-dione | 2-bromo-5-[(3,3-dimethyl-2H-benzofuran-4-yl)oxy]pyrazine (Intermediate 4) | 5,5-dimethylimidazolidine-2,4-dione | LC/MS: QC_3_MIN: Rt = 2.288 min; m/z 369 [M+H]+. $^1$H-NMR (500 MHz; DMSO-d6): δ ppm 8.73 (bs, 1H), 8.60 (d, 1H), 8.32 (d, 1H), 7.17 (dd, 1H), 6.70 (d, 1H), 6.66 (d, 1H), 4.23 (s, 2H), 1.42 (s, 6H), 1.28 (s, 6H). |
| 3 | 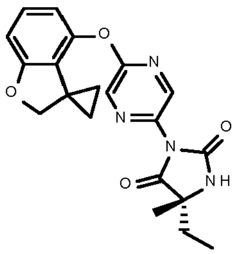 | (5R)-5-ethyl-5-methyl-3-(5-spiro[2H-benzofuran-3,1'-cyclopropane]-4-yloxypyrazin-2-yl) imidazolidine-2,4-dione | 2-bromo-5-spiro[2H-benzofuran-3,1'-cyclopropane]-4-yloxy-pyrazine (Intermediate 2) | (5R)-5-ethyl-5-methylimidazolidine-2,4-dione (Intermediate 17) | LC/MS: QC_3_MIN: Rt = 2.228 min; m/z 381 [M+H]+. |

Example 1 route 2

**5,5-dimethyl-3-[5-(7-methylspiro[2H-benzofuran-3,1'-cyclopropane]-4-yl)oxypyrazin-2-yl]imidazolidine-2,4-dione**

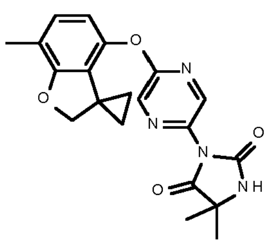

To a solution of 3-(5-chloropyrazin-2-yl)-5,5-dimethyl-imidazolidine-2,4-dione (Intermediate 5, 20mg, 0.083mmol) and 7-methylspiro[2H-benzofuran-3,1'-cyclopropane]-4-ol (Intermediate 156 WO2012076877, 22mg, 0.125mmol) in acetonitrile (1mL), dipotassium carbonate (17.2mg, 0.12mmol) was added. The reaction mixture was stirred overnight at 60°C and then for 3h at 80°C. The reaction mixture was concentrated under vacuum and the crude was purified by flash chromatography on silica gel (BIOTAGE SYSTEM) using a SNAP 10g as column and Chexane/EtOAc from 80/20 to 20/80 as eluent. The fraction were still impure and they were purified by reverse chromatography using a SNAP C-18 as column and H2O/ACN from 95/5 to 5/95 as eluent affording 5,5-dimethyl-3-[5-(7-methylspiro[2H-benzofuran-3,1'-cyclopropane]-4-yl)oxypyrazin-2-yl]imidazolidine-2,4-dione (9.4mg) as a white solid.

LC/MS: QC_3_MIN: Rt = 2.224 min; m/z 381 [M+H]+.

The following compounds were prepared using the foregoing methodology, replacing 7-methylspiro[2H-benzofuran-3,1'-cyclopropane]-4-ol with the appropriate phenol and use 3-(5-chloropyrazin-2-yl)-5,5-dimethyl-imidazolidine-2,4-dione (Intermediate 5) or replace it with the appropriate chloropyrazine intermediate. Final products were purified by flash-chromatography (Silica cartridge; Cyclohexane/EtOAc or other appropriate solvent system) and/or reverse chromatography (C-18 cartridge; water/acetonitrile or other appropriate solvent system).

| Ex. | Structure | Name | Phenol | Chloropyrazine intermediate | LCMS/NMR |
|---|---|---|---|---|---|
| 4 | 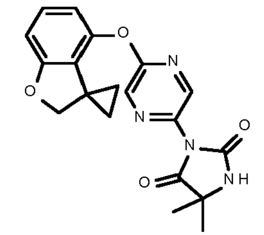 | 5,5-dimethyl-3-(5-spiro[2H-benzofuran-3,1'-cyclopropane]-4-yloxypyrazin-2-yl)imidazolidine-2,4-dione | spiro[2H-benzofuran-3,1'-cyclopropane]-4-ol (Intermediate 85, WO2012/0768 77) | 3-(5-chloropyrazin-2-yl)-5,5-dimethylimidazolidine-2,4-dione (Intermediate 5) | LC/MS: QC_3_MIN: Rt = 2.085 min; m/z 367 [M+H]+. $^{1}$H-NMR (500 MHz; DMSO-d6): δ ppm 8.73 (bs, 1H), 8.54 (d, 1H), 8.32 (d, 1H), 7.11 (dd, 1H), 6.71 (d, 1H), 6.62 (d, 1H), 4.46 (s, 2H), 1.42 (s, 6H), 1.12-1.16 (m, 2H), 0.92-0.97 (m, 5H). |
| 5 | 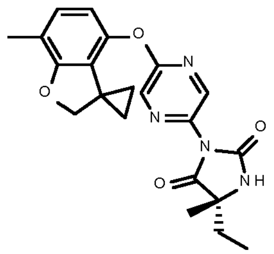 | (5R)-5-ethyl-5-methyl-3-[5-(7-methylspiro[2H-benzofuran-3,1'-cyclopropane]-4-yl) oxypyrazin-2-yl]imidazolidine-2,4-dione | 7-methylspiro[2H -benzofuran-3,1'-cyclopropane]-4-ol (Intermediate 156 WO2012/0768 77) | 5R)-3-(5-chloropyrazin-2-yl)-5-ethyl-5-methylimidazolidine-2,4-dione (Intermediate 6) | LC/MS: QC_3_MIN: Rt = 2.361 min; m/z 395 [M+H]+. $^{1}$H-NMR (500 MHz; DMSO-d6): δ ppm 8.64 (bs, 1H), 8.48 (d, 1H), 8.25 (d, 1H), 6.91 (dd, 1H), 6.49 (d, 1H), 4.42 (s, 2H), 2.11 (s, 3H), 1.71-1.79 (m, 1H), 1.60-1.68 (m, 1H), 1.38 (s, 3H), 1.02-1.09 (m, 2H), 0.82-0.92 (m, 5H). |
| 6 | 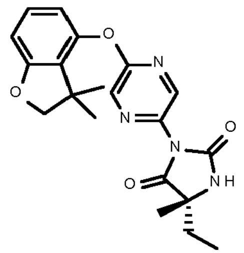 | (5R)-3-[5-[(3,3-dimethyl-2H-benzofuran-4-yl)oxy]pyrazin-2-yl]-5-ethyl-5-methylimidazolidine-2,4-dione | 3,3-dimethyl-2H-benzofuran-4-ol (Intermediate 50 WO2012/0768 77) | 5R)-3-(5-chloropyrazin-2-yl)-5-ethyl-5-methylimidazolidine-2,4-dione (Intermediate 6) | LC/MS: QC_3_MIN: Rt = 2.008 min; m/z 383 [M+H]+. |
| 7 | 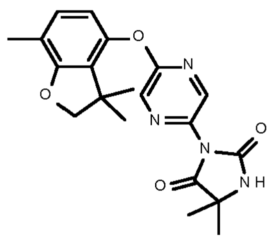 | 5,5-dimethyl-3-[5-[(3,3,7-trimethyl-2H-benzofuran-4-yl) oxy]pyrazin-2-yl]imidazolidine-2,4-dione | 3,3,7-trimethyl-2H-benzofuran-4-ol (Intermediate 184 WO2012/0768 77) | 3-(5-chloropyrazin-2-yl)-5,5-dimethylimidazolidine-2,4-dione (Intermediate 5) | LC/MS: QC_3_MIN: Rt = 2.025 min; m/z 383 [M+H]+. |

(continued)

| Ex. | Structure | Name | Phenol | Chloropyrazine intermediate | LCMS/NMR |
|---|---|---|---|---|---|
| 8 | 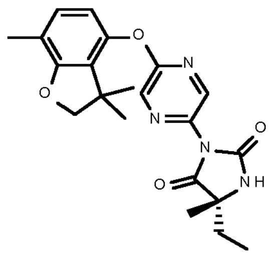 | (5R)-5-ethyl-5-methyl-3-[5-[(3,3,7-trimethyl-2H-benzofuran-4-yl)oxy]pyrazin-2-yl]imidazolidine-2,4-dione | 3,3,7-trimethyl-2H-benzofuran-4-ol (Intermediate 184 WO2012/0768 77) | (5R)-3-(5-chloropyrazin-2-yl)-5-ethyl-5-methylimidazolidine-2,4-dione (Intermediate 6) | LC/MS: QC_3_MIN: Rt = 2.111 min; m/z 397 [M+H]+. |

Example 9 (route 1)

**(5R)-5-ethyl-3-[5-(7-methylspiro[2H-benzofuran-3,1'-cyclopropane]-4-yl)oxypyrazin-2-yl]imidazolidine-2,4-dione**

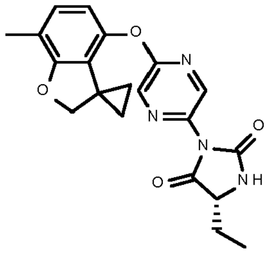

A mixture of (2R)-2-amino-N-[5-(7-methylspiro[2H-benzofuran-3,1'-cyclopropane]-4-yl)oxypyrazin-2-yl]butanamide (Intermediate 13, 13mg, 0.037mmol) and N,N-diethylethanamine (11mg, 0.11mmol) in dichloromethane (2mL) was cooled to 0°C. A solution of bis(trichloromethyl) carbonate (4,5mg, 0.015mmol) in dichloromethane (0.5mL) was added dropwise and the reaction mixture was stirred for 1 hour at the same temperature. Additional bis(trichloromethyl) carbonate (1.5mg) in dichloromethane (0.5mL) was added and stirring continued for 30 minutes. The mixture was allowed to warm to room temperature. The reaction mixture was diluted with dichloromethane (20 ml) and the organic phase was washed with an aqueous solution 0.1N HCl (20 ml) and then with brine (20 ml). Phases were separated and the organic layer was dried over $Na_2SO_4$, filtered and evaporated. The residue was purified by reverse phase chromatography using a SNAP C-18 column, eluting with water:acetonitrile from 90:10 to 0:100. The appropriate fractions were combined and evaporated to dryness, affording (5R)-5-ethyl-3-[5-(7-methylspiro[2H-benzofuran-3,l'-cyclopropane]-4-yl)oxypyrazin-2-yl]imidazolidine-2,4-dione (7.5mg) as a white solid.

LC/MS: QC_3_MIN: Rt = 2.305 min; m/z 381 [M+H]+. Enantiomeric purity was confirmed as >95% using Chiral Control method.

Example 9 (route 2)

**(5R)-5-ethyl-3-[5-(7-methylspiro[2H-benzofuran-3,1'-cyclopropane]-4-yl)oxypyrazin-2-yl]imidazolidine-2,4-dione**

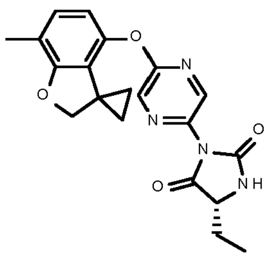

To a solution of (2R)-2-amino-N-[5-(7-methylspiro[2H-benzofuran-3,1'-cyclopropane]-4-yl)oxypyrazin-2-yl]butanamide (Intermediate 13, 21g, 59.26mmol) in ethyl acetate (500mL) 1-1'-carbonyldiimidazole (10.57g, 65.18mmol) was added in 5 portions of about 2g each, and stirred at room temperature for 4h. The reaction was quenched with ice and an aqueous 0.2N solution of HCl (250ml) was added. The two phases were separated and the organic layer was washed with an aqueous 0.2N solution of HCl (250ml) and with brine (200ml), then dried with sodium sulphate, filtered and evaporated to dryness. The crude was split into 4 aliquots of ~4.2g each and each aliquot was purified by flash chromatography on silica gel using a SNAP (100G) as column and Cyclohexane/Ethyl acetate from 80/20 to 20/80 as eluent. The desired fractions from each run were collected and the solvent evaporated to dryness. The obtained light-yellow solid was suspended in a solution of Cyclohexane/Ethyl acetate (1/1, 3volumes) (90ml) and stirred for 2h at 50°C. The mixture was then allowed to cool to room temperature and filtered under vacuum. The wet cake was washed with

ice cold cyclohexane (15ml), the solid was collected and dried to afford the title compound (5R)-5-ethyl-3-[5-(7-methylspiro[2H-benzofuran-3,1'-cyclopropane]-4-yl)oxypyrazin-2-yl]imidazolidine-2,4-dione (13.6g) as a white solid. $^1$H-NMR (500 MHz; DMSO-d6): δ ppm 8.69 (bs, 1H), 8.52 (d, 1H), 8.26 (d, 1H), 6.94 (d, 1H), 6.53 (d, 1H), 4.46 (s, 2H), 4.26-4.30 (m, 1H), 2.14 (s, 3H), 1.77-1.86 (m, 1H), 1.65-1.76 (m, 1H), 1.07-1.12 (m, 2H), 0.90-0.99 (m, 5H).

The following compounds were prepared using the foregoing methodology (either route 1 or route 2), replacing (2R)-2-amino-N-[5-(7-methylspiro[2H-benzofuran-3,1'-cyclopropane]-4-yl)oxypyrazin-2-yl]butanamide (Intermediate 13) with the appropriate butanamide. Final products were purified by flash-chromatography (Silica cartridge; Cyclohexane/EtOAc or other appropriate solvent system) and/or reverse chromatography (C-18 cartridge; water/acetonitrile or other appropriate solvent system).

| Ex. | Structure | Name | Butanamide | LCMS/NMR |
|---|---|---|---|---|
| 10 | 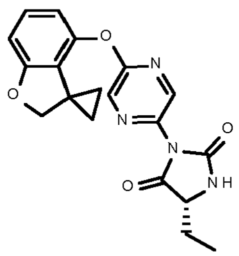 | (5R)-5-ethyl-3-(5-spiro[2H-benzofuran-3,1'-cyclopropane]-4-yloxypyrazin-2-yl) imidazolidine-2,4-dione | (2R)-2-amino-N-(5-spiro[2H-benzofuran-3,1'-cyclopropane]-4-yloxypyrazin-2-yl) butanamide (Intermediate 14) | LC/MS: QC_3_MIN: Rt = 2.081 min; m/z 367 [M+H]+. Enantiomeric purity was confirmed as >95% using Chiral Control method. $^1$H-NMR (500 MHz; DMSO-d6): δ ppm 8.70 (bs, 1H), 8.55 (d, 1H), 8.27 (d, 1H), 7.11 (dd, 1H), 6.71 (dd, 1H), 6.62 (dd, 1H), 4.46 (s, 2H), 4.27-4.31 (m, 1H), 1.76-1.87 (m, 1H), 1.65-1.76 (m, 1H), 1.11-1.17 (m, 2H), 0.92-0.98 (m, 5H). |
| 11 | 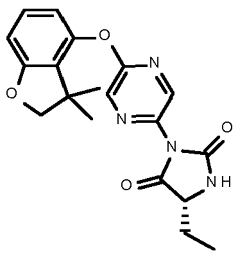 | (5R)-3-[5-[(3,3-dimethyl-2H-benzofuran-4-yl) oxy]pyrazin-2-yl]-5-ethylimidazolidine-2,4-dione | (2R)-2-amino-N-[5-[(3,3-dimethyl-2H-benzofuran-4-yl) oxy]pyrazin-2-yl] butanamide (Intermediate 16) | LC/MS: QC_3_MIN: Rt = 2.142 min; m/z 369 [M+H]+. |
| 12 | 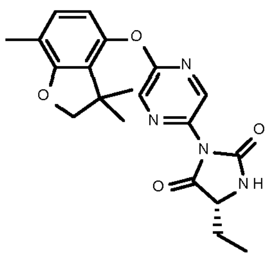 | (5R)-5-ethyl-3-[5-[(3,3,7-trimethyl-2H-benzofuran-4-yl) oxy]pyrazin-2-yl] imidazolidine-2,4-dione | (2R)-2-amino-N-[5-[(3,3,7-trimethyl-2H-benzofuran-4-yl) oxy]pyrazin-2-yl] butanamide (Intermediate 15) | LC/MS: QC_3_MIN: Rt = 2.111 min; m/z 383 [M+H]+. |
| 13 | 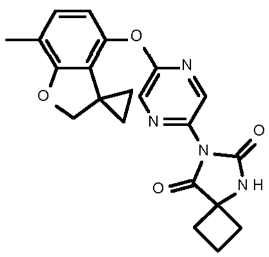 | 7-[5-(7-methylspiro [2H-benzofuran-3,1'-cyclopropane]-4-yl)oxypyrazin-2-yl]-5,7-diazaspiro [3.4]oct ane-6,8-dione | 1-amino-N-[5-(7-methylspiro[2H-benzofuran-3,1'-cyclopropane]-4-yl)oxypyrazin-2-yl]cyclobutanecar boxamide (Intermediate 20) | LC/MS: QC_3_MIN: Rt = 2.309 min; 393 m/z [M+H]+. |

(continued)

| Ex. | Structure | Name | Butanamide | LCMS/NMR |
|---|---|---|---|---|
| 14 | 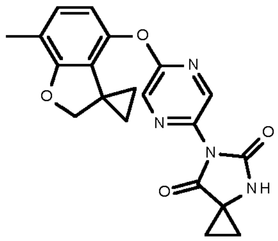 | 6-[5-(7-methylspiro [2H-benzofuran-3,1'-cyclopropane]-4-yl)oxypyrazin-2-yl]-4,6-diazaspiro [2.4]he ptane-5,7-dione | 1-amino-N-[5-(7-methylspiro[2H-benzofuran-3,1'-cyclopropane]-4-yl)oxypyrazin-2-yl] cyclopropaneca rboxamide | LC/MS: QC_3_MIN: Rt = 2.236 min; 379 m/z [M+H]+. |

Example 15

**(5S)-5-ethyl-3-[5-(7-methylspiro[2H-benzofuran-3,1'-cyclopropane]-4-yl)oxypyrazin-2-yl]imidazolidine-2,4-dione**

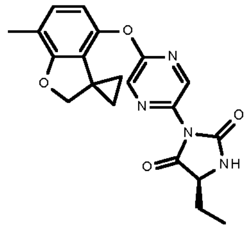

The title compound was synthesized following the "route 1" methodology used for the synthesis of Intermediate 9 replacing (2R)-2-amino-N-[5-(7-methylspiro[2H-benzofuran-3,1'-cyclopropane]-4-yl)oxypyrazin-2-yl]butanamide (Intermediate 13) with (2S)-2-amino-N-[5-(7-methylspiro[2H-benzofuran-3,1'-cyclopropane]-4-yl)oxypyrazin-2-yl]butanamide (Intermediate 34)

LC/MS: QC_3_MIN: Rt = 2.29 min; m/z 381 [M+H]+.

Biological Examples

Biological Example 1: Measurement of Kv3.1, Kv3.2 and Kv3.3 channel modulation

The ability of the compounds of the invention to modulate the voltage-gated potassium channel subtypes Kv3.3/Kv3.2/Kv3.1 may be determined using the following assay. Analogous methods may be used to investigate the ability of the compounds of the invention to modulate other channel subtypes.

*Cell biology*

To assess compound effects on human Kv3.3 channels (hKv3.3), a stable cell line expressing human Kv3.3 channels is created by transfecting Chinese Hamster Ovary (CHO)-K1 cells with a pBacMire_KCNC-3 vector. Cells are cultured in DMEM/F12 (Gibco) supplemented with 10% Foetal Bovine Serum (Gibco), 1X non-essential amino acids (Invitrogen) and geneticin (G418) 400 microg/mL. Cells are grown and maintained at 37 °C in a humidified environment containing 5% $CO_2$ in air.

To assess compound effects on human Kv3.2 channels (hKv3.2), a stable cell line expressing human Kv3.2 channels (hKv3.2) is created by transfecting CHO-K1 cells with a pCIH5-hKv3.2 vector. Cells are cultured in DMEM/F12 medium supplemented by 10% Foetal Bovine Serum, 1X non-essential amino acids (Invitrogen) and 500ug/ml of Hygromycin-B (Invitrogen). Cells are grown and maintained at 37 °C in a humidified environment containing 5% $CO_2$ in air.

To assess compound effects on human Kv3.1 channels (hKv3.1):

Human embryonic kidney (HEK)-hKv3.1 cell line is generated by transfecting HEK-293 cells with an expression

vector with human Kv3.1 (NM_004976.4). Cells are cultured with MEM supplemented with 10% Heat-Inactivated FBS, 2 mM L-glutamine, 1% Penicillin-Streptomycin, and 0.6 mg/ml of Geneticin (G418). HEK-hKv3.1b cells were amplified in T175 cm2 flask at 37°C with 5% CO2, using MEM amplification medium, containing the G418 selection antibiotic (0.6mg/ml). Cells were detached every 3-4 days, using DPBS to wash twice the flask, then TrypLE to dislodge the cells, and re-plated at a density of 2-4x106 cells/flask.

*Cell preparation for Ion Works Quattro™ experiments*

The day of the experiment, cells are removed from the incubator and the culture medium removed. Cells are washed with 5 ml of Dulbecco's PBS (DPBS) calcium and magnesium free and detached by the addition of 3 ml Versene (Invitrogen, Italy) followed by a brief incubation at 37 °C for 5 minutes. The flask is tapped to dislodge cells and 10 ml of DPBS containing calcium and magnesium is added to prepare a cell suspension. The cell suspension is then placed into a 15 ml centrifuge tube and centrifuged for 2 min at 1200 rpm. After centrifugation, the supernatant is removed and the cell pellet re-suspended in 4 ml of DPBS containing calcium and magnesium using a 5 ml pipette to break up the pellet. Cell suspension volume is then corrected to give a cell concentration for the assay of approximately 3 million cells per ml.

All the solutions added to the cells are pre-warmed to 37 °C.

*Electrophysiology*

*Ionworks*

Experiments are conducted at r.t. using IonWorks Quattro™ planar array electrophysiology technology (Molecular Devices Corp.) with PatchPlate™ PPC. Stimulation protocols and data acquisition are carried out using a microcomputer (Dell Pentium 4). Planar electrode hole resistances (Rp) are determined by applying a 10 mV voltage step across each well. These measurements are performed before cell addition. After cell addition and seal formation, a seal test is performed by applying a voltage step from -80 mV to -70 mV for 160 ms. Following this, amphotericin-B solution is added to the intracellular face of the electrode to achieve intracellular access. Cells are held at -70 mV. Leak subtraction is conducted in all experiments by applying 50 ms hyperpolarizing (10 mV) prepulses to evoke leak currents followed by a 20 ms period at the holding potential before test pulses.

For hKv3.2 and hKv3.1, assays from the holding potential of -70 mV, a first test pulse at -15 mV was applied for 100 ms and after 100 ms at -70 mV a second pulse at +40 mV was applied for 50 ms. Cells were then maintained for 100 ms at -100 mV and another pulse from -70mV to +40 mV (duration 50 ms) was applied to clamp later the voltage at -40 mV during 200ms

For hKv3.3 assays, from the holding potential of -70 mV, a first test pulse to 0 mV is applied for 500 ms and following a further 100 ms at -70 mV, a second pulse to 40 mV is applied for 200 ms. These longer test pulses are used to study inactivation of hKv3.3 channels. Test pulses protocol may be performed in the absence (pre-read) and presence (post-read) of the test compound. Pre- and post-reads may be separated by the compound addition followed by a 3 minute incubation.

*Solutions and drugs*

The intracellular solution contains the following (in mM): K-gluconate 100, KCl 54, $MgCl_2$ 3.2, HEPES 5, adjusted to pH 7.3 with KOH. Amphotericin-B solution is prepared as 50mg/ml stock solution in DMSO and diluted to a final working concentration of 0.1 mg/ml in intracellular solution. The external solution is Dulbecco's Phosphate Buffered Saline (DPBS) and contained the following (in mM): $CaCl_2$ 0.90, KCl 2.67, $KH_2PO_4$ 1.47, $MgCl.6H_2O$ 0.493, NaCl 136.9, $Na_3PO_4$ 8.06, with a pH of 7.4.

Compounds of use in the invention (or reference compounds such as *N*-cyclohexyl-*N*-[(7,8-dimethyl-2-oxo-1,2-dihydro-3-quinolinyl)methyl]-*N*'-phenylurea) are dissolved in dimethylsulfoxide (DMSO) at a stock concentration of 10 mM. These solutions are further diluted with DMSO using a Biomek FX (Beckman Coulter) in a 384 compound plate. Each dilution (1 μL) is transferred to another compound plate and external solution containing 0.05% pluronic acid (66 μL) is added. 3.5 μL from each plate containing a compound of the invention is added and incubated with the cells during the IonWorks Quattro™ experiment. The final assay dilution is 200 and the final compound concentrations are in the range 50 μM to 50 nM.

*Data analysis*

The recordings are analysed and filtered using both seal resistance (>20 MΩ) and peak current amplitude

(>500 pA at the voltage step of 40 mV) in the absence of compound to eliminate unsuitable cells from further analysis. For hKv3.2 and hKv3.1 assays, paired comparisons of evoked currents between pre- and post-drug additions measured for the -15 mV voltage step are used to determine the positive modulation effect of each compound. Kv3 channel-mediated outward currents are measured determined from the mean amplitude of the current over the final 10 ms of the -15 mV voltage pulse minus the mean baseline current at -70 mV over a 10 ms period just prior to the -15 mV step. These Kv3 channel currents following addition of the test compound are then compared with the currents recorded prior to compound addition. Data are normalised to the maximum effect of the reference compound (50microM of *N*-cyclohexyl-*N*-[(7,8-dimethyl-2-oxo-1,2-dihydro-3-quinolinyl)methyl]-*N*'-phenylurea) and to the effect of a vehicle control (0.5% DMSO). The normalised data are analysed using ActivityBase or Excel software. The concentration of compound required to increase currents by 50% of the maximum increase produced by the reference compound (ECso) is determined by fitting of the concentrationresponse data using a four parameter logistic function in ActivityBase. For hKv3.3 assays, paired comparisons of evoked currents between pre- and post-drug additions are measured for the 0mV step, considering the peak current and the decay (inactivation) of the current over the duration of the 0mv test pulse (500 ms).

*N*-cyclohexyl-*N*-[(7,8-dimethyl-2-oxo-1,2-dihydro-3-quinolinyl)methyl]-*N*'-phenylurea is obtained from ASINEX (Registry Number: 552311-06-5).

| Ex. | Compound | Kv3.1 pEC50 | Kv3.1 max R% | Reference / LCMS |
|---|---|---|---|---|
| RE1 | 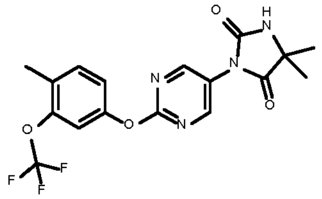 | 4.78 | 105 | Ex57 WO2011/069951 |
| RE2 | 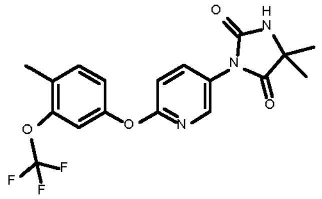 | 5.25 | 118 | Ex45 WO2011/069951 |
| RE3 | 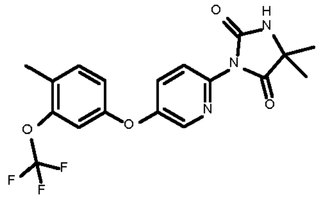 | 4.89 | 80 | LC/MS: QC_3_MIN: Rt = 2.376 min; m/z 396 [M+H]+. |
| RE4 | 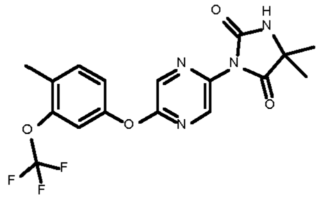 | <4.3 | 24 | LC/MS: QC_3_MIN: Rt = 2.346 min; m/z 397 [M+H]+. |

As shown by testing of RE1-RE4, the incorporation of a pyrazine ring can detrimentally impact the pEC50 and maxR of Kv3.1 modulators.

| Ex. | Compound | Kv3.1 pEC50 | Kv3.1 max R% | Reference / LCMS |
|---|---|---|---|---|
| RE5 | 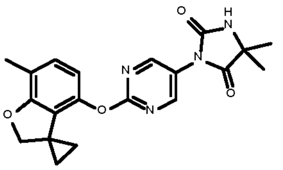 | 5.16 | 155 | Ex58 WO2012/076877 |
| RE6 | 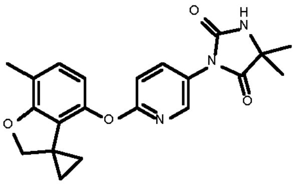 | 5.55 | 139 | Ex70 WO2012/076877 |
| RE7 | 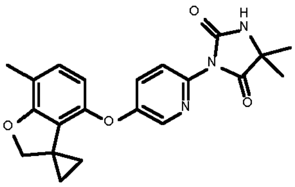 | 5.54 | 121 | Ex3 WO2017/103604 |
| RE8 | 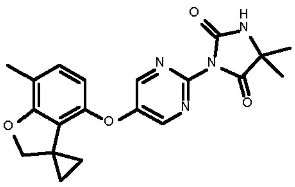 | 4.98 | 42 | LC/MS: QC_3_MIN: Rt = 2.224 min; m/z 381 [M+H]+. |
| RE9 | 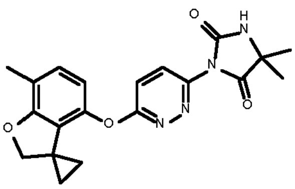 | <4.3 | 16 | LC/MS: QC_3_MIN: Rt = 2.043 min; m/z 381 [M+H]+. |
| 1⁺ | 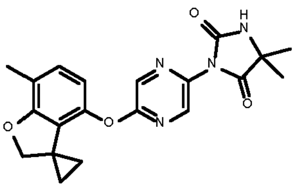 | 5.47 | 164 | Example 1 |
| ⁺ *n=10. For n=18,* pEC50 was 5.56 and maxR% 152 | | | | |

As shown by testing of RE5-RE9 as compared to Example 1, the incorporation of a pyrazine ring in Example 1 unexpectedly results in high pEC50 and high maxR in the Kv3.1 assay.

| Example | Kv3.1 pEC50 | Kv3.1 max R% |
|---|---|---|
| 1⁺ | 5.47 | 164 |
| 2 | 4.68 | 149 |
| 3 | 5.15 | 205 |
| 4 | 5.17 | 170 |
| 5 | 5.69 | 149 |

(continued)

| Example | Kv3.1 pEC50 | Kv3.1 max R% |
|---|---|---|
| 6 | 4.75 | 165 |
| 7 | 5.12 | 134 |

| Example | Kv3.1 pEC50 | Kv3.1 max R% |
|---|---|---|
| 8 | 5.29 | 119 |
| 9* | 5.88 | 172 |
| 10$ | 5.45 | 153 |
| 11 | 4.89 | 165 |
| 12 | 5.56 | 118 |
| 13 | 5.09 | 165 |
| 14 | 5.51 | 145 |
| 15 | 5.10 | 136 |
| + *n=10. For n=18,* pEC50 was 5.56 and maxR% 152<br>* *n=4. For n=22,* pEC50 was 5.90 and maxR% 146<br>$ *n=2. For n=26,* pEC50 was 5.63 and maxR% 147 | | |

All tested examples of the compounds of formula (I) are shown above and demonstrate good pEC50 and maxR properties in the Kv3.1 assay.

A secondary analysis of the data from the hKv3.1, hKv3.2 and hKv3.3 assays described in may be used to investigate the effect of the compounds on rate of rise of the current from the start of the depolarising voltage pulses. The magnitude of the effect of a compound can be determined from the time constant ($Tau_{act}$) obtained from a non-linear fit, using the equation given below, of the rise in Kv3.1, Kv3.2 and Kv3.3 currents following the start of the -15mV depolarising voltage pulse.

$$Y = (Y0 - Ymax) * \exp(-K*X) + Ymax$$

where:

Y0 is the current value at the start of the depolarising voltage pulse;
Ymax is the plateau current;
K is the rate constant, and $Tau_{act}$ is the activation time constant, which is the reciprocal of K.

Similarly, the effect of the compounds on the time taken for Kv3.1, Kv3.2 or Kv3.3 currents to decay on closing of the channels at the end of the -15mV depolarising voltage pulses can also be investigated. In this latter case, the magnitude of the effect of a compound on channel closing can be determined from the time constant ($Tau_{deact}$) of a non-linear fit of the decay of the current ("tail current") immediately following the end of the depolarising voltage pulse.

Kv3.1, Kv3.2 and Kv3.3 channels must activate and deactivate very rapidly in order to allow neurons to fire actions potentials at high frequency (Rudy *et al.,* 2001). Slowing of activation is likely to delay the onset of action potential repolarisation; slowing of deactivation could lead to hyperpolarising currents that reduce the excitability of the neuron and delay the time before the neuron can fire a further action potential. Together these two slowing effects on channel activation and deactivation are likely to lead to a reduction rather than a facilitation of the neurons ability to fire at high frequencies. Thus compounds that have this slowing effect on the Kv3.1 and/or Kv3.2, and/or Kv3.3 channels will effectively behave as negative modulators of the channels, leading to a slowing of neuronal firing. This latter effect has been shown for certain of the compounds disclosed in WO2011/069951, where marked increases in $Tau_{act}$ can be observed from recordings made from "fast-firing" interneurons in the cortex of rat brain, using electrophysiological techniques, *in vitro.* The addition of the relevant compounds reduces the ability of the neurons to fire in response to trains

of depolarising pulses at 300Hz.

Therefore, although certain compounds may be identified act as positive modulators in the recombinant cell assay, those compounds which markedly increase the value of $Tau_{act}$ can reduce the ability of neurons in native tissues to fire at high frequency.

Biological Example 2: Determination of blood and brain tissue binding

*Materials and Methods*

Rat whole blood, collected on the week of the experiment using K3-EDTA as an anti-coagulant, is diluted with isotonic phosphate buffer 1:1 (v/v). Rat whole brain, stored frozen at -20 °C, is thawed and homogenised in artificial cerebrospinal fluid (CSF) 1 :2 (w/v).

An appropriate amount of test compound is dissolved in DMSO to give a 5 millimolar solution. Further dilutions, to obtain a 166.7 micromolar working solution are then prepared using 50% acetonitrile in MilliQ water. This working solution is used to spike the blood to obtain a final concentration of 0.5micromolar in whole blood. Similarly, the working solution is used to spike brain samples to obtain a final concentration of 5 micromolar in whole brain. From these spiked blood and brain preparations, control samples (n=3), are immediately extracted and used to calculate the initial recovery of the test items.

150 microL of compound-free buffer (isotonic phosphate buffer for blood or artificial CSF buffer for brain) is dispensed in one half-well and 150 microL of spiked matrix (blood or brain) is loaded in the other half-well, with the two halves separated by a semi-permeable membrane. After an equilibration period of 5 h at 37°C, 50 microL of dialysed matrix (blood or brain) is added to 50 microL of corresponding compound-free buffer, and vice-versa for buffer, such that the volume of buffer to matrix (blood or brain) remains the same. Samples are then extracted by protein precipitation with 300 microL of acetonitrile containing rolipram (control for positive ionization mode) or diclofenac (control for negative ionization mode) as internal standards and centrifuged for 10min at 2800rpm. Supernatants are collected (100 microL), diluted with 18% ACN in MilliQ water (200 microL) and then injected into an HPLC-MS/MS or UPLC-MS/MS system to determine the concentration of test compound present.

*Analysis*

Blood and brain tissue binding are then determined using the following formulas:

$$\text{Afu=Buffer/Blood or Afu=CSF/Brain}$$

Where Afu = apparent fraction unbound; Buffer= analyte/intemal standard ratio determined in the buffer compartment; Blood= analyte/intemal standard ratio determined in the blood compartment; Brain= analyte/intemal standard ratio determined in the brain compartment.

$$\text{Fucr} = \frac{1/D}{[(1/\text{Afu} - 1)+1/D]}$$

where: fucr = Fraction unbound corrected; D =matrix dilution factor (D=2 for blood and D=3 for brain).
Then:

$$\%\text{Binding} = (1 - \text{fucr}) \times 100$$

$$\%\text{Unbound} = 100 - \%\text{Bound}$$

*Brain/Blood partition ratio (Kbb) Determination*

For compounds freely permeable across the blood/brain barrier (BBB), the unbound concentrations in blood and brain would be equivalent under steady-state distribution conditions. Therefore, the Kbb value could be calculated as:

$$Fu(blood)/Fu(brain)$$

which is expected to be equivalent to the brain-to-blood concentration ratio (Ct(brain)/Ct(blood)) if efflux pump transporters are not involved.

Biological Example 3: Determination of *in vivo* pharmacokinetic parameters

*Materials and Methods*

Adult male rats (Charles River, Italy) are dosed with test compound orally at 1 mg/kg (5 ml/kg, in 5% v/v DMSO, 0.5% w/v HPMC in water) and intravenously at 0.5mg/kg (2ml/kg, in 5% v/v DMSO 40% w/v PEG400 in saline). After oral administration, blood samples are collected under deep Isofluorane anesthesia from the portal vein and heart of each rat (1 rat per time point). After intravenous administration, serial blood samples are collected from the lateral tail vein of each rat. A further group of rats (n=1 per test compound) receive a single intravenous administration of the PgP transport inhibitor, Elacridar (3 mg/kg) shortly before the oral administration of the test compound at 1 mg/kg, as above. Blood and brain samples ar collected at a single timepoint of 0.5 h after dose administration for these animals. In all cases, blood samples are collected into potassium EDTA tubes.

Blood and brain samples can be assayed for test compound concentration using a method based on protein precipitation with acetonitrile followed by HPLC/MS-MS analysis with an optimized analytical method.

*Analysis*

The concentrations of test compound in blood (expressed as ng/ml) and brain (expressed as ng/g) at the different time points following either oral or intravenous dosing are analysed using a noncompartmental pharmacokinetic model using WinNonLin Professional version 4.1. The following parameters are derived:

Intravenous dosing: Maximum concentration over time (Cmax), integrated concentration over time (AUC), clearance (Clb), volume of distribution (Vss) and half-life (t1/2).

Oral dosing: Cmax, time of maximum concentration (Tmax), AUC, bioavailability (F%), fraction absorbed (Fa%), blood to brain ratio (AUC BB), and Fold-change in AUC BB in the presence of Elacridar.

Compounds of the invention may be expected to demonstrate good availability in brain tissue.

Additional animal models

Patent applications WO2011/069951, WO2012/076877, WO2012/168710, WO2013/083994 WO2013/175215 and WO2013/182851 (all incorporated by reference for the purpose of illustrating the potential utility of the compounds and providing animal models for the testing of compounds) demonstrate the activity of compounds which are modulators of Kv3.1 and Kv3.2 in animal models of seizure, hyperactivity, sleep disorders, psychosis, hearing disorders and bipolar disorders.

Patent application WO2013/175211 (incorporated by reference for the purpose of illustrating the potential utility of the compounds and providing animal models for the testing of compounds) demonstrates the efficacy of a compound which is a modulator of Kv3.1 and Kv3.2 in a model of acute noise-induced hearing loss in the chinchilla, and also evaluates the efficacy of the compound in a model of central auditory processing deficit and in a model of tinnitus.

Glait et al 2018, Anderson et al 2018 and Chamber et al 2018 demonstrate the efficacy of a modulator of Kv3.1 and Kv3.2 in hearing associated models.

Patent application WO2017/098254 (incorporated by reference for the purpose of illustrating the potential utility of the compounds and providing animal models for the testing of compounds) demonstrates the efficacy of a compound which is a modulator of Kv3.1 and Kv3.2 in models of neuropathic and inflammatory pain.

Throughout the specification and the claims which follow, unless the context requires otherwise, the word ’comprise’, and variations such as ’comprises’ and ’comprising’, will be understood to imply the inclusion of a stated integer, step, group of integers or group of steps but not to the exclusion of any other integer, step, group of integers or group of steps.

References

Anderson LA et al. Increased spontaneous firing rates in auditory midbrain following noise exposure are specifically abolished by a Kv3 channel modulator. Hear Res. 2018 Aug;365:77-89 Aroniadou-Anderjaska V et al. Mechanisms regulating GABAergic inhibitory transmission in the basolateral amygdala: implications for epilepsy and anxiety disorders. Amino Acids 2007 Aug;32:305-315.

Baranauskas G, Nistri A. Sensitization of pain pathways in the spinal cord: cellular mechanisms. Prog. Neurobiol. 1998 Feb;54(3):349-65.

Baron R et al. Peripheral input and its importance for central sensitization. Ann. Neurol. 2013 Nov;74(5):630-6.

Ben-Ari Y. Seizure Beget Seizure: The Quest for GABA as a Key Player. Crit. Rev. Neurobiol. 2006;18(1-2): 135-144.

Benes FM et al. Circuitry-based gene expression profiles in GABA cells of the trisynaptic pathway in schizophrenics versus bipolars. PNAS 2008 Dec;105(52):20935-20940.

Bennett DL, Woods CG. Painful and painless channelopathies. Lancet Neurol. 2014 Jun;13(6):587-99.

Berge S etal. Pharmaceutical Salts. J. Pharm. Sci. 1977;66;1-19.

Brambilla P et al. GABAergic dysfunction in mood disorders. Mol. Psych. 2003 Apr;8:721-737.

Brooke RE et al. Spinal cord interneurones labelled transneuronally from the adrenal gland by a GFP-herpes virus construct contain the potassium channel subunit Kv3.1b. Auton. Neurosci. 2002 Jun;98(1-2):45-50.

Brooke RE et al. Association of potassium channel Kv3.4 subunits with pre- and post-synaptic structures in brainstem and spinal cord. Neuroscience 2004;126(4):1001-10.

Brooke RE et al. Immunohistochemical localisation of the voltage gated potassium ion channel subunit Kv3.3 in the rat medulla oblongata and thoracic spinal cord. Brain Res. 2006 Jan;1070(1):101-15.

Cervero F. Spinal cord hyperexcitability and its role in pain and hyperalgesia. Exp. Brain Res. 2009 Jun;196(1):129-37.

Chambers AR et al. Pharmacological modulation of Kv3.1 mitigates auditory midbrain temporal processing deficits following auditory nerve damage. Sci Rep. 2017 Dec 13;7(1):17496

Chang SY et al. Distribution of Kv3.3 Potassium Channel Subunits in Distinct Neuronal Populations of Mouse Brain. J. Comp. Neuro. 2007 Feb;502:953-972.

Chien LY et al. Reduced expression of A-type potassium channels in primary sensory neurons induces mechanical hypersensitivity. J. Neurosci. 2007 Sep;27(37):9855-65.

Chow A et al. K+ Channel Expression Distinguishes Subpopulations of Parvalbumin- and Somatostatin-Containing Neocortical Interneurons. J. Neurosci. 1999 Nov;19(21):9332-9345.

Desai R et al. Protein Kinase C Modulates Inactivation of Kv3.3 Channels. J. Biol. Chem. 2008;283;22283-22294.

Deuchars SA et al. Properties of interneurones in the intermediolateral cell column of the rat spinal cord: role of the potassium channel subunit Kv3.1. Neuroscience 2001;106(2):433-46.

Devulder J. Flupirtine in pain management: pharmacological properties and clinical use. CNS Drugs 2010 Oct;24(10):867-81.

Dib-Hajj SD et al. The Na(V)1.7 sodium channel: from molecule to man. Nat. Rev. Neurosci. 2013 Jan;14(1):49-62.

Diochot S et al. Sea Anemone Peptides with a Specific Blocking Activity against the Fast Inactivating Potassium Channel Kv3.4. J. Biol. Chem. 1998 Mar;273(12);6744-6749.

Engel AK et al. Dynamic Predictions: Oscillations and Synchrony in Top-Down Processing. Nat. Rev. Neurosci. 2001 Oct;2(10):704-716.

Espinosa F et al. Alcohol Hypersensitivity, Increased Locomotion, and Spontaneous Myoclonus in Mice Lacking the Potassium Channels Kv3.1 and Kv3.3. J. Neurosci. 2001 Sep;21(17):6657-6665.

Espinosa F et al. Ablation of Kv3.1 and Kv3.3 Potassium Channels Disrupts Thalamocortical Oscillations In Vitro and In Vivo. J. Neurosci. 2008 May;28(21):5570-5581.

Figueroa K et al. KCNC3: phenotype, mutations, channel biophysics - a study of 260 familial ataxia patients. Human Mutation. 2010;31;191-196.

Finnerup NB et al. Pharmacotherapy for neuropathic pain in adults: a systematic review and meta-analysis. Lancet Neurol. 2015 Feb;14(2):162-73.

Fisahn A. Kainate receptors and rhythmic activity in neuronal networks: hippocampal gamma oscillations as a tool. J. Physiol. 2005 Oct;561 (1):65-72.

Glait L et al. Effects of AUT00063, a Kv3.1 channel modulator, on noise-induced hyperactivity in the dorsal cochlear nucleus. Hear Res. 2018 Apr;361:36-44

Greene TW, Wuts, PG. Greene's Protective Groups in Organic Synthesis, 2006, 4th Edition, John Wiley & Sons, Inc., Hoboken, NJ, USA.

Joho RH et al. Increased $\gamma$- and Decreased $\delta$-Oscillations in a Mouse Deficient for a Potassium Channel Expressed

in Fast-Spiking Interneurons. J. Neurophysiol. 1999 Jun;82:1855-1864.

Joho RH, Hurlock EC. The Role of Kv3-type Potassium Channels in Cerebellar Physiology and Behavior. Cerebellum 2009 Feb;8:323-333.

Jung D et al. Age-related changes in the distribution of Kv1.1 and Kv3.1 in rat cochlear nuclei. Neurol. Res.2005;27;436-440.

Kasten MR et al. Differential regulation of action potential firing in adult murine thalamocortical neurons by Kv3.2, Kv1, and SK potassium and N-type calcium channels. J. Physiol. 2007;584(2):565-582.

Kaczmarek L et al. Regulation of the timing of MNTB neurons by short-term and long-term modulation of potassium channels. Hearing Res. 2005;206;133-145.

Lau D *et al.* Impaired Fast-Spiking, Suppressed Cortical Inhibition, and

Increased Susceptibility to Seizures in Mice Lacking Kv3.2 $K^+$ Channel Proteins. J. Neurosci. 2000 Dec;20(24):9071-9085.

Li W et al. Localization of Two High-Threshol Potassium Channel Subunits in the Rat Central Auditory System. J. Comp. Neuro. 2001 May;437:196-218.

Lu R et al. Slack channels expressed in sensory neurons control neuropathic pain in mice. J. Neurosci. 2015 Jan;35(3): 1125-35.

Markram H et al. Interneurons of the neocortical inhibitory system. Nat. Rev. Neurosci. 2004 Oct;5:793-807.

Martina M et al. Functional and Molecular Differences between Voltage-Gated K+ Channels of Fast-Spiking Interneurons and Pyramidal Neurons of Rat Hippocampus. J. Neurosci. 1998 Oct;18(20):8111-8125.

McCarberg BH et al. The impact of pain on quality of life and the unmet needs of pain management: results from pain sufferers and physicians participating in an Internet survey. Am. J. Ther. 2008 Jul-Aug;15(4):312-20.

McDonald AJ, Mascagni F. Differential expression of Kv3.1b and Kv3.2 potassium channel subunits in interneurons of the basolateral amygdala. Neuroscience 2006;138:537-547.

McMahon A et al. Allele-dependent changes of olivocerebellar circuit properties in the absence of the voltage-gated potassium channels Kv3.1 and Kv3.3. Eur. J. Neurosci. 2004 Mar;19:3317-3327.

Mitchell I et al. Aryl Pyrazoles as Potent Inhibitors of Arginine Methyltransferases: Identification of the First PRMT6 Tool Compound. ACS Med. Chem. Lett. 2015;6(6);655-659.

Muona M, et al. A recurrent de novo mutation in KCNC1 causes progressive myoclonus epilepsy. Nat Genet. 2015 Jan;47(1):39-46.

Muqeem T et al. Regulation of Nociceptive Glutamatergic Signaling by Presynaptic Kv3.4 Channels in the Rat Spinal Dorsal Horn J Neurosci. 2018 Apr 11;38(15):3729-3740

Olsen T et al. Kv3 K+ currents contribute to spike-timing in dorsal cochlear nucleus principal cells. Neuropharmacology 2018 May 1;133:319-333

Pilati N et al.. Acoustic over-exposure triggers burst firing in dorsal cochlear nucleus fusiform cells. Hearing Research 2012;283;98-106.

Puente N et al. Precise localization of the voltage-gated potassium channel subunits Kv3.1b and Kv3.3 revealed in the molecular layer of the rat cerebellar cortex by a pre-embedding immunogold method. Histochem. Cell. Biol. 2010 Sep;134:403-409.

Reynolds GP et al. Calcium Binding Protein Markers of GABA Deficits in Schizophrenia - Post Mortem Studies and Animal Models. Neurotox. Res. 2004 Feb;6(1):57-62.

Ritter DM et al. Modulation of Kv3.4 channel N-type inactivation by protein kinase C shapes the action potential in dorsal root ganglion neurons. J. Physiol. 2012 Jan;590(Pt 1):145-61.

Ritter DM et al. Dysregulation of Kv3.4 channels in dorsal root ganglia following spinal cord injury. J. Neurosci. 2015 Jan;35(3):1260-73.

Roberts L et al. Ringing Ears: The Neuroscience of Tinnitus. J. Neurosci. 2010:30(45);14972-14979.

Rudy B, McBain CJ. Kv3 channels: voltage-gated K+ channels designed for high-frequency repetitive firing. TRENDS in Neurosci. 2001 Sep;24(9):517-526.

Sacco T et al. Properties and expression of Kv3 channels in cerebellar Purkinje cells. Mol. Cell. Neurosci. 2006 Jul;33:170-179.

Schulz P, Steimer T. Neurobiology of Circadian Systems. CNS Drugs 2009;23(Suppl. 2):3-13.

Song P et al. Acoustic environment determines phosphorylation state of the Kv3.1 potassium channel in auditory neurons Nat. Neurosci. 2005 Oct;8(10): 1335-1342.

Spencer KM et al. Neural synchrony indexes disordered perception and cognition in schizophrenia. PNAS 2004 Dec;101(49):17288-17293.

Sun S et al. Inhibitors of voltage-gated sodium channel Nav1.7: patent applications since 2010. Pharm. Pat. Anal. 2014 Sep;3(5):509-21.

U.S. Department of Health and Human Services, Food and Drug Administration. Draft Guidance for Industry Analgesic Indications: Developing Drug and Biological Products: http://www.fda.gov/downloads/drugs/guidancecompliancerequlatoryinformation/guidances/ucm3 84691.pdf 2014 Feb.
von Hehn C et al. Loss of Kv3.1 Tonotopicity and Alterations in cAMP Response Element-Binding Protein Signaling in Central Auditory Neurons of Hearing Impaired Mice. J. Neurosci. 2004;24: 1936-1940.
Weiser M et al. Differential Expression of Shaw-related K+ Channels in the Rat Central Nervous System. J. Neurosci. 1994 Mar;14(3):949-972.
Wickenden AD, McNaughton-Smith G. Kv7 channels as targets for the treatment of pain. Curr. Pharm. Des. 2009;15(15):1773-98.
Woolf CJ. What is this thing called pain? J. Clin. Invest. 2010 Nov;120(11):3742-4.
Woolf CJ. Central sensitization: implications for the diagnosis and treatment of pain. Pain 2011 Mar;152(3 Suppl):S2-15.
Yanagi M et al. Kv3.1-containing K(+) channels are reduced in untreated schizophrenia and normalized with antipsychotic drugs. Mol Psychiatry. 2014. 19(5):573-9.
Yeung SYM et al. Modulation of Kv3 Subfamily Potassium Currents by the Sea Anemone Toxin BDS: Significance for CNS and Biophysical Studies. J. Neurosci. 2005 Mar;25(38):8735-8745.
Zamponi GW et al. The Physiology, Pathology, and Pharmacology of Voltage-Gated Calcium Channels and Their Future Therapeutic Potential Pharmacol Rev. 2015 Oct;67(4):821-70.

## Claims

**1.** A compound of formula (I):

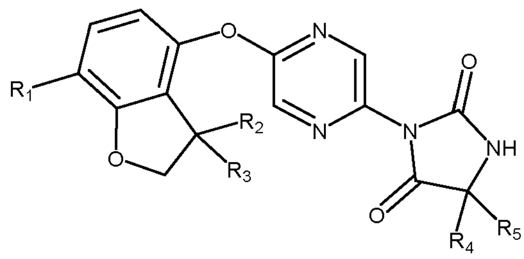
(I)

wherein:

$R_1$ is H or methyl;
$R_2$ and $R_3$ are both methyl, or $R_2$ and $R_3$, together with the carbon atom to which they are attached, are a spirocyclopropyl ring;
$R_4$ is methyl or ethyl;
$R_5$ is H or methyl;
or $R_4$ and $R_5$, together with the carbon atom to which they are attached, form a $C_3$-$C_4$ spiro carbocyclyl;
or a salt and/or solvate and/or derivative thereof of formula (Z),
wherein said derivative of formula (Z) is functionalised at the secondary nitrogen of the hydantoin with group L as illustrated below:

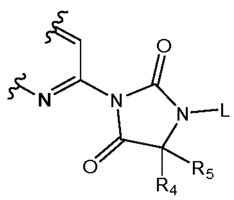
(Z),

and wherein L is selected from:

a) $-PO(OH)O^{-} \bullet M^{+}$, wherein $M^{+}$ is a pharmaceutically acceptable monovalent counterion,
b) $-PO(O^{-})_2 \bullet 2M^{+}$,
c) $-PO(O^{-})_2 \bullet D^{2+}$, wherein $D^{2+}$ is a pharmaceutically acceptable divalent counterion,
d) $-CH(R^x)-PO(OH)O^{-} \bullet M^{+}$, wherein $R^x$ is hydrogen or $C_{1-3}$ alkyl,

e) $-CH(R^x)-PO(O^-)_2 \cdot 2M^+$,
f) $-CH(R^x)-PO(O^-)_2 \cdot D^{2+}$,
g) $-SO_3^- \cdot M^+$,
h) $-CH(R^X)-SO_3^- \cdot M^+$, and
i) $-CO-CH_2CH_2-CO_2 \cdot M^+$.

2. The salt and/or solvate and/or derivative thereof of formula (Z) according to claim 1, wherein the salt and/or solvate and/or derivative of formula (Z) is a pharmaceutically acceptable salt and/or solvate and/or derivative of formula (Z) thereof.

3. The pharmaceutically acceptable salt according to claim 2.

4. The pharmaceutically acceptable solvate according to claim 2.

5. The compound according to claim 1.

6. The compound, pharmaceutically acceptable salt and/or solvate and/or derivative thereof according to any one of claims 2 to 5 wherein $R_1$ is H.

7. The compound, pharmaceutically acceptable salt and/or solvate and/or derivative thereof according to any one of claims 2 to 5 wherein $R_1$ is methyl.

8. The compound, pharmaceutically acceptable salt and/or solvate and/or derivative thereof according to any one of claims 2 to 7, wherein $R_2$ and $R_3$ are a spiro cyclopropyl.

9. The compound, pharmaceutically acceptable salt and/or solvate and/or derivative thereof according to any one of claims 2 to 8, wherein $R_4$ is ethyl and $R_5$ is H.

10. The compound, pharmaceutically acceptable salt and/or solvate thereof according to any one of claims 2 to 5 selected from the group consisting of:

5,5-dimethyl-3-[5-(7-methylspiro[2H-benzofuran-3,1’-cyclopropane]-4-yl)oxypyrazin-2-yl]imidazolidine-2,4-dione;
3-[5-[(3,3-dimethyl-2H-benzofuran-4-yl)oxy]pyrazin-2-yl]-5,5-dimethyl-imidazolidine-2,4-dione;
(5R)-5-ethyl-5-methyl-3-(5-spiro[2H-benzofuran-3,1’-cyclopropane]-4-yloxypyrazin-2-yl)imidazolidine-2,4-dione;
5,5-dimethyl-3-(5-spiro[2H-benzofuran-3,1’-cyclopropane]-4-yloxypyrazin-2-yl)imidazolidine-2,4-dione;
(5R)-5-ethyl-5-methyl-3-[5-(7-methylspiro[2H-benzofuran-3,1’-cyclopropane]-4-yl)oxypyrazin-2-yl]imidazolidine-2,4-dione;
(5R)-3-[5-[(3,3-dimethyl-2H-benzofuran-4-yl)oxy]pyrazin-2-yl]-5-ethyl-5-methylimidazolidine-2,4-dione;
5,5-dimethyl-3-[5-[(3,3,7-trimethyl-2H-benzofuran-4-yl)oxy]pyrazin-2-yl]imidazolidine-2,4-dione;
(5R)-5-ethyl-5-methyl-3-[5-[(3,3,7-trimethyl-2H-benzofuran-4-yl)oxy]pyrazin-2-yl]imidazolidine-2,4-dione;
(5R)-5-ethyl-3-[5-(7-methylspiro[2H-benzofuran-3,1’-cyclopropane]-4-yl)oxypyrazin-2-yl]imidazolidine-2,4-dione;
(5R)-5-ethyl-3-(5-spiro[2H-benzofuran-3,1’-cyclopropane]-4-yloxypyrazin-2-yl)imidazolidine-2,4-dione;
(5R)-3-[5-[(3,3-dimethyl-2H-benzofuran-4-yl)oxy]pyrazin-2-yl]-5-ethyl-imidazolidine-2,4-dione;
(5R)-5-ethyl-3-[5-[(3,3,7-trimethyl-2H-benzofuran-4-yl)oxy]pyrazin-2-yl]imidazolidine-2,4-dione;
7-[5-(7-methylspiro[2H-benzofuran-3,1’-cyclopropane]-4-yl)oxypyrazin-2-yl]-5,7-diazaspiro[3.4]octane-6,8-dione;
6-[5-(7-methylspiro[2H-benzofuran-3,l’-cyclopropane]-4-yl)oxypyrazin-2-yl]-4,6-diazaspiro[2.4]heptane-5,7-dione; and
(5S)-5-ethyl-3-[5-(7-methylspiro[2H-benzofuran-3,1’-cyclopropane]-4-yl)oxypyrazin-2-yl]imidazolidine-2,4-dione

or a pharmaceutically acceptable salt and/or solvate thereof.

11. The compound according to claim 1 which is:
(5R)-5-ethyl-3-[5-(7-methylspiro[2H-benzofuran-3,1’-cyclopropane]-4-yl)oxypyrazin-2-yl]imidazolidine-2,4-dione

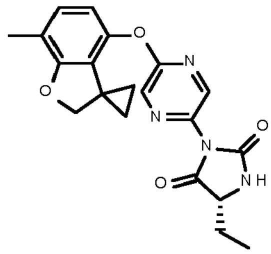

.

**12.** The pharmaceutically acceptable salt according to claim 2 which is a pharmaceutically acceptable salt of: (5R)-5-ethyl-3-[5-(7-methylspiro[2H-benzofuran-3,1'-cyclopropane]-4-yl)oxypyrazin-2-yl]imidazolidine-2,4-dione

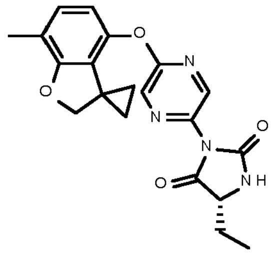

.

**13.** The compound according to claim 1 which is: (5R)-5-ethyl-3-(5-spiro[2H-benzofuran-3,1'-cyclopropane]-4-yloxypyrazin-2-yl)imidazolidine-2,4-dione

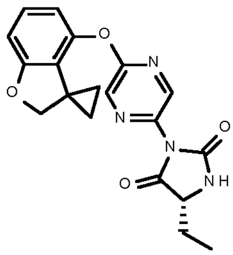

.

**14.** The pharmaceutically acceptable salt according to claim 2 which is a pharmaceutically acceptable salt of: (5R)-5-ethyl-3-(5-spiro[2H-benzofuran-3,1'-cyclopropane]-4-yloxypyrazin-2-yl)imidazolidine-2,4-dione

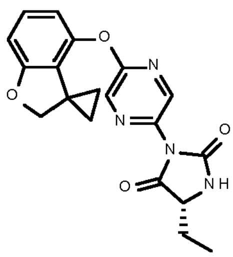

.

**15.** The compound, pharmaceutically acceptable salt and/or solvate and/or derivative thereof according to any one of claims 2 to 14 for use as a medicament.

**16.** The compound, pharmaceutically acceptable salt and/or solvate and/or derivative thereof according to any one of claims 2 to 14 for use in the prophylaxis or treatment of a disease or disorder selected from the group consisting of hearing disorders, schizophrenia, depression and mood disorders, bipolar disorder, substance abuse disorders, anxiety disorders, sleep disorders, hyperacusis and disturbances of loudness perception, Ménière's disease, disorders of balance, and disorders of the inner ear, impulse control disorder, personality disorders, attention-deficit/hyperactivity disorder, autism spectrum disorders, eating disorders, cognition impairment, ataxia, pain such as neuropathic pain, inflammatory pain and miscellaneous pain, Lewy body dementia and Parkinson's disease.

**17.** A pharmaceutical composition comprising a compound, pharmaceutically acceptable salt and/or solvate thereof according to any one of claims 2 to 14 and a pharmaceutically acceptable carrier or excipient.

**18.** A compound of formula (II),

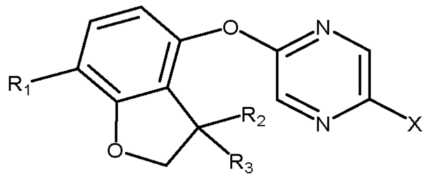

(II),

wherein $R_1$, $R_2$ and $R_3$ are as defined in claim 1, X is halo, such as Br; or
a compound of formula (XVI):

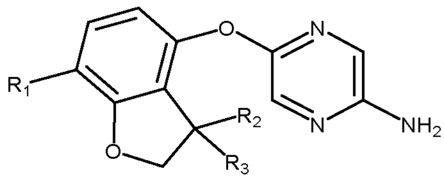

(XVI),

wherein $R_1$, $R_2$ and $R_3$ are as defined in claim 1, X is halo, such as Br; or
a compound of formula (IV):

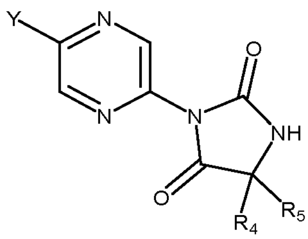

(IV)

wherein $R_4$ and $R_5$ are as defined in claim 1, Y is halo, such as Cl.

**Patentansprüche**

**1.** Verbindung der Formel (I):

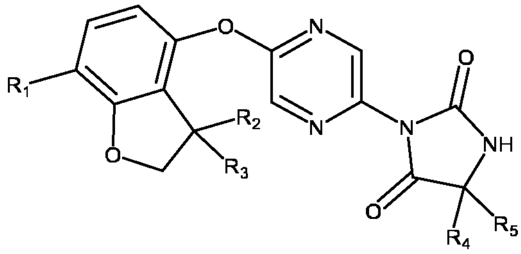

(I)

wobei:

$R_1$ H oder Methyl ist;

$R_2$ und $R_3$ beide Methyl sind oder $R_2$ und $R_3$ zusammen mit dem Kohlenstoffatom, an das sie gebunden sind, ein Spirocyclopropylring sind;
$R_4$ Methyl oder Ethyl ist;
$R_5$ H oder Methyl ist;
oder $R_4$ und $R_5$ zusammen mit dem Kohlenstoffatom, an das sie gebunden sind, ein $C_3$-$C_4$ -Spirocarbocyclyl bilden;
oder Salz und/oder Solvat und/oder Derivat davon der Formel (Z), wobei das Derivat der Formel (Z) an dem sekundären Stickstoff des Hydantoins mit der Gruppe L wie nachstehend veranschaulicht funktionalisiert ist:

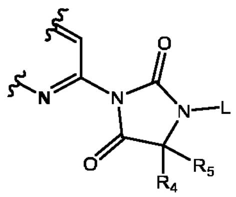
(Z),

und wobei L aus Folgendem ausgewählt ist:

a) $-PO(OH)O^{-}\bullet M^{+}$, wobei $M^{+}$ ein pharmazeutisch verträgliches monovalentes Gegenion ist,
b) $-PO(O^{-})_2\bullet 2M^{+}$,
c) $-PO(O^{-})_2\bullet D^{2+}$, wobei $D^{2+}$ ein pharmazeutisch verträgliches zweiwertiges Gegenion ist,
d) $-CH(R^{X})-PO(OH)O^{-}\bullet M^{+}$, wobei $R^{X}$ Wasserstoff oder $C_{1-3}$-Alkyl ist,
e) $-CH(R^{X})-PO(O^{-})_2\bullet 2M^{+}$,
f) $-CH(R^{X})-PO(O^{-})_2\bullet D^{2+}$,
g) $-SO_3^{-}\bullet M^{+}$,
h) $-CH(R^{X})-SO_3^{-}\bullet M^{+}$ und
i) $-CO-CH_2CH_2-CO_2\bullet M^{+}$.

**2.** Salz und/oder Solvat und/oder Derivat davon der Formel (Z) nach Anspruch 1, wobei das Salz und/oder Solvat und/oder Derivat der Formel (Z) ein pharmazeutisch verträgliches Salz und/oder Solvat und/oder Derivat der Formel (Z) davon ist.

**3.** Pharmazeutisch verträgliche Salz nach Anspruch 2.

**4.** Pharmazeutisch verträgliche Solvat nach Anspruch 2.

**5.** Verbindung nach Anspruch 1.

**6.** Verbindung, pharmazeutisch verträgliches Salz und/oder Solvat und/oder Derivat davon nach einem der Ansprüche 2 bis 5, wobei $R_1$ H ist.

**7.** Verbindung, pharmazeutisch verträgliches Salz und/oder Solvat und/oder Derivat davon nach einem der Ansprüche 2 bis 5, wobei $R_1$ Methyl ist.

**8.** Verbindung, pharmazeutisch verträgliches Salz und/oder Solvat und/oder Derivat davon nach einem der Ansprüche 2 bis 7, wobei $R_2$ und $R_3$ ein Spirocyclopropyl sind.

**9.** Verbindung, pharmazeutisch verträgliches Salz und/oder Solvat und/oder Derivat davon nach einem der Ansprüche 2 bis 8, wobei $R_4$ Ethyl ist und $R_5$ H ist.

**10.** Verbindung, pharmazeutisch verträgliches Salz und/oder Solvat davon nach einem der Ansprüche 2 bis 5, ausgewählt aus der Gruppe bestehend aus:

5,5-Dimethyl-3-[5-(7-methylspiro[2H-benzofuran-3,1'-cyclopropan]-4-yl)oxypyrazin-2-yl]imidazolidin-2,4-dion;
3-[5-[(3,3-Dimethyl-2H-benzofuran-4-yl)oxy]pyrazin-2-yl]-5,5-dimethyl-imidazolidin-2,4-dion;
(5R)-5-Ethyl-5-methyl-3-(5-spiro[2H-benzofuran-3,1'-cyclopropan]-4-yloxypyrazin-2-yl)imidazolidin-2,4-dion;
5,5-Dimethyl-3-(5-spiro[2H-benzofuran-3,1'-cyclopropan]-4-yloxypyrazin-2-yl)imidazolidin-2,4-dion;

(5R)-5-Ethyl-5-methyl-3-[5-(7-methylspiro[2H-benzofuran-3,1'-cyclopropan]-4-yl)oxypyrazin-2-yl]imidazolidin-2,4-dion;
(5R)-3-[5-[(3,3-Dimethyl-2H-benzofuran-4-yl)oxy]pyrazin-2-yl]-5-ethyl-5-methylimidazolidin-2,4-dion;
5,5-Dimethyl-3-[5-[(3,3,7-trimethyl-2H-benzofuran-4-yl)oxy]pyrazin-2-yl]imidazolidin-2,4-dion;
(5R)-5-Ethyl-5-methyl-3-[5-[(3,3,7-trimethyl-2H-benzofuran-4-yl)oxy]pyrazin-2-yl]imidazolidin-2,4-dion;
(5R)-5-Ethyl-3-[5-(7-methylspiro[2H-benzofuran-3,1'-cyclopropan]-4-yl)oxypyrazin-2-yl)imidazolidin-2,4-dion;
(5R)-5-Ethyl-3-(5-spiro[2H-benzofuran-3,1'-cyclopropan]-4-yloxypyrazin-2-yl)imidazolidin-2,4-dion;
(5R)-3-[5-[(3,3-Dimethyl-2H-benzofuran-4-yl)oxy]pyrazin-2-yl]-5-ethylimidazolidin-2,4-dion;
(5R)-5-Ethyl-3-[5-[(3,3,7-trimethyl-2H-benzofuran-4-yl)oxy]pyrazin-2-yl]imidazolidin-2,4-dion;
7-[5-(7-Methylspiro[2H-benzofuran-3,1'-cyclopropan]-4-yl)oxypyrazin-2-yl]-5,7-diazaspiro[3.4]octan-6,8-dion;
6-[5-(7-Methylspiro[2H-benzofuran-3,1'-cyclopropan]-4-yl)oxypyrazin-2-yl]-4,6-diazaspiro[2.4]heptan-5,7-dion; und
(5S)-5-Ethyl-3-[5-(7-methylspiro[2H-benzofuran-3,1'-cyclopropan]-4-yl)oxypyrazin-2-yl]imidazolidin-2,4-dion

oder ein pharmazeutisch verträgliches Salz und/oder Solvat davon.

**11.** Verbindung nach Anspruch 1, die Folgendes ist:
(5R)-5-Ethyl-3-[5-(7-methylspiro[2H-benzofuran-3,1'-cyclopropan]-4-yl)oxypyrazin-2-yl]imidazolidin-2,4-dion

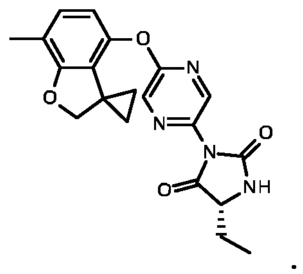

**12.** Pharmazeutisch verträgliches Salz nach Anspruch 2, das ein pharmazeutisch verträgliches Salz von Folgendem ist:
(5R)-5-Ethyl-3-[5-(7-methylspiro[2H-benzofuran-3,1'-cyclopropan]-4-yl)oxypyrazin-2-yl]imidazolidin-2,4-dion

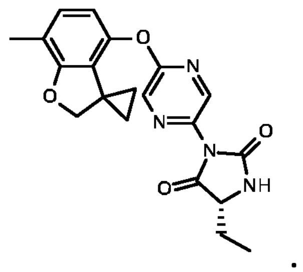

**13.** Verbindung nach Anspruch 1, die Folgendes ist:
(5R)-5-Ethyl-3-(5-spiro[2H-benzofuran-3,1'-cyclopropan]-4-yloxypyrazin-2-yl)imidazolidin-2,4-dion

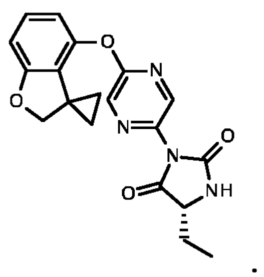

**14.** Pharmazeutisch verträgliches Salz nach Anspruch 2, das ein pharmazeutisch verträgliches Salz von Folgendem ist:
(5R)-5-Ethyl-3-(5-spiro[2H-benzofuran-3,1'-cyclopropan]-4-yloxypyrazin-2-yl)imidazolidin-2,4-dion

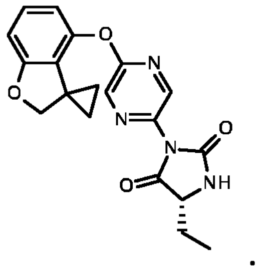

15. Verbindung, pharmazeutisch verträgliches Salz und/oder Solvat davon nach einem der Ansprüche 2 bis 14 zur Verwendung als Medikament.

16. Verbindung, pharmazeutisch verträgliche Salz und/oder Solvat und/oder Derivat davon nach einem der Ansprüche 2 bis 14 zur Verwendung bei der Prophylaxe oder Behandlung einer Krankheit oder Störung, ausgewählt aus der Gruppe bestehend aus Hörstörungen, Schizophrenie, Depression und Stimmungsstörungen, bipolarer Störung, Substanzmissbrauchsstörungen, Angststörungen, Schlafstörungen, Hyperakusis und Störungen der Lautstärkewahrnehmung, Morbus Menière, Gleichgewichtsstörungen und Störungen des Innenohrs, Impulskontrollstörungen, Persönlichkeitsstörungen, Aufmerksamkeitsdefizit-/Hyperaktivitätsstörung, Störungen aus dem autistischen Spektrum, Essstörungen, kognitiven Beeinträchtigungen, Ataxie, Schmerzen, wie etwa neuropathischen Schmerzen, Entzündungsschmerzen und sonstigen Schmerzen, Lewy-Body-Demenz und Parkinson-Krankheit.

17. Pharmazeutische Zusammensetzung, umfassend eine Verbindung, ein pharmazeutisch verträgliches Salz und/oder ein Solvat davon nach einem der Ansprüche 2 bis 14 und einen pharmazeutisch verträglichen Träger oder Hilfsstoff.

18. Verbindung der Formel (II),

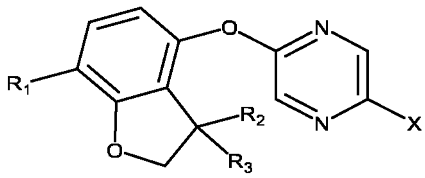
(II),

wobei $R_1$, $R_2$ und $R_3$ wie in Anspruch 1 definiert sind, X ein Halogen ist, wie etwa Br; oder Verbindung der Formel (XVI):

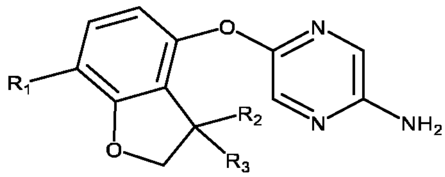
(XVI),

wobei $R_1$, $R_2$ und $R_3$ wie in Anspruch 1 definiert sind, X ein Halogen ist, wie etwa Br; oder Verbindung der Formel (IV):

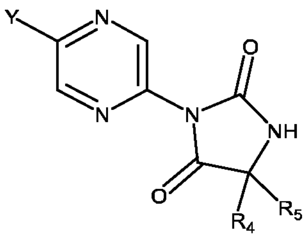
(IV)

wobei $R_4$ und $R_5$ wie in Anspruch 1 definiert sind, Y ein Halogen ist, wie etwa Cl.

## Revendications

1. Composé de formule (I) :

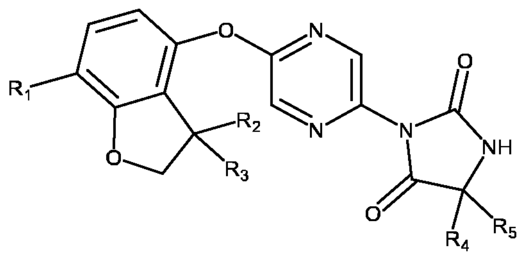

(I)

dans lequel :

$R_1$ est H ou méthyle ;
$R_2$ et $R_3$ sont tous deux méthyle, ou $R_2$ et $R_3$, ensemble avec l'atome de carbone auquel ils sont attachés, sont un noyau spirocyclopropyle ;
$R_4$ est méthyle ou éthyle ;
$R_5$ est H ou méthyle ;
ou $R_4$ et $R_5$, ensemble avec l'atome de carbone auquel ils sont attachés, forment un spirocarbocyclyle en $C_3$-$C_4$ ;
ou un sel et/ou solvate et/ou dérivé de celui-ci de formule (Z),
dans lequel ledit dérivé de formule (Z) est fonctionnalisé au niveau de l'azote secondaire de l'hydantoïne avec un groupe L comme illustré ci-dessous :

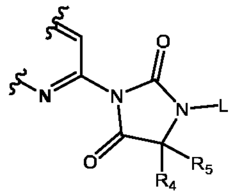

(Z),

et dans lequel L est choisi parmi :

a) $-PO(OH)O^- \cdot M^+$, dans lequel $M^+$ est un contre-ion monovalent pharmaceutiquement acceptable,
b) $-PO(O^-)_2 \cdot 2M^+$,
c) $-PO(O^-)_2 \cdot D^{2+}$, dans lequel $D^{2+}$ est un contre-ion divalent pharmaceutiquement acceptable,
d) $-CH(R^X)-PO(OH)O^- \cdot M^+$, dans lequel $R^X$ est hydrogène ou alkyle en $C_{1-3}$,
e) $-CH(R^X)-PO(O^-)_2 \cdot 2M^+$,
f) $-CH(R^X)-PO(O^-)_2 \cdot D^{2+}$,
g) $-SO_3^- \cdot M^+$,
h) $-CH(R^X)-SO_3^- \cdot M^+$, et
i) $-CO-CH_2CH_2-CO_2 \cdot M^+$.

2. Sel et/ou solvate et/ou dérivé de celui-ci de formule (Z) selon la revendication 1, dans lequel le sel et/ou solvate et/ou dérivé de formule (Z) est un sel et/ou solvate et/ou dérivé pharmaceutiquement acceptable de formule (Z) de celui-ci.

3. Sel pharmaceutiquement acceptable selon la revendication 2.

4. Solvate pharmaceutiquement acceptable selon la revendication 2.

5. Composé selon la revendication 1.

6. Composé, sel et/ou solvate et/ou dérivé pharmaceutiquement acceptable de celui-ci selon l'une quelconque des revendications 2 à 5, dans lequel $R_1$ est H.

**7.** Composé, sel et/ou solvate et/ou dérivé pharmaceutiquement acceptable de celui-ci selon l'une quelconque des revendications 2 à 5, dans lequel $R_1$ est méthyle.

**8.** Composé, sel et/ou solvate et/ou dérivé pharmaceutiquement acceptable de celui-ci selon l'une quelconque des revendications 2 à 7, dans lequel $R_2$ et $R_3$ sont un spirocyclopropyle.

**9.** Composé, sel et/ou solvate et/ou dérivé pharmaceutiquement acceptable de celui-ci selon l'une quelconque des revendications 2 à 8, dans lequel $R_4$ est éthyle et $R_5$ est H.

**10.** Composé, sel et/ou solvate pharmaceutiquement acceptable de celui-ci selon l'une quelconque des revendications 2 à 5 choisi dans le groupe constitué de :

5,5-diméthyl-3-[5-(7-méthylspiro[2H-benzofuran-3,1'-cyclopropan]-4-yl)oxypyrazin-2-yl]imidazolidin-2,4-dione ;
3-[5-[(3,3-diméthyl-2H-benzofuran-4-yl)oxy]pyrazin-2-yl]-5,5-diméthyl-imidazolidin-2,4-dione ;
(5R)-5-éthyl-5-méthyl-3-(5-spiro[2H-benzofuran-3,1'-cyclopropan]-4-yloxypyrazin-2-yl)imidazolidin-2,4-dione ;
5,5-diméthyl-3-(5-spiro[2H-benzofuran-3,1'-cyclopropan]-4-yloxypyrazin-2-yl)imidazolidin-2,4-dione ;
(5R)-5-éthyl-5-méthyl-3-[5-(7-méthylspiro[2H-benzofuran-3,1'-cyclopropan]-4-yl)oxypyrazin-2-yl]imidazolidin-2,4-dione ;
(5R)-3-[5-[(3,3-diméthyl-2H-benzofuran-4-yl)oxy]pyrazin-2-yl]-5-éthyl-5-méthyl-imidazolidin-2,4-dione ;
5,5-diméthyl-3-[5-[(3,3,7-triméthyl-2H-benzofuran-4-yl)oxy]pyrazin-2-yl]imidazolidin-2,4-dione ;
(5R)-5-éthyl-5-méthyl-3-[5-[(3,3,7-triméthyl-2H-benzofuran-4-yl)oxy]pyrazin-2-yl]imidazolidin-2,4-dione ;
(5R)-5-éthyl-3-[5-(7-méthylspiro[2H-benzofuran-3,1'-cyclopropan]-4-yl)oxypyrazin-2-yl]imidazolidin-2,4-dione ;
(5R)-5-éthyl-3-(5-spiro[2H-benzofuran-3,1'-cyclopropan]-4-yloxypyrazin-2-yl)imidazolidin-2,4-dione ;
(5R)-3-[5-[(3,3-diméthyl-2H-benzofuran-4-yl)oxy]pyrazin-2-yl]-5-éthyl-imidazolidin-2,4-dione ;
(5R)-5-éthyl-3-[5-[(3,3,7-triméthyl-2H-benzofuran-4-yl)oxy]pyrazin-2-yl]imidazolidin-2,4-dione ;
7-[5-(7-méthylspiro[2H-benzofuran-3,1'-cyclopropan]-4-yl)oxypyrazin-2-yl]-5,7-diazaspiro[3.4]octan-6,8-dione ;
6-[5-(7-méthylspiro[2H-benzofuran-3,1'-cyclopropan]-4-yl)oxypyrazin-2-yl]-4,6-diazaspiro[2.4]heptan-5,7-dione ; et
(5S)-5-éthyl-3-[5-(7-méthylspiro[2H-benzofuran-3,1'-cyclopropan]-4-yl)oxypyrazin-2-yl]imidazolidin-2,4-dione

ou un sel et/ou solvate pharmaceutiquement acceptable de ceuxci.

**11.** Composé selon la revendication 1 qui est :
La (5R)-5-éthyl-3-[5-(7-méthylspiro[2H-benzofuran-3,1'-cyclopropan]-4-yl)oxypyrazin-2-yl]imidazolidin-2,4-dione

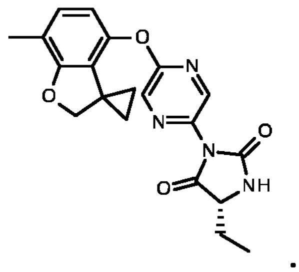

**12.** Sel pharmaceutiquement acceptable selon la revendication 2 qui est un sel pharmaceutiquement acceptable de : la (5R)-5-éthyl-3-[5-(7-méthylspiro[2H-benzofuran-3,1'-cyclopropan]-4-yl)oxypyrazin-2-yl]imidazolidin-2,4-dione

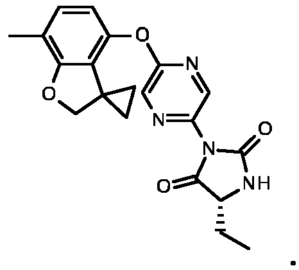

13. Composé selon la revendication 1 qui est :
La (5R)-5-éthyl-3-(5-spiro[2H-benzofuran-3,1'-cyclopropan]-4-yloxypyrazin-2-yl)imidazolidin-2,4-dione

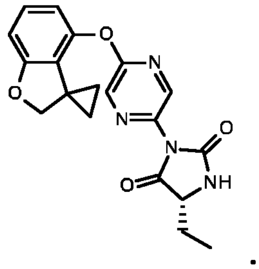

14. Sel pharmaceutiquement acceptable selon la revendication 2 qui est un sel pharmaceutiquement acceptable de :
la (5R)-5-éthyl-3-(5-spiro[2H-benzofuran-3,1'-cyclopropan]-4-yloxypyrazin-2-yl)imidazolidin-2,4-dione

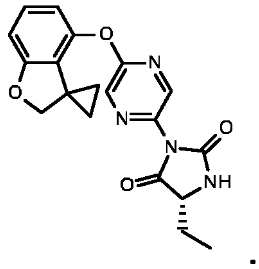

15. Composé, sel et/ou solvate et/ou dérivé pharmaceutiquement acceptable de celui-ci selon l'une quelconque des revendications 2 à 14 destiné à être utilisé comme médicament.

16. Composé, sel et/ou solvate et/ou dérivé pharmaceutiquement acceptable de celui-ci selon l'une quelconque des revendications 2 à 14 destiné à être utilisé dans la prophylaxie ou le traitement d'une maladie ou d'un trouble choisi dans le groupe constitué des troubles de l'audition, de la schizophrénie, des troubles de la dépression et de l'humeur, des troubles bipolaires, des troubles liés à la toxicomanie, des troubles anxieux, des troubles du sommeil, de l'hyperacousie et des troubles de la perception sonore, de la maladie de Ménière, des troubles de l'équilibre et des troubles de l'oreille interne, du trouble du contrôle des impulsions, des troubles de la personnalité, du trouble déficitaire de l'attention/hyperactivité, des troubles du spectre autistique, des troubles de l'alimentation, de la déficience cognitive, de l'ataxie, des douleurs telles que les douleurs neuropathiques, les douleurs inflammatoires et les douleurs diverses, de la démence à corps de Lewy et de la maladie de Parkinson.

17. Composition pharmaceutique comprenant un composé, un sel et/ou solvate pharmaceutiquement acceptable de celui-ci selon l'une quelconque des revendications 2 à 14 et un support ou excipient pharmaceutiquement acceptable.

18. Composé de formule (II),

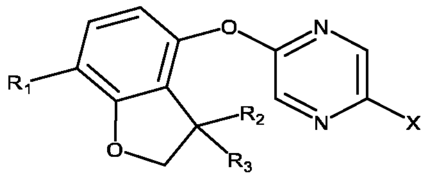

(II)

dans lequel $R_1$, $R_2$ et $R_3$ sont tels que définis dans la revendication 1, X est halogène, tel que Br ; ou un composé de formule (XVI) :

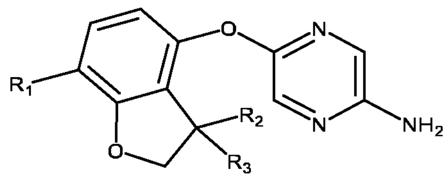

(XVI),

dans lequel $R_1$, $R_2$ et $R_3$ sont tels que définis dans la revendication 1, X est halogène, tel que Br ; ou un composé de formule (IV) :

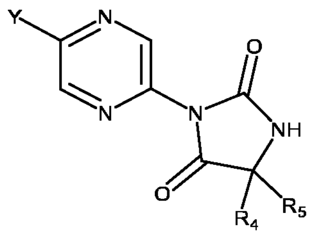

(IV)

dans laquelle $R_4$ et $R_5$ sont tels que définis dans la revendication 1, Y est halogéno, tel que Cl.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017098254 A
- WO 2011069951 A
- WO 2012076877 A
- WO 2012168710 A
- WO 2013175215 A
- WO 2013083994 A
- WO 2013182850 A
- WO 2017103604 A
- WO 2018020263 A
- WO 2018109484 A
- WO 2013182851 A
- WO 2013175211 A


### Non-patent literature cited in the description


- **BERGE ; BIGHLEY ; MONKHOUSE.** *J.Pharm.Sci.,* 1977, vol. 66, 1-19
- Diagnostic and Statistical Manual of Mental Disorders. American Psychiatric Association
- **ANDERSON LA et al.** Increased spontaneous firing rates in auditory midbrain following noise exposure are specifically abolished by a Kv3 channel modulator. *Hear Res.,* August 2018, vol. 365, 77-89
- **ARONIADOU-ANDERJASKA V et al.** Mechanisms regulating GABAergic inhibitory transmission in the basolateral amygdala: implications for epilepsy and anxiety disorders. *Amino Acids,* August 2007, vol. 32, 305-315
- **BARANAUSKAS G ; NISTRI A.** Sensitization of pain pathways in the spinal cord: cellular mechanisms. *Prog. Neurobiol.,* February 1998, vol. 54 (3), 349-65
- **BARON R et al.** Peripheral input and its importance for central sensitization. *Ann. Neurol.,* November 2013, vol. 74 (5), 630-6
- **BEN-ARI Y.** Seizure Beget Seizure: The Quest for GABA as a Key Player. *Crit. Rev. Neurobiol.,* 2006, vol. 18 (1-2), 135-144
- **BENES FM et al.** Circuitry-based gene expression profiles in GABA cells of the trisynaptic pathway in schizophrenics versus bipolars. *PNAS,* December 2008, vol. 105 (52), 20935-20940
- **BENNETT DL ; WOODS CG.** Painful and painless channelopathies. *Lancet Neurol.,* June 2014, vol. 13 (6), 587-99
- **BERGE S et al.** Pharmaceutical Salts. *J. Pharm. Sci.,* 1977, vol. 66, 1-19
- **BRAMBILLA P et al.** GABAergic dysfunction in mood disorders. *Mol. Psych.,* April 2003, vol. 8, 721-737
- **BROOKE RE et al.** Spinal cord interneurones labelled transneuronally from the adrenal gland by a GFP-herpes virus construct contain the potassium channel subunit Kv3.1b. *Auton. Neurosci.,* vol. 98 (1-2), 45-50
- **BROOKE RE et al.** Association of potassium channel Kv3.4 subunits with pre- and post-synaptic structures in brainstem and spinal cord. *Neuroscience,* 2004, vol. 126 (4), 1001-10
- **BROOKE RE et al.** Immunohistochemical localisation of the voltage gated potassium ion channel subunit Kv3.3 in the rat medulla oblongata and thoracic spinal cord. *Brain Res.,* vol. 1070 (1), 101-15
- **CERVERO F.** Spinal cord hyperexcitability and its role in pain and hyperalgesia. *Exp. Brain Res.,* vol. 196 (1), 129-37
- **CHAMBERS AR et al.** Pharmacological modulation of Kv3.1 mitigates auditory midbrain temporal processing deficits following auditory nerve damage. *Sci Rep.,* December 2017, vol. 7 (1), 17496
- **CHANG SY et al.** Distribution of Kv3.3 Potassium Channel Subunits in Distinct Neuronal Populations of Mouse Brain. *J. Comp. Neuro.,* February 2007, vol. 502, 953-972
- **CHIEN LY et al.** Reduced expression of A-type potassium channels in primary sensory neurons induces mechanical hypersensitivity. *J. Neurosci.,* vol. 27 (37), 9855-65
- **CHOW A et al.** K+ Channel Expression Distinguishes Subpopulations of Parvalbumin- and Somatostatin-Containing Neocortical Interneurons. *J. Neurosci.,* November 1999, vol. 19 (21), 9332-9345
- **DESAI R et al.** Protein Kinase C Modulates Inactivation of Kv3.3 Channels. *J. Biol. Chem.,* 2008, vol. 283, 22283-22294

- **DEUCHARS SA et al.** Properties of interneurones in the intermediolateral cell column of the rat spinal cord: role of the potassium channel subunit Kv3.1. *Neuroscience,* 2001, vol. 106 (2), 433-46
- **DEVULDER J.** Flupirtine in pain management: pharmacological properties and clinical use. CNS. *Drugs,* vol. 24 (10), 867-81
- **DIB-HAJJ SD et al.** The Na(V)1.7 sodium channel: from molecule to man. *Nat. Rev. Neurosci.,* vol. 14 (1), 49-62
- **DIOCHOT S et al.** Sea Anemone Peptides with a Specific Blocking Activity against the Fast Inactivating Potassium Channel Kv3.4. *J. Biol. Chem.,* vol. 273 (12), 6744-6749
- **ENGEL AK et al.** Dynamic Predictions: Oscillations and Synchrony in Top-Down Processing. *Nat. Rev. Neurosci.,* vol. 2 (10), 704-716
- **ESPINOSA F et al.** Alcohol Hypersensitivity, Increased Locomotion, and Spontaneous Myoclonus in Mice Lacking the Potassium Channels Kv3.1 and Kv3.3. *J. Neurosci.,* September 2001, vol. 21 (17), 6657-6665
- **ESPINOSA F et al.** Ablation of Kv3.1 and Kv3.3 Potassium Channels Disrupts Thalamocortical Oscillations. *In Vitro and In Vivo. J. Neurosci.,* May 2008, vol. 28 (21), 5570-5581
- **FIGUEROA K et al.** KCNC3: phenotype, mutations, channel biophysics - a study of 260 familial ataxia patients. *Human Mutation.,* 2010, vol. 31, 191-196
- **FINNERUP NB et al.** Pharmacotherapy for neuropathic pain in adults: a systematic review and meta-analysis. *Lancet Neurol.,* February 2015, vol. 14 (2), 162-73
- **FISAHN A.** Kainate receptors and rhythmic activity in neuronal networks: hippocampal gamma oscillations as a tool. *J. Physiol.,* October 2005, vol. 561 (1), 65-72
- **GLAIT L et al.** Effects of AUT00063, a Kv3.1 channel modulator, on noise-induced hyperactivity in the dorsal cochlear nucleus. *Hear Res.,* vol. 361, 36-44
- **GREENE TW ; WUTS, PG.** Greene's Protective Groups in Organic Synthesis. John Wiley & Sons, Inc, 2006
- **JOHO RH et al.** Increased γ- and Decreased δ-Oscillations in a Mouse Deficient for a Potassium Channel Expressed in Fast-Spiking Interneurons. *J. Neurophysiol.,* June 1999, vol. 82, 1855-1864
- **JOHO RH ; HURLOCK EC.** The Role of Kv3-type Potassium Channels in Cerebellar Physiology and Behavior. *Cerebellum,* February 2009, vol. 8, 323-333
- **JUNG D et al.** Age-related changes in the distribution of Kv1.1 and Kv3.1 in rat cochlear nuclei. *Neurol. Res.,* 2005, vol. 27, 436-440
- **KASTEN MR et al.** Differential regulation of action potential firing in adult murine thalamocortical neurons by Kv3.2, Kv1, and SK potassium and N-type calcium channels. *J. Physiol.,* 2007, vol. 584 (2), 565-582
- **KACZMAREK L et al.** Regulation of the timing of MNTB neurons by short-term and long-term modulation of potassium channels. *Hearing Res.,* 2005, vol. 206, 133-145
- **LAU D.** Impaired Fast-Spiking, Suppressed Cortical Inhibition, and Increased Susceptibility to Seizures in Mice Lacking Kv3.2 K+ Channel Proteins. *J. Neurosci.,* December 2000, vol. 20 (24), 9071-9085
- **LI W et al.** Localization of Two High-Threshol Potassium Channel Subunits in the Rat Central Auditory System. *J. Comp. Neuro.,* vol. 437, 196-218
- **LU R et al.** Slack channels expressed in sensory neurons control neuropathic pain in mice. *J. Neurosci.,* vol. 35 (3), 1125-35
- **MARKRAM H et al.** Interneurons of the neocortical inhibitory system. *Nat. Rev. Neurosci.,* October 2004, vol. 5, 793-807
- **MARTINA M et al.** Functional and Molecular Differences between Voltage-Gated K+ Channels of Fast-Spiking Interneurons and Pyramidal Neurons of Rat Hippocampus. *J. Neurosci.,* October 1998, vol. 18 (20), 8111-8125
- **MCCARBERG BH et al.** The impact of pain on quality of life and the unmet needs of pain management: results from pain sufferers and physicians participating in an Internet survey. *Am. J. Ther.,* July 2008, vol. 15 (4), 312-20
- **MCDONALD AJ ; MASCAGNI F.** Differential expression of Kv3.1b and Kv3.2 potassium channel subunits in interneurons of the basolateral amygdala. *Neuroscience,* 2006, vol. 138, 537-547
- **MCMAHON A et al.** Allele-dependent changes of olivocerebellar circuit properties in the absence of the voltage-gated potassium channels Kv3.1 and Kv3.3. *Eur. J. Neurosci.,* March 2004, vol. 19, 3317-3327
- **MITCHELL I et al.** Aryl Pyrazoles as Potent Inhibitors of Arginine Methyltransferases: Identification of the First PRMT6 Tool Compound. *ACS Med. Chem. Lett.,* 2015, vol. 6 (6), 655-659
- **MUONA M et al.** A recurrent de novo mutation in KCNC1 causes progressive myoclonus epilepsy. *Nat Genet.,* vol. 47 (1), 39-46
- **MUQEEM T et al.** Regulation of Nociceptive Glutamatergic Signaling by Presynaptic Kv3.4 Channels in the Rat Spinal Dorsal Horn. *J Neurosci,* 11 April 2018, vol. 38 (15), 3729-3740
- **OLSEN T et al.** Kv3 K+ currents contribute to spike-timing in dorsal cochlear nucleus principal cells. *Neuropharmacology,* 01 May 2018, vol. 133, 319-333

- **PILATI N et al.** Acoustic over-exposure triggers burst firing in dorsal cochlear nucleus fusiform cells. *Hearing Research,* 2012, vol. 283, 98-106
- **PUENTE N et al.** Precise localization of the voltage-gated potassium channel subunits Kv3.1b and Kv3.3 revealed in the molecular layer of the rat cerebellar cortex by a pre-embedding immunogold method. *Histochem. Cell. Biol.,* September 2010, vol. 134, 403-409
- **REYNOLDS GP et al.** *Neurotox. Res.,* February 2004, vol. 6 (1), 57-62
- **RITTER DM et al.** Modulation of Kv3.4 channel N-type inactivation by protein kinase C shapes the action potential in dorsal root ganglion neurons. *J. Physiol.,* January 2012, vol. 590 (1), 145-61
- **RITTER DM et al.** Dysregulation of Kv3.4 channels in dorsal root ganglia following spinal cord injury. *J. Neurosci.,* January 2015, vol. 35 (3), 1260-73
- **ROBERTS L et al.** Ringing Ears: The Neuroscience of Tinnitus. *J. Neurosci.,* 2010, vol. 30 (45), 14972-14979
- **RUDY B ; MCBAIN CJ.** Kv3 channels: voltage-gated K+ channels designed for high-frequency repetitive firing. *TRENDS in Neurosci.,* September 2001, vol. 24 (9), 517-526
- **SACCO T et al.** Properties and expression of Kv3 channels in cerebellar Purkinje cells. *Mol. Cell. Neurosci.,* July 2006, vol. 33, 170-179
- **SCHULZ P ; STEIMER T.** Neurobiology of Circadian Systems. *CNS Drugs,* 2009, vol. 23 (2), 3-13
- **SONG P et al.** Acoustic environment determines phosphorylation state of the Kv3.1 potassium channel in auditory neurons. *Nat. Neurosci.,* October 2005, vol. 8 (10), 1335-1342
- **SPENCER KM et al.** Neural synchrony indexes disordered perception and cognition in schizophrenia. *PNAS,* December 2004, vol. 101 (49), 17288-17293
- **SUN S et al.** Inhibitors of voltage-gated sodium channel Nav1.7: patent applications since 2010. *Pharm. Pat. Anal.,* September 2014, vol. 3 (5), 509-21
- *Draft Guidance for Industry Analgesic Indications: Developing Drug and Biological Products,* February 2014, http://www.fda.gov/downloads/drugs/guidancecompliancerequlatoryinformation/guidances/ucm3 84691.pdf
- **VON HEHN C et al.** Loss of Kv3.1 Tonotopicity and Alterations in cAMP Response Element-Binding Protein Signaling in Central Auditory Neurons of Hearing Impaired Mice. *J. Neurosci.,* 2004, vol. 24, 1936-1940
- **WEISER M et al.** Differential Expression of Shaw-related K+ Channels in the Rat Central Nervous System. *J. Neurosci.,* March 1994, vol. 14 (3), 949-972
- **WICKENDEN AD ; MCNAUGHTON-SMITH G.** Kv7 channels as targets for the treatment of pain. *Curr. Pharm. Des.,* 2009, vol. 15 (15), 1773-98
- **WOOLF CJ.** What is this thing called pain?. *J. Clin. Invest.,* November 2010, vol. 120 (11), 3742-4
- **WOOLF CJ.** Central sensitization: implications for the diagnosis and treatment of pain. *Pain,* March 2011, vol. 152 (3), S2-15
- **YANAGI M et al.** Kv3.1-containing K(+) channels are reduced in untreated schizophrenia and normalized with antipsychotic drugs. *Mol Psychiatry.,* 2014, vol. 19 (5), 573-9
- **YEUNG SYM et al.** Modulation of Kv3 Subfamily Potassium Currents by the Sea Anemone Toxin BDS: Significance for CNS and Biophysical Studies. *J. Neurosci.,* March 2005, vol. 25 (38), 8735-8745
- **ZAMPONI GW et al.** The Physiology, Pathology, and Pharmacology of Voltage-Gated Calcium Channels and Their Future Therapeutic Potential. *Pharmacol Rev,* October 2015, vol. 67 (4), 821-70